# EUROPEAN PATENT APPLICATION

(11) **EP 4 548 936 A1**
(43) Date of publication of application: **07.05.2025**
(21) Application number: 23206826.2
(22) Date of filing: 30.10.2023
(51) Int. Cl.: A61K 47/68, A61P 35/00

(54) **ANTIBODY-DRUG CONJUGATES HAVING A TAILOR-MADE DRUG-TO-ANTIBODY RATIO**

(71) Applicant: BioNTech SE, 55131 Mainz (DE)
(72) Inventor: BOGEN, Jan Patrick, Mainz (DE); WÜSTEHUBE-LAUSCH, Joycelyn, Mainz (DE); SCHMOLDT, Hans-Ulrich, Mainz (DE)
(74) Representative: D Young & Co LLP

(57) **Abstract**

The present invention provides an antibody-drug conjugate (ADC), wherein the antibody is conjugated to the drug by cysteine-based site-specific conjugation, wherein the antibody comprises a first polypeptide comprising an immunoglobulin fragment crystallisable (Fc) region, at least one variable domain N-terminal of the Fc region and an engineered IgG hinge region between the Fc region and the at least one variable domain, and wherein the engineered IgG hinge region is heterologous to the Fc region and/or is mutated to provide a predetermined number of cysteine residues.

## Description

### FIELD OF THE INVENTION

The present invention relates to antibody-drug conjugates (ADCs) having a tailor-made drug-to-antibody ratio (DAR). In particular, the ADCs of the invention comprise an engineered IgG hinge region that provides a predetermined number of cysteine residues. This enables the production of ADCs having the desired DAR and a homogenous ADC product using site-specific cysteine conjugation methods. The present invention also relates to methods of producing the ADCs and the therapeutic use of the ADCs.

### BACKGROUND TO THE INVENTION

Antibody-based therapies have proven revolutionary in the treatment of diseases such as cancer. Traditional use of monoclonal antibodies (mAbs) has certain limitations, including a lack of efficacy in certain indications or low tumour expression of the antigen the mAb is targeting. This has led to the development of next-generation antibody-based therapies, including bispecific antibodies (bsAbs), mRNA-encoded antibodies and antibody drug conjugates (ADCs).

ADCs are conjugates of mAbs, bsAbs or antigen-binding antibody fragments that may be specific for a cancer related antigen, with a chemical linker sequence and a drug (often referred to as a "payload"). The payload is generally a molecule with cytotoxic activity, including small molecules, peptide toxins and radionuclides. By generating an ADC, the payload benefits from a long *in vivo* half-life and directed tumour targeting, avoiding off-target activity, due to the conjugated antibody or antigen-binding fragment. Additionally, the chemical linker between the antibody and payload may be designed in a cleavable manner, thus upon internalization of the ADC, tumour or endosome-specific proteases may free the payload enabling cell-specific activity. The payload is then free to mediate toxicity via different pathways, including the inhibition of topoisomerases, the inhibition of polymerization of tubulin or DNA damage.

The DAR is a key property used to measure the quality of ADCs because it can significantly affect ADC efficacy. It has been demonstrated that higher DARs mediate a higher potency of an ADC. It is essential to measure the DAR because a low DAR value may indicate decreased efficacy, while a relatively high DAR value may negatively impact safety. The optimal DAR value for a given ADC varies depending upon several factors such as the payload and target tissue.

Various strategies exist for the conjugation of antibodies to linker-payload molecules, including random conjugation to primary amines present on surface exposed lysine side chains, enzyme-mediated conjugation to specific peptide tags (e.g. transglutaminase or sortase tags) or the utilization of introduced non-natural amino acids or direct conjugation to the N297 glycan structure. However, coupling via lysine side chains yields heterogenic products with varying drug-to-antibody ratios (DAR). Enzyme-mediated conjugation approaches also come with limitations, including the additional manufacturing steps to produce the enzymes and remove the enzymes after the conjugation steps since any residual bacterial enzymes would be immunogenic. Moreover, for sortase a peptide tag must be added to the antibody, which can change the physicochemical properties of the protein, and which might be immunogenic as well.

Despite the success of some first-generation ADCs, their complex heterogeneity has been associated with several problems, including suboptimal therapeutic index, higher clearance rates for high-DAR species, narrower therapeutic windows, and poor stability. To overcome these challenges, second-generation, site-specific ADCs have been designed and developed. The production of site-specific ADCs precisely controls not only the average DARs but also the number of unique conjugation sites. Compared with the production of first-generation ADCs, which yields thousands to millions of different structures, the production of second-generation ADCs yields only a few unique structures, which can be optimized, developed, and manufactured more easily.

Numerous approaches have been developed to generate site-specific ADCs. Conjugation strategies include site-specific coupling via cysteines, which is based on the incorporation of a thiol-reactive group on the linker-payload molecule. One such approach, the Thiomab approach, relies on artificially introducing cysteine residues in the heavy chain (HC) and/or light chain (LC) of the antibody, wherein a toxin-linker comprising maleimide, a moiety capable of covalently conjugating to thiol groups of cysteines, conjugates to the antibody of interest. However, free (unpaired) cysteines and the complicated conjugation strategy can negatively impact the chemistry, manufacturing and controls (CMC) development process of the antibody (Kostova V et al., Pharmaceuticals, 2021; 14: 442). These CMC challenges include, but are not limited to: antibody heterogeneity, low stability, aggregation, low solubility and reduced potency. Furthermore, artificial introduction of cysteine residues may occur at reactive positions within the antibody (for example, in conserved regions of the antibody backbone) and alter the immunogenicity profile of the antibody.

In view of the limitations associated with introducing cysteine residues into the HC and/or LC of an antibody, an alternative conjugation strategy relies on the cysteine residues that are naturally occurring within the antibody. For example, an IgG1 antibody displays four inter-chain disulphide bonds, two between the HCs and one between each HC with its respective LC. It is therefore possible to reduce the inter-chain disulphides to expose eight free cysteines that can be subsequently conjugated to the linker-payloads. Using such an approach, a homogenous product with a DAR of eight can be generated. Antibody fragments such as VHHs, however, are limited in their number of disulphide bridges. A conventional VHH-Fc, for example, exhibits two inter-chain disulphide bridges between the hinge regions of the two HCs. Upon reduction and subsequent conjugation to a linker-payload conjugate via thiol-reactive coupling strategy, such as a maleimide-based conjugation, a homogenous product with a DAR of four may therefore be generated. However, relying upon the naturally occurring cysteine residues within the antibody limits the DAR of the ADC product to the number of naturally occurring inter-chain disulphide-forming cysteine residues within the antibody.

Single domain antibodies (sdAb) like VHHs gained interest in the scientific community due to their small size and high versatility as fusion partners. Due to their small size (~15 KDa) they allow for deep tissue and tumour penetration. However, they generally also exhibit very short half-lives in the range of minutes. As such, VHHs are often fused to half-life extending fusion partners such as serum albumin binding moieties (e.g. peptides or VHHs) or IgG1-derived fragment crystallizable (Fc) portions. VHH-Fc fusions are able to mediate effector functions in a similar fashion to conventional IgGs, while being smaller in size, being less complex in architecture, but still having similar half-lives to conventional IgGs. However, the utilization of VHHs in the context of ADCs is still in an early phase. Moreover, these antibody formats have intrinsic limitations in their use as ADCs.

Thus, there is a demand for new and effective ADC therapeutics, and methods of producing said therapeutics.

### SUMMARY OF THE INVENTION

The present invention is based, at least in part, on the inventors' development of antibody-drug conjugates (ADCs) which have particularly advantageous and surprising properties.

It is desirable to be able to control the DAR of an ADC, for example to produce an ADC which is optimised for a specific therapeutic use by balancing efficacy and safety of the product. The present inventors have developed engineered IgG-derived hinge regions that connect an Fc region with a variable domain N-terminal of the Fc region, wherein the hinge region advantageously comprises a predetermined number of cysteine residues. This enables the production of a homogenous ADC product with the desired DAR via site-specific cysteine-based conjugation strategies. Moreover, by utilising an engineered hinge region that is based upon a naturally occurring IgG hinge region, immunogenicity is reduced. The present inventors have also demonstrated that modifications to avoid O-glycosylation are tolerated within the engineered IgG hinge regions. This is advantageous, since O-glycosylation may lead to CMC challenges of the later ADC, for example by increasing antibody heterogeneity.

The present inventors have surprisingly shown that an ADC comprising an engineered IgG hinge region demonstrates preserved or elevated ADC properties, including stability, cell binding, internalization, antibody-dependent cellular cytotoxicity (ADCC) and complement-dependent cytotoxicity (CDC). Furthermore, the present inventors have surprisingly found that, prior to conjugation, all of the cysteine residues within the engineered IgG hinge regions form stable inter-chain disulphide bonds, i.e. between the corresponding cysteine residues of the engineered IgG hinge regions of the first and second polypeptides of the antibody.

Accordingly, in a first aspect, the present invention provides an antibody-drug conjugate (ADC),
wherein the antibody is conjugated to the drug by cysteine-based site-specific conjugation,
wherein the antibody comprises a first polypeptide comprising an immunoglobulin fragment crystallisable (Fc) region, at least one variable domain N-terminal of the Fc region and an engineered IgG hinge region between the Fc region and the at least one variable domain, and
wherein the engineered IgG hinge region is heterologous to the Fc region and/or is mutated to provide a predetermined number of cysteine residues.

In a further aspect, the present invention provides a method of producing an antibody-drug conjugate (ADC) having a predetermined drug-to-antibody ratio (DAR), comprising the steps of:
(i)
   (a) providing at least one polynucleotide sequence encoding an antibody comprising a first polypeptide comprising an immunoglobulin fragment crystallisable (Fc) region and at least one variable domain N-terminal of the Fc region;
   (b) modifying the at least one polynucleotide sequence to encode an engineered IgG hinge region between the Fc region and the at least one variable domain, wherein the engineered IgG hinge region is heterologous to the Fc region and/or is mutated to provide a predetermined number of cysteine residues;
   (c) introducing the at least one polynucleotide sequence into a cell;
   (d) culturing the cell under conditions suitable for the expression of the at least one polynucleotide sequence;
   (e) isolating the antibody expressed by the cell;
(ii) reducing the antibody with a reducing agent; and
(iii) conjugating the reduced antibody with the drug.

In some embodiments, the at least one variable domain is selected from the group consisting of a single-chain variable fragment (scFv); an Fab; an Fab'; an F(ab)'2; an Fv; a single domain antibody (sdAb); a VHH; a single chain variable domain; a designed ankyrin repeat protein (DARPin); and an aptamer.

In some embodiments, the antibody is selected from the group consisting of a full-length immunoglobulin, a scFv-Fc, a Fab-Fc, an Fv-Fc, a sdAb-Fc, or a VHH-Fc.

In some embodiments, the antibody is a VHH-Fc.

In some embodiments, the antibody further comprises a second polypeptide comprising an Fc region, and step (i)(b) further comprises modifying the at least one polynucleotide sequence to encode an engineered IgG hinge region N-terminal of the Fc region of the second polypeptide, wherein the engineered IgG hinge region is heterologous to the Fc region of the second polypeptide and/or is mutated to provide a predetermined number of cysteine residues, and wherein the engineered IgG hinge region of the first polypeptide and the engineered IgG hinge region of the second polypeptide are complimentary engineered IgG hinge regions.

In some embodiments, the second polypeptide further comprises at least one variable domain N-terminal of the engineered IgG hinge region.

In a further aspect, the present invention provides an antibody-drug conjugate (ADC),
wherein the antibody is conjugated to the drug by cysteine-based site-specific conjugation,
wherein the antibody comprises a first polypeptide and a second polypeptide, wherein each of the first polypeptide and the second polypeptide comprises an immunoglobulin fragment crystallisable (Fc) region, at least one variable domain which is a VHH N-terminal of the Fc region and an engineered IgG hinge region between the Fc region and the at least one VHH,
wherein the engineered IgG hinge region of the first polypeptide and the engineered IgG hinge region of the second polypeptide are complimentary engineered IgG hinge regions, and
wherein the engineered IgG hinge region is heterologous to the Fc region and/or is mutated to provide a predetermined number of cysteine residues.

In a further aspect, the present invention provides a method of producing an antibody-drug conjugate (ADC) having a predetermined drug-to-antibody ratio (DAR), comprising the steps of:
(i) providing an antibody as defined herein;
(ii) reducing the antibody with a reducing agent; and
(iii) conjugating the reduced antibody with the drug.

In a further aspect, the present invention provides a method of producing an antibody-drug conjugate (ADC) having a predetermined drug-to-antibody ratio (DAR), comprising the steps of:
(i)
   (a) providing at least one polynucleotide sequence encoding an antibody wherein the antibody comprises a first polypeptide and a second polypeptide, wherein each of the first polypeptide and the second polypeptide comprises an immunoglobulin fragment crystallisable (Fc) region and at least one variable domain which is a VHH N-terminal of the Fc region;
   (b) modifying the at least one polynucleotide sequence to encode an engineered IgG hinge region between the Fc region and the at least one variable domain of each of the first polypeptide and the second polypeptide, wherein the engineered IgG hinge region of the first polypeptide and the engineered IgG hinge region of the second polypeptide are complimentary engineered IgG hinge regions, and wherein the engineered IgG hinge region is heterologous to the Fc region and/or is mutated to provide a predetermined number of cysteine residues;
   (c) introducing the at least one polynucleotide sequence into a cell;
   (d) culturing the cell under conditions suitable for the expression of the at least one polynucleotide sequence;
   (e) isolating the antibody expressed by the cell;
(ii) reducing the antibody with a reducing agent; and
(iii) conjugating the reduced antibody with the drug.

In some embodiments, the engineered IgG hinge region of the first polypeptide or the engineered IgG hinge region of each of the first polypeptide and the second polypeptide comprises at least two cysteine residues, preferably wherein the engineered IgG hinge region comprises three, four, five, six, seven, eight or nine cysteine residues.

In some embodiments, the engineered IgG hinge region of the first polypeptide comprises at least one additional cysteine residue compared to the native hinge region of the Fc region of the first polypeptide, preferably two, three, four, five, six, seven, eight or nine additional cysteine residues compared to the native hinge region of the Fc region of the first polypeptide.

In some embodiments, the engineered IgG hinge region of the first polypeptide comprises at least one fewer cysteine residue compared to the native hinge region of the Fc region of the first polypeptide, preferably two, three, four, five, six, seven, eight or nine fewer cysteine residues compared to the native hinge region of the Fc region of the first polypeptide.

In some embodiments, the engineered IgG hinge region of the second polypeptide comprises at least one additional cysteine residue compared to the native hinge region of the Fc region of the second polypeptide, preferably two, three, four, five, six, seven, eight or nine additional cysteine residues compared to the native hinge region of the Fc region of the second polypeptide.

In some embodiments, the engineered IgG hinge region of the second polypeptide comprises at least one fewer cysteine residue compared to the native hinge region of the Fc region of the second polypeptide, preferably two, three, four, five, six, seven, eight or nine fewer cysteine residues compared to the native hinge region of the Fc region of the second polypeptide.

In some embodiments, the engineered IgG hinge region comprises a sequence having at least 70% identity to a sequence as set forth in any one of SEQ ID NOs: 1, 2, 3, 4 or 5.

In some embodiments, the engineered IgG hinge region is an IgG1 hinge region.

In some embodiments, the engineered IgG hinge region has an amino acid sequence as set forth in SEQ ID NO: 1, or is a fragment thereof and/or a variant thereof comprising an amino acid sequence having at least 70% identity to SEQ ID NO: 1.

In some embodiments, the engineered IgG hinge region is an IgG2 hinge region.

In some embodiments, the engineered IgG hinge region has an amino acid sequence as set forth in SEQ ID NO: 2, or is a fragment thereof and/or a variant thereof comprising or an amino acid sequence having at least 70% identity to SEQ ID NO: 2.

In some embodiments, the engineered IgG hinge region is an IgG3 hinge region.

In some embodiments, the engineered IgG hinge region has an amino acid sequence as set forth in SEQ ID NO: 3 or SEQ ID NO: 4, or is a fragment thereof and/or a variant thereof comprising an amino acid sequence having at least 70% identity to SEQ ID NO: 3 or to SEQ ID NO: 4.

In some embodiments, the engineered IgG hinge region has an amino acid sequence as set forth in SEQ ID NO: 6 or SEQ ID NO: 7, or is a fragment thereof and/or a variant thereof comprising an amino acid sequence having at least 70% identity to SEQ ID NO: 6 or to SEQ ID NO: 7.

In some embodiments, the engineered IgG hinge region is an IgG4 hinge region.

In some embodiments, the engineered IgG hinge region has an amino acid sequence as set forth in SEQ ID NO: 5, or is a fragment thereof and/or a variant thereof comprising an amino acid sequence having at least 70% identity to SEQ ID NO: 5.

In some embodiments, the engineered IgG hinge region of the first polypeptide and/or second polypeptide comprises at least one amino acid modification to avoid O-glycosylation compared to the native hinge region of the Fc region of the first polypeptide and/or second polypeptide.

In some embodiments, the at least one amino acid modification to avoid O-glycosylation is an amino acid substitution.

In some embodiments, the engineered IgG hinge region is an IgG3 hinge region and the at least one amino acid modification is selected from the group consisting of S^{241MM}X, T^{241JJ}X and the combination thereof, wherein the numbering is according to the according to the Kabat numbering scheme.

In some embodiments, the engineered IgG hinge region has an amino acid sequence as set forth in any one of SEQ ID NOs: 8-118, preferably an amino acid sequence as set forth in SEQ ID NO: 8, 9, 12, 13, 18, 51, 67, 74, 84, 100, 113 or 118.

In some embodiments, the engineered IgG hinge region is or is derived from a human IgG hinge region.

In some embodiments, the antibody is selected from the group consisting of a human antibody, a humanized antibody, a chimeric antibody, a multispecific antibody, a monoclonal antibody, and a polyclonal antibody.

In some embodiments, the at least one variable domain of the first polypeptide, the second polypeptide or both the first polypeptide and the second polypeptide is specific for a cancer antigen.

In some embodiments, the antibody is conjugated to the drug by cysteine-based site-specific conjugation through all of the cysteine residues within the engineered IgG hinge region.

In some embodiments, the ADC has a drug-to-antibody-ratio (DAR) of from about 2 to about 12, preferably a DAR of from about 4 to about 8.

In some embodiments, the ADC has a drug-to-antibody-ratio (DAR) of about 2, 4, 6, 8, 10, or 12, preferably a DAR of about 8.

In some embodiments, the drug is selected from the group consisting of a cytotoxic drug, an antimicrobial agent, or an immunomodulatory agent.

In some embodiments, the step of reducing the antibody in step (ii) is a partial reduction of the antibody to reduce the inter-chain disulphide bonds of the antibody.

In some embodiments, the reducing agent is dithiothreitol (DTT) or tris (2-carboxyethyl) phosphine (TCEP).

In some embodiments, the drug is in the form of a linker-drug conjugate comprising a thiol-reactive group.

In some embodiments, the thiol-reactive group is selected from the group consisting of a maleimide, bromoacetamide, disulphide, α-haloacetamide, α-halocarbonyl, vinylsulfone, heteroaryl sulfone, thiosulfonate, electron deficient aryl halide, ethynylphosphonamidate, vinylphosphonite, palladiumoxidative-addition complex.

In some embodiments, step (iii) is performed using a thiol-reactive coupling strategy.

In some embodiments, the thiol-reactive coupling strategy is maleimide-based conjugation.

In some embodiments, the method of the invention provides homogenous ADCs.

In some embodiments, all of the cysteine residues within the engineered IgG hinge region form stable inter-chain disulphide bonds prior to step (ii).

In some embodiments, in step (iii) the antibody is conjugated to the drug by cysteine-based site-specific conjugation through all of the cysteine residues within the engineered IgG hinge region.

In a further aspect, the present invention provides an ADC obtained or obtainable by the methods of the invention.

In a further aspect, the present invention provides a pharmaceutical composition comprising the ADC according to the invention and a pharmaceutically acceptable carrier, excipient and/or diluent.

In a further aspect, the present invention provides an ADC of the invention or a pharmaceutical composition of the invention for use in therapy.

In a further aspect, the present invention provides an ADC of the invention, or a pharmaceutical composition of the invention for use in the treatment of a disease or condition selected from the group consisting of cancer, autoimmune disease, infection, infectious diseases, cardiovascular diseases and liver metabolic disorders.

### BRIEF DESCRIPTION OF THE FIGURES

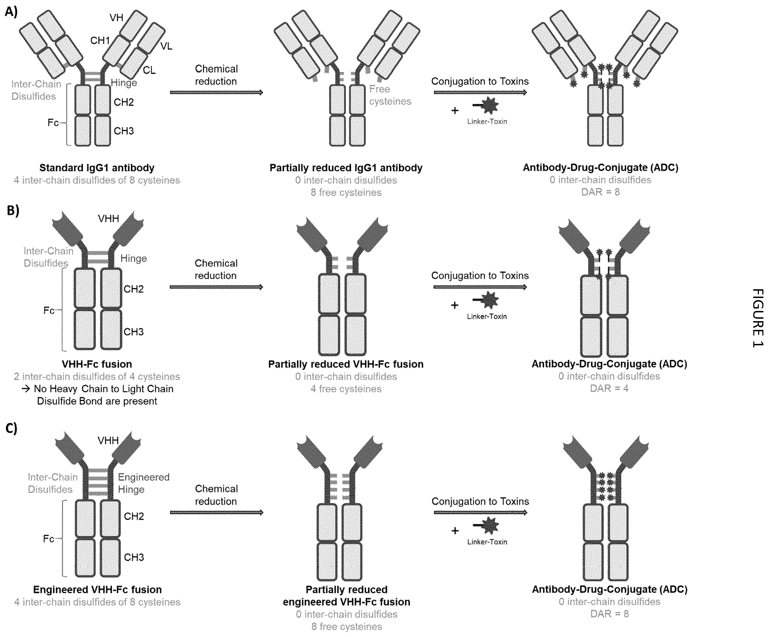

Figure 1: Schematic of cysteine-mediated conjugation of partially reduced antibodies. A. Cysteine-mediated conjugation of a partially reduced IgG1 antibody resulting in an ADC having a DAR of 8. B. Cysteine-mediated conjugation of a partially reduced VHH-Fc fusion resulting in an ADC having a DAR of 4. As VHH-Fcs do not exhibit light chains, two inter-chain disulphides are not present compared to conventional IgG1 antibodies, resulting in a maximal DAR of 4. C. Cysteine-mediated conjugation of a partially reduced, hinge-engineered VHH-Fc fusion resulting in an ADC having a DAR of 8. By engineering a total number of four inter-chain disulphides within the hinge region, this VHH-Fc design allows for an increased DAR compared to a conventional VHH-Fc fusion.

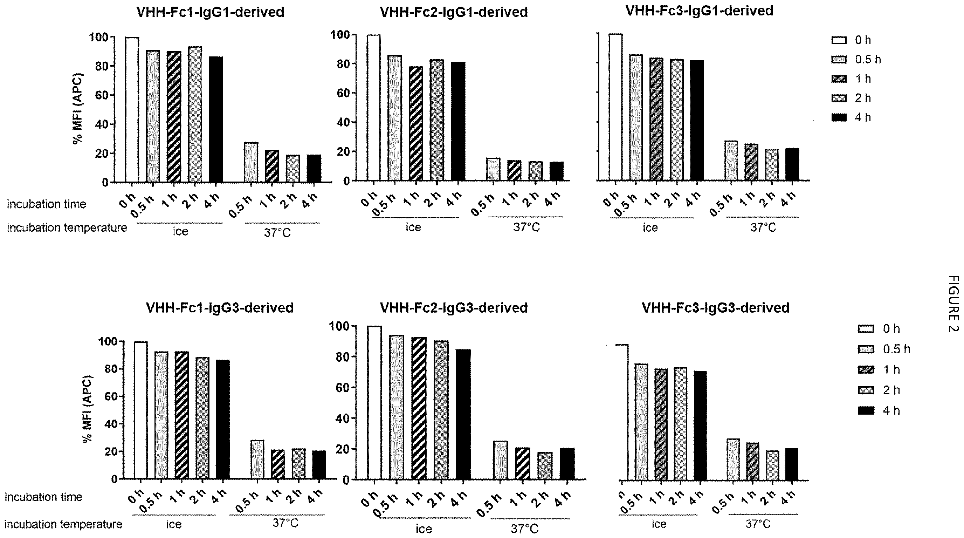

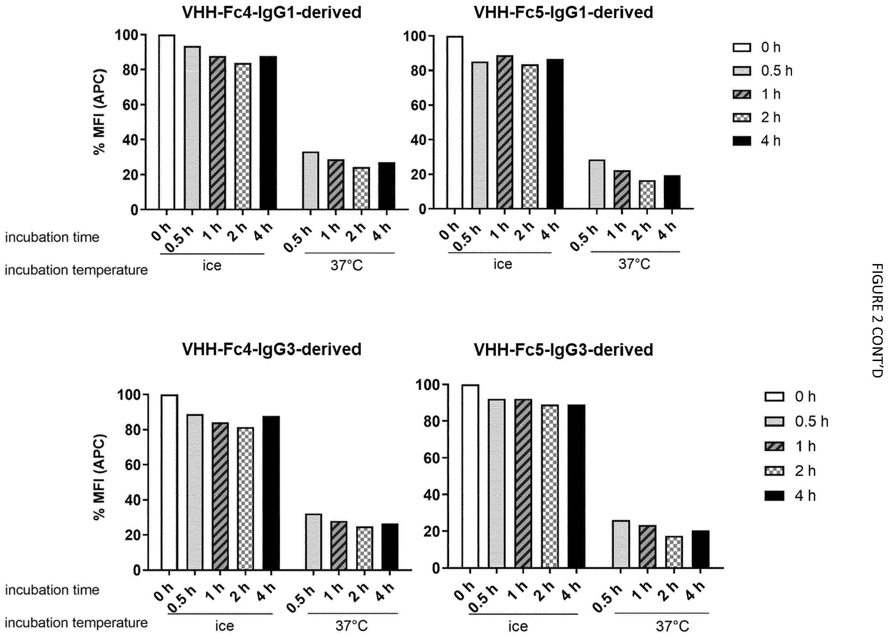

Figure 2: Internalization of VHH-Fc fusions. Internalization of VHH-Fcs into target positive tumour cells was investigated using a FACS-based assay.

### DETAILED DESCRIPTION OF THE INVENTION

Conventional ADC conjugation approaches rely on non-specific/stochastic coupling of drug-linkers to lysines (about 40 residues per IgG1) or naturally occurring inter-chain disulphide forming cysteines within an antibody (about 8 residues per IgG1). These approaches often result in a heterogeneous profile of ADCs with a varying DAR between 2 to 4, leading to CMC challenges.

To overcome these disadvantages, next generation site-specific antibody-drug conjugation methods have been developed. To date, twelve ADCs have gained FDA approval for the treatment of various cancer types, of these, eight ADCs use cysteine-based site-specific conjugation strategies to form the product. ADCs generated using the next generation cysteine-based site-specific antibody-drug conjugation methods show high homogeneity, which is advantageous. However, existing cysteine-based site-specific antibody-drug conjugation methods (e.g. the Thiomab approach) require genetically engineering the antibody backbone to comprise free cysteine residues for conjugation, leading to CMC challenges (Kostova V et al., Pharmaceuticals, 2021; 14: 442) and might increase the immunogenicity of the product. For example, the cysteine mutations are formed as mixed disulphides with cysteine or glutathione, such that the resulting ADC is heterogeneous after production. Moreover, in the Thiomab approach, the reduction of the engineered antibody for conjugation results in partial reduction of the inter-chain disulphides as well as the cysteines genetically engineered into the antibody backbone. This necessitates the re-oxidization of the inter-chain disulphides after the reduction step and before the free cysteines which were genetically engineered into the antibody can be conjugated, i.e. complicates the CMC process.

The present inventors have developed a method for producing ADCs using cysteine-based site-specific conjugation without the need for incorporating artificial cysteine residues within the backbone of the antibody (potentially conserved regions of the antibody backbone). Hence, the immunogenicity profile of the product stays unaltered and no time intensive genetic engineering is needed. Moreover, the methods of producing an ADC provided herein are compatible with established cysteine-based site-specific conjugation strategies which have been used for FDA-approved ADCs.

In particular, the present inventors have engineered the IgG hinge region within the antibody to contain a predetermined number of cysteine residues. As described herein, varying the number of cysteine residues present within the hinge region of the antibodies permits the control of the DAR of the ADC. It is desirable to be able to control the DAR of an ADC, for example, to produce an ADC which is optimised for a specific therapeutic use by balancing efficacy and safety of the product. Engineering the IgG hinge region within the antibody to contain a predetermined number of cysteine residues enables the production of a homogenous ADC product with the desired DAR via cysteine-based site-specific conjugation strategies. Moreover, by utilising an engineered hinge region that is based upon a naturally occurring IgG hinge region, immunogenicity is reduced.

The present inventors have also demonstrated that modifications to avoid O-glycosylation are tolerated within the engineered IgG hinge regions. Furthermore, the present inventors have surprisingly found that, prior to conjugation, all of the cysteine residues within the engineered IgG hinge regions form stable inter-chain disulphide bonds, i.e. between the corresponding cysteine residues of the engineered IgG hinge regions of the first and second polypeptides of the antibody. This is advantageous, since both O-glycosylation and free cysteines will lead to CMC challenges of the later ADC, for example by increasing antibody heterogeneity.

The present inventors have surprisingly demonstrated that an ADC comprising an engineered IgG hinge region demonstrates preserved or elevated ADC properties, including stability, cell binding, internalization, antibody-dependent cellular cytotoxicity (ADCC) and complement-dependent cytotoxicity (CDC).

### Antibody Drug Conjugate

The present invention generally relates to antibody-drug conjugates (ADCs).

ADCs are a class of targeted therapeutics that improves both the selectivity and the activity (e.g. cytotoxic activity) of drugs, such as cancer drugs, by targeting the drugs to specific targets such as cancer cells.

In general, ADCs comprise three main components: (i) an antibody (such as a monoclonal antibody) conjugated to (ii) a linker, which in turn is also conjugated to (iii) a cargo or payload (such as a cytotoxic or chemotherapeutic drug).

In a first aspect, the present invention provides an antibody-drug conjugate (ADC),
wherein the antibody is conjugated to the drug by cysteine-based site-specific conjugation,
wherein the antibody comprises a first polypeptide comprising an immunoglobulin fragment crystallisable (Fc) region, at least one variable domain N-terminal of the Fc region and an engineered IgG hinge region between the Fc region and the at least one variable domain, and
wherein the engineered IgG hinge region is heterologous to the Fc region and/or is mutated to provide a predetermined number of cysteine residues.

In some embodiments, the antibody further comprises a second polypeptide comprising an Fc region and an engineered IgG hinge region N-terminal of the Fc region, wherein the engineered IgG hinge region is heterologous to the Fc region of the second polypeptide and/or is mutated to provide a predetermined number of cysteine residues, and wherein the engineered IgG hinge region of the first polypeptide and the engineered IgG hinge region of the second polypeptide are complimentary engineered IgG hinge regions.

In one embodiment, the second polypeptide does not comprise a variable domain.

In some embodiments, the second polypeptide further comprises at least one variable domain N-terminal of the engineered IgG hinge region.

In some embodiments, the antibody is a VHH-Fc.

Accordingly, in a further aspect, the present invention provides an antibody-drug conjugate (ADC),
wherein the antibody is conjugated to the drug by cysteine-based site-specific conjugation,
wherein the antibody comprises a first polypeptide and a second polypeptide,
wherein each of the first polypeptide and the second polypeptide comprises an immunoglobulin fragment crystallisable (Fc) region, at least one variable domain which is a VHH N-terminal of the Fc region and an engineered IgG hinge region between the Fc region and the at least one VHH,
wherein the engineered IgG hinge region of the first polypeptide and the engineered IgG hinge region of the second polypeptide are complimentary engineered IgG hinge regions, and
wherein the engineered IgG hinge region is heterologous to the Fc region and/or is mutated to provide a predetermined number of cysteine residues.

Cysteine-based site-specific conjugation strategies are known in the art. For example, such strategies are reviewed by Kostova et al. and by Wlash et al. (Kostova V et al., Pharmaceuticals, 2021; 14: 442; and Walsh et al., Chem. Soc. Rev., 2021,50, 1305-1353). Suitably, the cysteine-based site-specific conjugation may be performed using any suitable method known in the art. Suitably, the cysteine-based site-specific conjugation may be performed as described herein (see Examples). Thus, the production of an ADC using cysteine-based site-specific conjugation in accordance with the invention is within the capabilities of a person of ordinary skill in the art.

In some embodiments, the antibody is conjugated to the drug by cysteine-based site-specific conjugation through cysteine residues within the engineered IgG hinge region.

In some embodiments, the antibody is conjugated to the drug by cysteine-based site-specific conjugation through all of the cysteine residues within the engineered IgG hinge region.

In some embodiments, the ADC has a drug-to-antibody-ratio (DAR) of from about 2 to about 22, such as from about 2 to about 20, from about 2 to about 12, from about 4 to about 10, or from about 6 to about 8.

In some embodiments, the ADC has a drug-to-antibody-ratio (DAR) of from about 2 to about 12, preferably a DAR of from about 4 to about 8.

In some embodiments, the ADC has a drug-to-antibody-ratio (DAR) of about 2, 4, 6, 8, 10, 12, 14, 16, 18, 20 or 22. Suitably, the ADC has a DAR of about 4, 6, or 8.

In some embodiments, the ADC has a drug-to-antibody-ratio (DAR) of about 2, 4, 6, 8, 10, or 12, preferably a DAR of about 8.

It will be understood that the term "drug-to-antibody ratio (DAR)" in the context of ADCs refers to the average number of drug molecules connected (i.e. conjugated) to a single antibody. Thus, the "DAR" can be considered as the average number of linked payload (e.g. cytotoxic drugs) molecules per antibody. The number of drug molecules per antibody molecule can be characterized by conventional methods such as UV/visible spectroscopy, mass spectrometry, ELISA assays and HPLC.

The DAR is a key property used to measure the quality of an ADC because it can significantly affect ADC efficacy and CMC challenges for the ADC. It will also be understood that the DAR can affect the safety and therapeutic effectiveness of the ADC. In particular, the DAR value affects the efficacy of the drug, as low drug loading reduces the potency, while high drug loading can negatively affect pharmacokinetics and toxicity.

It is therefore advantageous to be able to control the DAR of an ADC product in order to provide an optimised ADC product having the desired properties including good therapeutic efficacy and safety. By "control the DAR" it is meant that the ADC product can be designed (e.g. engineered) to have a desired DAR. Thus, a tailor-made ADC product with a predetermined DAR can be produced. Suitably, the ADC product may also be homogenous.

As used herein, the terms "homogenous ADCs" and "homogenous ADC product" refer to ADCs having a substantially uniform DAR. Suitably, the ADCs of the invention and produced by the methods of the invention have a uniform DAR, i.e. have the same DAR, wherein the DAR is an integer value. Suitably, the ADCs of the invention and produced by the methods of the invention may be fully conjugated, i.e. all of the cysteine residues which form stable inter-chain disulphide bonds within the antibody (prior to reduction and conjugation) may be conjugated to the drug. Thus, the ADCs of the invention and produced by the methods of the invention may lack unconjugated or incompletely conjugated antibodies (i.e. lack antibodies having free cysteine residues). Suitably, the DAR value may correspond to the number of cysteine residues within the antibody which form stable inter-chain disulphide bonds within the antibody (prior to reduction and conjugation). Cysteine residues forming stable inter-chain disulphide bonds within the antibody (prior to reduction and conjugation) may be located within the hinge region(s) (e.g. the engineered IgG hinge region(s) of the invention) and within the LC, where present. Hence, the DAR value can be controlled for any given antibody format (e.g. antibody formats including a LC, such as an IgG antibody, and antibody formats which do not comprise a LC, such as a VHH-Fc antibody), by providing an engineered IgG hinge region comprising a predetermined number of cysteine residues.

As such, varying the number of cysteine residues present within the hinge region of the antibodies permits the control of the DAR of the ADC. Thus, the DAR directly correlates with the number of predetermined cysteine residues within the engineered IgG hinge region of the first polypeptide and, where present, the engineered IgG hinge region of the second polypeptide. Thus, the methods of the invention provide an ADC having a predetermined DAR through the use of the engineered IgG hinge region(s) as defined herein.

In one embodiment, the DAR value corresponds to the total number of cysteine residues within the antibody which form stable inter-chain disulphide bonds within the antibody prior to reduction and conjugation to the drug.

In one embodiment, the DAR value corresponds to the total number of cysteine residues within the engineered IgG hinge region of the first polypeptide and, where present, the engineered IgG hinge region of the second polypeptide.

By way of example, Figure 1 provides a schematic overview of the production of ADCs via cysteine-based site-specific conjugation based upon a conventional IgG1 antibody (Figure 1A), a conventional VHH-Fc fusion (Figure 1B) and an exemplary antibody in accordance with the invention comprising engineered IgG hinge regions as described herein (Figure 1C).

A conventional IgG1 antibody displays four inter-chain disulphide bonds in total: two between the HCs and one between each HC with its respective LC. It is therefore possible to reduce the inter-chain disulphides to expose eight free cysteines that can be subsequently conjugated to the linker-payloads. Using such an approach, a homogenous product with a DAR of eight can be generated (Figure 1A).

Antibody fragments such as a VHH, however, are limited in their number of disulphide bridges. A conventional VHH-Fc fusion (wherein each polypeptide chain comprises a VHH, a truncated IgG1 hinge and an IgG1 Fc), for example, exhibits two inter-chain disulphide bridges between the two HCs. Since no LCs are present in this antibody format, there are no inter-chain disulphide bridges between a HC and its respective LC and a truncated IgG1 hinge region (SEQ ID NO: 12) is used that comprises two cysteine residues per chain (as opposed to two cysteine residues per chain for the full-length IgG1 hinge region). It is therefore possible to reduce the inter-chain disulphides to expose four free cysteines that can be subsequently conjugated to the linker-payloads. Upon reduction and subsequent conjugation to a linker-payload conjugate via thiol-reactive coupling strategy, such as a maleimide-based conjugation, a homogenous product with a DAR of four may therefore be generated (Figure 1B).

By contrast, by incorporating a total number of four inter-chain disulphides within an engineered IgG hinge region in accordance with the invention, an exemplary engineered VHH-Fc in accordance with the invention exhibits four inter-chain disulphide bridges between the two HCs. Since no LCs are present in this antibody format, there are no inter-chain disulphide bridges between a HC and its respective LC. It is therefore possible to reduce the inter-chain disulphides of this exemplary engineered VHH-Fc in accordance with the invention to expose eight free cysteines that can be subsequently conjugated to the linker-payloads. Upon reduction and subsequent conjugation to a linker-payload conjugate via thiol-reactive coupling strategy, such as a maleimide-based conjugation, a homogenous product with a DAR of eight may therefore be generated (Figure 1C).

Hence, the present invention enables the production of an ADC having a predetermined DAR. In the context of the exemplary ADC provided in Figure 1C, the predetermined DAR is eight. The present invention is not limited to this exemplary ADC - the present invention enables the production of an ADC having any predetermined DAR, wherein the predetermined DAR is an integer which is 2 or a multiple thereof.

The antibody component and the drug component of the antibody-drug conjugate of the invention are linked (i.e. conjugated) to each other via the linker as defined herein.

Such linkers typically have chemically reactive groups at each end. These linkers can form a covalent attachment between two molecules, e.g. the antibody and the drug. Thus, the antibody and the drug may be covalently linked to a linker. Suitably, one region of the linker may bind to the antibody and another region of the linker may bind to the drug.

In some embodiments, the linker may be a cleavable linker. In some embodiments, the linker may be a maleimide tetrapeptide-based cleavable linker.

In some embodiments, the linker comprises a thiol-reactive group selected from the group consisting of a maleimide, bromoacetamide, disulphide, α-haloacetamide, α-halocarbonyl, vinylsulfone, heteroaryl sulfone, thiosulfonate, electron deficient aryl halide, ethynylphosphonamidate, vinylphosphonite, palladiumoxidative-addition complex, bis-sulfone, water-soluble allyl sulfone, thiol-yne bioconjugation with terminal alkyne or cyclooctyne, dibromo- (DBM) and dithio-maleimide (DTM), hybrid thiobromomaleimide (TBM), dibromopyridazinediones, divinylpyrimidine, and DiPODS (two oxadiazolyl methyl sulfone moieties connected by a phenyl group).

In some embodiments, the linker comprises a thiol-reactive group selected from the group consisting of a maleimide, bromoacetamide, disulphide, α-haloacetamide, α-halocarbonyl, vinylsulfone, heteroaryl sulfone, thiosulfonate, electron deficient aryl halide, ethynylphosphonamidate, vinylphosphonite, and palladiumoxidative-addition complex.

Linkers for use in a cysteine-based site-specific conjugation strategy in accordance with the invention are known in the art. The linker for use according to the present invention may be any suitable linker known in the art.

Without wishing to be bound by theory, the antibody-drug conjugate according to the invention may have (but is not limited to) one or more of the following features:
- High potency of payload;
- High drug-to-antibody ratio;
- Stable linker-payload;
- Tumour-selective cleavable linker
- ADCC activity; and/or
- ADCC activity and bystander antitumour effect.

### Engineered IgG hinge region

As discussed above, the present invention permits the control of the DAR of the ADC by varying the number of cysteine residues present within the hinge region of the antibodies. Without wishing to be bound by theory, even though the inter-chain disulphides between the hinge regions of the antibody prior to conjugation stabilize the IgG molecule, they are not essential for the structural integrity of the antibody. Hence, it is possible to conjugate the antibody to the linker-drug via all of the cysteine residues within the engineered IgG hinge region without compromising the structural integrity of the antibody.

In one embodiment, the engineered IgG hinge region of the first polypeptide is heterologous to the Fc region of the first polypeptide. The use of the engineered IgG hinge region which is heterologous to the Fc region of the first polypeptide thereby provides the predetermined number of cysteine residues.

In one embodiment, the engineered IgG hinge region of the first polypeptide is homologous to the Fc region of the first polypeptide and is mutated to provide a predetermined number of cysteine residues. The use of the engineered IgG hinge region which is mutated as described herein thereby provides the predetermined number of cysteine residues.

In one embodiment, the engineered IgG hinge region of the first polypeptide is heterologous to the Fc region of the first polypeptide and is mutated to provide a predetermined number of cysteine residues. The use of the engineered IgG hinge region which is heterologous to the Fc region of the first polypeptide and mutated as described herein thereby provides the predetermined number of cysteine residues.

In one embodiment, the engineered IgG hinge region of the second polypeptide is heterologous to the Fc region of the second polypeptide. The use of the engineered IgG hinge region which is heterologous to the Fc region of the second polypeptide thereby provides the predetermined number of cysteine residues.

In one embodiment, the engineered IgG hinge region of the second polypeptide is homologous to the Fc region of the second polypeptide and is mutated to provide a predetermined number of cysteine residues. The use of the engineered IgG hinge region which is mutated as described herein thereby provides the predetermined number of cysteine residues.

In one embodiment, the engineered IgG hinge region of the second polypeptide is heterologous to the Fc region of the second polypeptide and is mutated to provide a predetermined number of cysteine residues. The use of the engineered IgG hinge region which is heterologous to the Fc region of the first polypeptide and mutated as described herein thereby provides the predetermined number of cysteine residues.

As used herein, the term "IgG hinge region" refers to the region of the native IgG chain which is denoted the hinge region. For example, for human IgG isotypes, the IgG hinge region may be denoted according to the Kabat numbering scheme.

As used herein, the term "engineered IgG hinge region" refers to an IgG hinge region that is heterologous to the Fc region of the first polypeptide or of the second polypeptide, that is mutated to provide a predetermined number of cysteine residues as described herein or that is both heterologous to the Fc region of the first polypeptide or of the second polypeptide and mutated to provide a predetermined number of cysteine residues as described herein.

As used herein, the term "heterologous to the Fc region" refers to an engineered IgG hinge region that is or is based upon the hinge region of a different immunoglobulin isotype to the Fc region of the polypeptide or to the Fc region variant (as described herein) of the polypeptide. For example, an IgG2 hinge, an IgG3 hinge and an IgG4 hinge are each heterologous to an IgG1 Fc region or a variant thereof; an IgG1 hinge, an IgG3 hinge and an IgG4 hinge are each heterologous to an IgG2 Fc region or a variant thereof; and so on. The heterologous hinge region may additionally be mutated as described herein.

As used herein, the terms "homologous to the Fc region" and "native hinge region of the Fc region" refers to a hinge region (e.g. an engineered IgG hinge region) that is the hinge region of the same immunoglobulin isotype to the Fc region of the polypeptide or to the Fc region variant (as described herein) of the polypeptide. For example, an IgG1 hinge is homologous to an IgG1 Fc region or a variant thereof; an IgG2 hinge is homologous to an IgG2 Fc region or a variant thereof; and so on. The homologous hinge region is mutated as described herein to form an engineered IgG hinge region in accordance with the invention.

A native IgG hinge region may be mutated by addition, deletion and/or substitution of at least one residue present in the naturally-occurring hinge region to provide the engineered IgG hinge region having a preselected number of cysteine residues in accordance with the present invention. Suitably, the nucleotide sequence encoding a native IgG hinge region may be mutated by addition, deletion and/or substitution of at least one nucleotide present in the sequence encoding the naturally-occurring hinge region to encode the engineered IgG hinge region having a preselected number of cysteine residues in accordance with the present invention.

In some embodiments, the native IgG hinge region is mutated by addition to provide a predetermined number of cysteine residues.

In some embodiments, the native IgG hinge region is mutated by deletion to provide a predetermined number of cysteine residues. Thus, the engineered IgG hinge region may be a truncation of a native IgG hinge region. The native IgG hinge region may be truncated to provide the predetermined number of cysteine residues.

In some embodiments, the native IgG hinge region is mutated by substitution to provide a predetermined number of cysteine residues.

In some embodiments, the native IgG hinge region is mutated by addition and deletion to provide a predetermined number of cysteine residues.

In some embodiments, the native IgG hinge region is mutated by addition and substitution to provide a predetermined number of cysteine residues.

In some embodiments, the native IgG hinge region is mutated by deletion and substitution to provide a predetermined number of cysteine residues.

The mutation of a native IgG hinge region to provide the predetermined number of cysteine residues using conventional techniques in molecular biology is within the capabilities of a person of ordinary skill in the art. Generally speaking, suitable routine methods include directed mutagenesis, gene synthesis and recombinant DNA/RNA technology.

As used herein, the term "complimentary engineered IgG hinge regions" means that the engineered IgG hinge regions of the first and second polypeptides have the same length and comprise cysteine residues at the same positions within the hinge region according to the Kabat numbering scheme. That the cysteine residues are located at the same positions within the hinge regions of the first and second polypeptides enables the cysteine residues within the engineered IgG hinge regions to form stable inter-chain disulphide bonds within the antibody prior to conjugation to the payload. Thus, the engineered IgG hinge regions of the first and second polypeptides each comprise the same number of cysteine residues, i.e. the predetermined number of cysteine residues for the engineered IgG hinge region of the first polypeptide and for the engineered IgG hinge region of the second polypeptide is identical. Suitably, the engineered IgG hinge regions of the first and second polypeptides may be identical.

In a preferred embodiment, the engineered IgG hinge region of the first and/or second polypeptide comprises a predetermined number of cysteine residues.

In some embodiments, the engineered IgG hinge region of the first polypeptide comprises at least one cysteine residue. Suitably, the engineered IgG hinge region comprises two, three, four, five, six, seven, eight, nine, ten or eleven cysteine residues.

The engineered IgG hinge regions of the first polypeptide and the second polypeptide each comprise at least one cysteine residue. In some embodiments, the engineered IgG hinge regions each comprise at least two, three, four, five, six, seven, eight, nine, ten or eleven cysteine residues. In some embodiments, the engineered IgG hinge regions each comprise two, three, four, five, six, seven, eight, nine, ten or eleven cysteine residues. Thus, the predetermined number of cysteine residues may be one, two, three, four, five, six, seven, eight, nine, ten or eleven cysteine residues.

In one embodiment, the engineered IgG hinge regions each comprise at least two cysteine residues. In one embodiment, the engineered IgG hinge regions each comprise at least three cysteine residues. In one embodiment, the engineered IgG hinge regions each comprise at least four cysteine residues. In one embodiment, the engineered IgG hinge regions each comprise at least five cysteine residues. In one embodiment, the engineered IgG hinge regions each comprise at least six cysteine residues. In one embodiment, the engineered IgG hinge regions each comprise at least seven cysteine residues. In one embodiment, the engineered IgG hinge regions each comprise at least eight cysteine residues. In one embodiment, the engineered IgG hinge regions each comprise at least nine cysteine residues. In one embodiment, the engineered IgG hinge regions each comprise at least ten cysteine residues. In one embodiment, the engineered IgG hinge regions each comprise at least eleven cysteine residues.

The predetermined number of cysteine residues is determined by the desired DAR. For example, if the desired DAR (also termed the 'predetermined DAR') is eight, and the antibody does not comprise any LCs, then the predetermined number of cysteine residues for the engineered IgG hinge region of each of the first polypeptide and the second polypeptide is four. By way of further example, if the desired DAR (also termed the 'predetermined DAR') is ten, and the antibody does not comprise any LCs, then the predetermined number of cysteine residues for the engineered IgG hinge region of each of the first polypeptide and the second polypeptide is five.

In some embodiments, the engineered IgG hinge region of the first polypeptide comprises at least one additional cysteine residue compared to the native hinge region of the Fc region of the first polypeptide. Suitably, the engineered IgG hinge region of the first polypeptide comprises two, three, four, five, six, seven, eight or nine additional cysteine residues compared to the native hinge region of the Fc region of the first polypeptide.

In some embodiments, the engineered IgG hinge region of the first polypeptide comprises at least one fewer cysteine residue compared to the native hinge region of the Fc region of the first polypeptide. Suitably, the engineered IgG hinge region of the first polypeptide comprises two, three, four, five, six, seven, eight or nine fewer cysteine residues compared to the native hinge region of the Fc region of the first polypeptide.

In some embodiments, the engineered IgG hinge region of the second polypeptide comprises at least one additional cysteine residue compared to the native hinge region of the Fc region of the second polypeptide. Suitably, the engineered IgG hinge region of the second polypeptide comprises two, three, four, five, six, seven, eight or nine additional cysteine residues compared to the native hinge region of the Fc region of the second polypeptide.

In some embodiments, the engineered IgG hinge region of the second polypeptide comprises at least one fewer cysteine residue compared to the native hinge region of the Fc region of the second polypeptide. Suitably, the engineered IgG hinge region of the second polypeptide comprises two, three, four, five, six, seven, eight or nine fewer cysteine residues compared to the native hinge region of the Fc region of the second polypeptide.

In some embodiments, all of the cysteine residues within the engineered IgG hinge regions of the first and second polypeptides form stable inter-chain disulphide bonds prior to drug-conjugation. In other words, the antibody does not comprise any free cysteine residues within the engineered IgG hinge regions of the first and second polypeptides. Thus, all of the cysteine residues within the engineered IgG hinge region of the first polypeptide may form stable inter-chain disulphide bonds with the corresponding cysteine residue (i.e. the cysteine residue at the same position according to the Kabat numbering system) within the engineered IgG hinge region of the second polypeptide. For an illustrative example of complete disulphide bond formation in an antibody format that does not comprise a LC, see the left hand panel of Figure 1B or of Figure 1C. Alternatively, all of the cysteine residues within the engineered IgG hinge region of the first polypeptide may form stable inter-chain disulphide bonds with the corresponding cysteine residue (i.e. cysteine residues at the same position according to the Kabat numbering system) within the engineered IgG hinge region of the second polypeptide and with at least one cysteine residue within the LC, where present. For an illustrative example of complete disulphide bond formation in an antibody format comprising a LC, see the left hand panel of Figure 1A. Free (unpaired) cysteines can negatively impact the chemistry, manufacturing and controls (CMC) development process of the antibody. These CMC challenges include, but are not limited to: antibody heterogeneity, low stability, aggregation, low solubility and reduced potency. Therefore, the ADCs of the invention have the advantage of avoiding these challenges associated with free cysteines.

Suitably, the engineered IgG hinge regions of the first and second polypeptides may be mutated as described herein such that they do not comprise any free cysteine residues prior to drug-conjugation. In this regard, the native human IgG1 hinge regions contain cysteine residues which form stable inter-chain disulphide bonds with the LC. For use with antibody formats that do not comprise a LC (e.g. a VHH-Fc), the native human IgG1 hinge regions may therefore be mutated as described herein to eliminate the cysteine residues (for example, by deletion or substitution) which do not form stable inter-chain disulphide bonds in the absence of a LC.

As described herein, the engineered IgG hinge region may be heterologous to the Fc region of the first polypeptide or of the second polypeptide. Thus, the engineered IgG hinge region may be a native IgG1, IgG2, IgG3 or IgG4 hinge region, with the proviso that the engineered IgG hinge region is heterologous to the Fc region of the first polypeptide or of the second polypeptide. For example, the engineered IgG hinge region may be a native IgG1 hinge region if the Fc region if the first and second polypeptides is an IgG2 Fc region.

Accordingly, in some embodiments, the engineered IgG hinge region has an amino acid sequence as set forth in any one of SEQ ID NOs: 1, 2, 3, 4, or 5.

The engineered IgG hinge region may be mutated to provide a predetermined number of cysteine residues by truncation of a native IgG hinge region. Thus, the engineered IgG hinge region may be a fragment of a native IgG1, IgG2, IgG3 or IgG4 hinge region that provides a predetermined number of cysteine residues. The engineered IgG hinge region may be heterologous to the Fc region of the first polypeptide or the second polypeptide and be mutated to provide a predetermined number of cysteine residues by truncation of a native IgG hinge region that is heterologous to the Fc region of the first polypeptide or the second polypeptide.

Accordingly, in some embodiments, the engineered IgG hinge region is a fragment of an amino acid sequence as set forth in any one of SEQ ID NOs: 1, 2, 3, 4, or 5. The fragment may have a length of between about 4 and about 60 (suitably, between about 5 and about 55, between about 10 and about 50, between about 15 and about 45, between about 20 and about 40, or between about 25 and about 35) amino acids. Suitably, the fragment may have a length of about 4, about 5, about 6, about 7, about 8, about 9, about 10, about 11, about 12, about 13, about 14, about 15, about 16, about 17, about 18, about 19 or about 20 amino acids. Suitably fragments are described herein (see, for example, Table 1).

The engineered IgG hinge region may be mutated to provide a predetermined number of cysteine residues by substitution of one or more amino acids of a native IgG hinge region to provide a predetermined number of cysteine residues. For example, the native IgG hinge region may be mutated by substituting one or more cysteine residues with an amino acid residue other than cysteine in order to reduce the number of cysteine residues within the resulting engineered IgG hinge region. Alternatively or additionally, the native IgG hinge region may be mutated by substituting one or more non-cysteine amino acid residues with a cysteine residue in order to increase the number of cysteine residues within the resulting engineered IgG hinge region.

The engineered IgG hinge region may be heterologous to the Fc region of the first polypeptide or the second polypeptide and be mutated to provide a predetermined number of cysteine residues by substitution of one or more amino acids of a native IgG hinge region that is heterologous to the Fc region of the first polypeptide or the second polypeptide.

Accordingly, in some embodiments, the engineered IgG hinge region has an amino acid sequence comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to any one of SEQ ID NOs: 1, 2, 3, 4, or 5.

The engineered IgG hinge region may be mutated to provide a predetermined number of cysteine residues by truncation of and by substitution of one or more amino acids of a native IgG hinge region.

The engineered IgG hinge region may be heterologous to the Fc region of the first polypeptide or the second polypeptide and be mutated to provide a predetermined number of cysteine residues by truncation of and by substitution of one or more amino acids of a native IgG hinge region that is heterologous to the Fc region of the first polypeptide or the second polypeptide.

Accordingly, in some embodiments, the engineered IgG hinge region is a fragment and a variant of any one of SEQ ID NOs: 1, 2, 3, 4 or 5 comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to any one of SEQ ID NOs: 1, 2, 3, 4, or 5.

In some embodiments, the engineered IgG hinge region is an IgG1 hinge region. Suitably, the engineered IgG hinge region is an IgG1 hinge region that has been mutated as described herein.

An illustrative human IgG1 sequence is as follows:
EPKSCDKTHTCPPCP (SEQ ID NO: 1)

In some embodiments, the engineered IgG hinge region has an amino acid sequence as set forth in SEQ ID NO: 1, or is a fragment thereof and/or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to SEQ ID NO: 1. Suitably, the variant comprises or consists of an amino acid sequence having at least 90% (suitably, at least 91%, at least 92%, at least 93%, at least 94%, at least 95%, at least 96%, at least 97%, at least 98%, at least 99%, at least 100%) identity to SEQ ID NO: 1.

In some embodiments, the engineered IgG hinge region is an IgG2 hinge region. Suitably, the engineered IgG hinge region is an IgG2 hinge region that has been mutated as described herein.

An illustrative human IgG2 sequence is as follows:
ERKCCVECPPCP (SEQ ID NO: 2)

In some embodiments, the engineered IgG hinge region has an amino acid sequence as set forth in SEQ ID NO: 2, or is a fragment thereof and/or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to SEQ ID NO: 2. Suitably, the variant comprises or consists of an amino acid sequence having at least 90% (suitably, at least 91%, at least 92%, at least 93%, at least 94%, at least 95%, at least 96%, at least 97%, at least 98%, at least 99%, at least 100%) identity to SEQ ID NO: 2.

In some embodiments, the engineered IgG hinge region is an IgG3 hinge region. Suitably, the engineered IgG hinge region is an IgG3 hinge region that has been mutated as described herein.

An illustrative human IgG3 sequence is as follows:
ELKTPLGDTTHTCPRCPEPKSCDTPPPCPRCPEPKSCDTPPPCPRCPEPKSCDTPPPCPRCP (SEQ ID NO: 3)

A further illustrative human IgG3 sequence is as follows:
ELKTPLGDTTHTCPRCPEPKSCDTPPPCPRCP (SEQ ID NO: 4)

In some embodiments, the engineered IgG hinge region has an amino acid sequence as set forth in SEQ ID NO: 3 or SEQ ID NO: 4, or is a fragment thereof and/or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to SEQ ID NO: 3 or to SEQ ID NO: 4. Suitably, the variant comprises or consists of an amino acid sequence having at least 90% (suitably, at least 91%, at least 92%, at least 93%, at least 94%, at least 95%, at least 96%, at least 97%, at least 98%, at least 99%, at least 100%) identity to SEQ ID NO: 3 or SEQ ID NO: 4.

The human IgG3*01 sequence (SEQ ID NO: 3) is arranged in four exons. The translated amino acid sequences of exons 2, 3 and 4 is identical. Any of the exon sequences may be used in the practice of the present invention. Thus, a fragment of SEQ ID NO: 3 may correspond to the sequence of exon 1 or of exon 2, 3 or 4.

An illustrative human IgG3 exon 1 sequence (translated to amino acids) is as follows:
ELKTPLGDTTHTCPRCP (SEQ ID NO: 6)

An illustrative human IgG3 exon 2, exon 3 or exon 4 sequence (translated to amino acids) is as follows:
EPKSCDTPPPCPRCP (SEQ ID NO: 7)

In some embodiments, the engineered IgG hinge region has an amino acid sequence as set forth in SEQ ID NO: 6 or SEQ ID NO: 7, or is a fragment thereof and/or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to SEQ ID NO: 6 or to SEQ ID NO: 7. Suitably, the variant comprises or consists of an amino acid sequence having at least 90% (suitably, at least 91%, at least 92%, at least 93%, at least 94%, at least 95%, at least 96%, at least 97%, at least 98%, at least 99%, at least 100%) identity to SEQ ID NO: 6 or SEQ ID NO: 7.

In some embodiments, the engineered IgG hinge region is an IgG4 hinge region. Suitably, the engineered IgG hinge region is an IgG4 hinge region that has been mutated as described herein.

An illustrative human IgG4 sequence is as follows:
ESKYGPPCPSCP (SEQ ID NO: 5)

In some embodiments, the engineered IgG hinge region has an amino acid sequence as set forth in SEQ ID NO: 5, or is a fragment thereof and/or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to SEQ ID NO: 5. Suitably, the variant comprises or consists of an amino acid sequence having at least 90% (suitably, at least 91%, at least 92%, at least 93%, at least 94%, at least 95%, at least 96%, at least 97%, at least 98%, at least 99%, at least 100%) identity to SEQ ID NO: 5.

It is known that the threonine and serine residues within the human IgG3 hinge are prone to O-glycosylation. The serine residues of the human IgG3 hinge as well as threonine and serine residues of other IgG isotype hinge regions may also be subject to O-glycosylation. Without wishing to be bound by theory, the O-glycosylation may lead to CMC challenges for the ADC, for example by increasing heterogeneity of the product. Therefore, it is desirable to avoid O-glycosylation of the antibody hinge region for ADC applications.

The modification of a native IgG hinge region to avoid O-glycosylation using conventional techniques in molecular biology is within the capabilities of a person of ordinary skill in the art. Generally speaking, suitable routine methods include directed mutagenesis, gene synthesis and recombinant DNA/RNA technology.

In some embodiments, the engineered IgG hinge region of the first polypeptide and/or second polypeptide comprises at least one amino acid modification to avoid O-glycosylation compared to the native IgG hinge region of the Fc region of the first polypeptide and/or second polypeptide. Suitably, the at least one amino acid modification may be a deletion or a substitution of a serine and/or threonine residue. Suitably, the at least one amino acid modification may be a deletion or a substitution of at least one serine residue. Suitably, the at least one amino acid modification may be a deletion or a substitution of all of the serine residues within the native IgG hinge region. Suitably, the at least one amino acid modification may be a deletion or a substitution of at least one threonine residue. Suitably, the at least one amino acid modification may be a deletion or a substitution of all of the threonine residues within the native IgG hinge region. Suitably, the at least one amino acid modification may be a deletion or a substitution of at least one serine and at least one threonine residue. Suitably, the at least one amino acid modification may be a deletion or a substitution of all of the serine and threonine residues within the native IgG hinge region.

In some embodiments, the at least one amino acid modification to avoid O-glycosylation is an amino acid substitution.

In some embodiments, the engineered IgG hinge region is an IgG3 hinge region and the at least one amino acid modification is selected from the group consisting of S^{241F}X, T^{241I}X and the combination thereof, wherein the numbering is according to the according to the Kabat numbering scheme, and wherein X is any amino acid except C, S or T.

In some embodiments, the engineered IgG hinge region is an IgG3 hinge region and the at least one amino acid modification is selected from the group consisting of S^{241U}X, T^{241X}X and the combination thereof, wherein the numbering is according to the according to the Kabat numbering scheme, and wherein X is any amino acid except C, S or T.

In some embodiments, the engineered IgG hinge region is an IgG3 hinge region and the at least one amino acid modification is selected from the group consisting of S^{241MM}X, T^{241JJ}X and the combination thereof, wherein the numbering is according to the according to the Kabat numbering scheme, and wherein X is any amino acid except C, S or T.

In one embodiment, the engineered IgG hinge region is an IgG3 hinge region and the at least one amino acid modification is S^{241MM}X, wherein the numbering is according to the according to the Kabat numbering scheme, and wherein X is any amino acid except C, S or T.

In some embodiments, the engineered IgG hinge region is an IgG3 hinge region and the at least one amino acid modification is T^{241JJ}X, wherein the numbering is according to the according to the Kabat numbering scheme, and wherein X is any amino acid except C or T.

In some embodiments, the engineered IgG hinge region is an IgG3 hinge region and the at least one amino acid modification is S^{241MM}X and T^{241JJ}X, wherein the numbering is according to the according to the Kabat numbering scheme, and wherein X is any amino acid except C, S or T.

In some embodiments, the engineered IgG hinge region is an IgG3 hinge region and the at least one amino acid modification is selected from the group consisting of S^{241F}X, T^{241I}X, S^{241U}X, T^{241X}X, S^{241MM}X, T^{241JJ}X and the combination thereof, wherein the numbering is according to the according to the Kabat numbering scheme, and wherein X is any amino acid except C, S or T.

Illustrative engineered IgG hinge region sequences which may be employed in the practice of the invention are detailed below in Table 1.

**Table 1** - **Illustrative engineered IgG hinge region sequences.** The predetermined DAR of an ADC wherein the first and the second polypeptide each comprise the hinge sequence is also provided (referred to as "DAR" in the table).

| **SEQ ID NO** | **Sequence** | **Description** | **DAR** |
|---|---|---|---|
| 1 | EPKSCDKTHTCPPCP | Human IgG1 hinge | 4 |
| 2 | ERKCCVECPPCP | Human IgG2 hinge | 8 |
| 3 | ELKTPLGDTTHTCPRCPEPKSCDTPPPCPR CPEPKSCDTPPPCPRCPEPKSCDTPPPCPR CP | IgG3*01 | 22 |
| 4 | ELKTPLGDTTHTCPRCPEPKSCDTPPPCPR CP | IGHG3*04-Hinge | 10 |
| 5 | ESKYGPPCPSCP | Human IgG4 hinge | 4 |
| 6 | ELKTPLGDTTHTCPRCP | IgG3 exon 1 | 4 |
| 7 | EPKSCDTPPPCPRCP | IgG3 exon 2, 3 or 4 | 6 |

| **IgG1-derived hinge** | | | |
|---|---|---|---|
| 8 | DKTHTXPPCP | Wherein the X at position 6 can be any amino acid except C, preferably S | 2 |
| 9 | DKTHTCPPXP | Wherein the X at position 9 can be any amino acid except C, preferably S | 2 |
| 10 | EPKSXDKTHTXPPCP | Wherein the X at position 5 is either C if a (human) IgG1 Fab is fused N-terminally or any amino acid except C, if any other (or no) binding domain is fused N-terminally, but preferably S Wherein the X at position 11 can be any amino acid except C, preferably S | 2 |
| 11 | EPKSXDKTHTCPPXP | Wherein the X at position 5 is either C if a (human) IgG1 Fab is fused N-terminally or any amino acid except C, if any other (or no) binding domain is fused N-terminally, but preferably S Wherein the X at position 14 can be any amino acid except C, preferably S | 2 |
| 12 | DKTHTCPPCP | Truncated human IgG1 hinge | 4 |
| 13 | EPKSXDKTHTCPPCP | Wherein the X at position 5 is either C if a (human) IgG1 Fab is fused N-terminally or any amino acid except C, if any other (or no) binding domain is fused N-terminally, but preferably S | 4 |

| **IgG2-derived hinge** | | | |
|---|---|---|---|
| 14 | ERKXXVEXPPCP | Wherein the X at position 4 can be any amino acid except C, preferably S | 2 |
| | | Wherein the X at position 5 can be any amino acid except C, preferably S | |
| | | Wherein the X at position 8 can be any amino acid except C, preferably S | |
| 15 | ERKXXVECPPXP | Wherein the X at position 4 can be any amino acid except C, preferably S | 2 |
| | | Wherein the X at position 5 can be any amino acid except C, preferably S | |
| | | Wherein the X at position 11 can be any amino acid except C, preferably S | |
| 16 | ERKXCVEXPPXP | Wherein the X at position 4 can be any amino acid except C, preferably S | 2 |
| | | Wherein the X at position 8 can be any amino acid except C, preferably S | |
| | | Wherein the X at position 11 can be any amino acid except C, preferably S | |
| 17 | ERKCXVEXPPXP | Wherein the X at position 5 can be any amino acid except C, preferably S | 2 |
| | | Wherein the X at position 8 can be any amino acid except C, preferably S | |
| | | Wherein the X at position 11 can be any amino acid except C, preferably S | |
| 18 | ERKXXVECPPCP | Wherein the X at position 4 can be any amino acid except C, preferably S | 4 |
| | | Wherein the X at position 5 can be any amino acid except C, preferably S | |
| 19 | ERKXCVEXPPCP | Wherein the X at position 4 can be any amino acid except C, preferably S | 4 |
| | | Wherein the X at position 8 can be any amino acid except C, preferably S | |
| 20 | ERKXCVECPPXP | Wherein the X at position 4 can be any amino acid except C, preferably S | 4 |
| | | Wherein the X at position 11 can be any amino acid except C, preferably S | |
| 21 | ERKCXVEXPPCP | Wherein the X at position 5 can be any amino acid except C, preferably S | 4 |
| | | Wherein the X at position 8 can be any amino acid except C, preferably S | |
| 22 | ERKCXVECPPXP | Wherein the X at position 5 can be any amino acid except C, preferably S | 4 |
| | | Wherein the X at position 11 can be any amino acid except C, preferably S | |
| 23 | ERKCCVEXPPXP | Wherein the X at position 8 can be any amino acid except C, preferably S | 4 |
| | | Wherein the X at position 11 can be any amino acid except C, preferably S | |
| 24 | ERKXCVECPPCP | Wherein the X at position 4 can be any amino acid except C, preferably S | 6 |
| 25 | ERKCXVECPPCP | Wherein the X at position 5 can be any amino acid except C, preferably S | 6 |
| 26 | ERKCCVEXPPCP | Wherein the X at position 8 can be any amino acid except C, preferably S | 6 |
| 27 | ERKCCVECPPXP | Wherein the X at position 11 can be any amino acid except C, preferably S | 6 |

| **IgG3-derived hinge** | | | |
|---|---|---|---|
| 28 | EPKSXDTPPPXPRCP | Wherein the X at position 5 can be any amino acid except C, preferably S | 2 |
| | | Wherein the X at position 11 can be any amino acid except C, preferably S | |
| | | Wherein any one or more of amino acids at any of positions 1-11 may be deleted | |
| 29 | PRCP | Truncated human IgG3 hinge | 2 |
| 30 | EPKSXDTPPPCPRXP | Wherein the X at position 5 can be any amino acid except C, preferably S | 2 |
| | | Wherein the X at position 14 can be any amino acid except C, preferably S | |
| | | Wherein any one or more of amino acids at any of positions 1-10 may be deleted | |
| 31 | CPRXP | Wherein the X at position 4 can be any amino acid except C, preferably S | 2 |
| 32 | EPKSCDTPPPXPRXP | Wherein the X at position 11 can be any amino acid except C, preferably S | 2 |
| | | Wherein the X at position 14 can be any amino acid except C, preferably S | |
| | | Wherein any one or more of amino acids at any of positions 1-4 may be deleted | |
| 33 | CDTPPPXPRXP | Wherein the X at position 7 can be any amino acid except C, preferably S | 2 |
| | | Wherein the X at position 10 can be any amino acid except C, preferably S | |
| 34 | EPKSXDXPPPXPRCP | Wherein the X at position 5 can be any amino acid except C, preferably S | 2 |
| | | Wherein the X at position 11 can be any amino acid except C, preferably S | |
| | | Wherein the X at position 7 can be any amino acid except C or T, preferably any amino acid except C, S or T, preferably wherein X is A | |
| | | Wherein any one or more of amino acids at any of positions 1-11 may be deleted | |
| 35 | EPKSXDXPPPCPRXP | Wherein the X at position 5 can be any amino acid except C, preferably S | 2 |
| | | Wherein the X at position 14 can be any amino acid except C, preferably S | |
| | | Wherein the X at position 7 can be any amino acid except C or T, preferably any amino acid except C, S or T, preferably wherein X is A | |
| | | Wherein any one or more of amino acids at any of positions 1-10 may be deleted | |
| 36 | EPKSCDXPPPXPRXP | Wherein the X at position 11 can be any amino acid except C, preferably S | 2 |
| | | Wherein the X at position 14 can be any amino acid except C, preferably S | |
| | | Wherein the X at position 7 can be any amino acid except C or T, preferably any amino acid except C, S or T, preferably wherein X is A | |
| | | Wherein any one or more of amino acids at any of positions 1-4 may be deleted | |
| 37 | CDXPPPXPRXP | Wherein the X at position 7 can be any amino acid except C, preferably S | 2 |
| | | Wherein the X at position 10 can be any amino acid except C, preferably S | |
| | | Wherein the X at position 3 can be any amino acid except C or T, preferably any amino acid except C, S or T, preferably wherein X is A | |
| 38 | EPKXXDTPPPXPRCP | Wherein the X at position 5 can be any amino acid except C, preferably S | 2 |
| | | Wherein the X at position 11 can be any amino acid except C, preferably S | |
| | | Wherein the X at position 4 can be any amino acid except C, T or S, preferably any amino acid except C, S or T, preferably wherein X is A | |
| | | Wherein any one or more of amino acids at any of positions 1-11 may be deleted | |
| 39 | EPKXXDTPPPCPRXP | Wherein the X at position 5 can be any amino acid except C, preferably S | 2 |
| | | Wherein the X at position 14 can be any amino acid except C, preferably S | |
| | | Wherein the X at position 4 can be any amino acid except C, T or S, preferably any amino acid except C, S or T, preferably wherein X is A | |
| | | Wherein any one or more of amino acids at any of positions 1-10 may be deleted | |
| 40 | EPKXCDTPPPXPRXP | Wherein the X at position 11 can be any amino acid except C, preferably S | 2 |
| | | Wherein the X at position 14 can be any amino acid except C, preferably S | |
| | | Wherein the X at position 4 can be any amino acid except C, T or S, preferably any amino acid except C, S or T, preferably wherein X is A | |
| | | Wherein any one or more of amino acids at any of positions 1-4 may be deleted | |
| 41 | EPKXXDXPPPXPRCP | Wherein the X at position 5 can be any amino acid except C, preferably S | 2 |
| | | Wherein the X at position 11 can be any amino acid except C, preferably S | |
| | | Wherein the X at position 4 can be any amino acid except C, T or S, preferably wherein X is A | |
| | | Wherein the X at position 7 can be any amino acid except C or T, preferably any amino acid except C, S or T, preferably wherein X is A | |
| | | Wherein any one or more of amino acids at any of positions 1-11 may be deleted | |
| 42 | EPKXXDXPPPCPRXP | Wherein the X at position 5 can be any amino acid except C, preferably S | 2 |
| | | Wherein the X at position 14 can be any amino acid except C, preferably S | |
| | | Wherein the X at position 4 can be any amino acid except C, T or S, preferably A | |
| | | Wherein the X at position 7 can be any amino acid except C or T, preferably any amino acid except C, S or T, preferably wherein X is A | |
| | | Wherein any one or more of amino acids at any of positions 1-10 may be deleted | |
| 43 | EPKXCDXPPPXPRXP | Wherein the X at position 11 can be any amino acid except C, preferably S | 2 |
| | | Wherein the X at position 14 can be any amino acid except C, preferably S | |
| | | Wherein the X at position 4 can be any amino acid except C, T or S, preferably A | |
| | | Wherein the X at position 7 can be any amino acid except C or T, preferably any amino acid except C, S or T, preferably wherein X is A | |
| | | Wherein any one or more of amino acids at any of positions 1-4 may be deleted | |
| 44 | CDXPPPXPRXP | Wherein the X at position 7 can be any amino acid except C, preferably S | 2 |
| | | Wherein the X at position 10 can be any amino acid except C, preferably S | |
| | | Wherein the X at position 3 can be any amino acid except C or T, preferably any amino acid except C, S or T, preferably wherein X is A | |
| 45 | EPKSXDTPPPCPRCP | Wherein the X at position 5 can be any amino acid except C, preferably S | 4 |
| | | Wherein any one or more of amino acids at any of positions 1-10 may be deleted | |
| 46 | CPRCP | Truncated human IgG3 hinge | 4 |
| 47 | EPKSCDTPPPXPRCP | Wherein the X at position 11 can be any amino acid except C, preferably S | 4 |
| | | Wherein any one or more of amino acids at any of positions 1-4 may be deleted | |
| 48 | CDTPPPXPRCP | Wherein the X at position 7 can be any amino acid except C, preferably S | 4 |
| 49 | EPKSCDTPPPCPRXP | Wherein the X at position 14 can be any amino acid except C, preferably S | 4 |
| | | Wherein any one or more of amino acids at any of positions 1-4 may be deleted | |
| 50 | CDTPPPCPRXP | Wherein the X at position 10 can be any amino acid except C, preferably S | 4 |
| 51 | EPKSXDXPPPCPRCP | Wherein the X at position 5 can be any amino acid except C, preferably S | 4 |
| | | Wherein the X at position 7 can be any amino acid except C or T, preferably any amino acid except C, S or T, preferably wherein X is A | |
| | | Wherein any one or more of amino acids at any of positions 1-10 may be deleted | |
| 52 | EPKSCDXPPPXPRCP | Wherein the X at position 11 can be any amino acid except C, preferably S | 4 |
| | | Wherein the X at position 7 can be any amino acid except C or T, preferably any amino acid except C, S or T, preferably wherein X is A | |
| | | Wherein any one or more of amino acids at any of positions 1-4 may be deleted | |
| 53 | CDXPPPXPRCP | Wherein the X at position 3 can be any amino acid except C or T, preferably any amino acid except C, S or T, preferably wherein X is A | 4 |
| | | Wherein the X at position 7 can be any amino acid except C, preferably S | |
| 54 | EPKSCDXPPPCPRXP | Wherein the X at position 14 can be any amino acid except C, preferably S | 4 |
| | | Wherein the X at position 7 can be any amino acid except C or T, preferably any amino acid except C, S or T, preferably wherein X is A | |
| | | Wherein any one or more of amino acids at any of positions 1-4 may be deleted | |
| 55 | CDXPPPCPRXP | Wherein the X at position 3 can be any amino acid except C or T, preferably any amino acid except C, S or T, preferably wherein X is A | 4 |
| | | Wherein the X at position 10 can be any amino acid except C, preferably S | |
| 56 | EPKXXDTPPPCPRCP | Wherein the X at position 5 can be any amino acid except C, preferably S | 4 |
| | | Wherein the X at position 4 can be any amino acid except C, T or S, preferably A | |
| | | Wherein any one or more of amino acids at any of positions 1-10 may be deleted | |
| 57 | EPKXCDTPPPXPRCP | Wherein the X at position 11 can be any amino acid except C, preferably S | 4 |
| | | Wherein the X at position 4 can be any amino acid except C, T or S, preferably A | |
| | | Wherein any one or more of amino acids at any of positions 1-4 may be deleted | |
| 58 | EPKXCDTPPPCPRXP | Wherein the X at position 14 can be any amino acid except C, preferably S | 4 |
| | | Wherein the X at position 4 can be any amino acid except C, T or S, preferably A | |
| | | Wherein any one or more of amino acids at any of positions 1-4 may be deleted | |
| 59 | EPKXXDXPPPCPRCP | Wherein the X at position 5 can be any amino acid except C, preferably S | 4 |
| | | Wherein the X at position 4 can be any amino acid except C, T or S, preferably A | |
| | | Wherein the X at position 7 can be any amino acid except C or T, preferably any amino acid except C, S or T, preferably wherein X is A | |
| | | Wherein any one or more of amino acids at any of positions 1-10 may be deleted | |
| 60 | EPKXCDXPPPXPRCP | Wherein the X at position 11 can be any amino acid except C, preferably S | 4 |
| | | Wherein the X at position 4 can be any amino acid except C, T or S, preferably A | |
| | | Wherein the X at position 7 can be any amino acid except C or T, preferably any amino acid except C, S or T, preferably wherein X is A | |
| | | Wherein any one or more of amino acids at any of positions 1-4 may be deleted | |
| 61 | EPKXCDXPPPCPRXP | Wherein the X at position 14 can be any amino acid except C, preferably S | 4 |
| | | Wherein the X at position 4 can be any amino acid except C, T or S, preferably A | |
| | | Wherein the X at position 7 can be any amino acid except C or T, preferably any amino acid except C, S or T, preferably wherein X is A | |
| | | Wherein any one or more of amino acids at any of positions 1-4 may be deleted | |
| 62 | EPKSCDTPPPCPRCP | Wherein any one or more of amino acids at any of positions 1-4 may be deleted | 6 |
| 63 | CDTPPPCPRCP | Truncated human IgG3 hinge | 6 |
| 64 | EPKSCDXPPPCPRCP | Wherein the X at position 7 can be any amino acid except C or T, preferably any amino acid except C, S or T, preferably wherein X is A | 6 |
| | | Wherein any one or more of amino acids at any of positions 1-4 may be deleted | |
| 65 | CDXPPPCPRCP | Wherein the X at position 3 can be any amino acid except C or T, preferably any amino acid except C, S or T, preferably wherein X is A | 6 |
| 66 | EPKXCDTPPPCPRCP | Wherein the X at position 4 can be any amino acid except C, T or S, preferably A | 6 |
| | | Wherein any one or more of amino acids at any of positions 1-4 may be deleted | |
| 67 | EPKXCDXPPPCPRCP | Wherein the X at position 4 can be any amino acid except C, T or S, preferably A | 6 |
| | | Wherein the X at position 7 can be any amino acid except C or T, preferably any amino acid except C, S or T, preferably wherein X is A | |
| | | Wherein any one or more of amino acids at any of positions 1-4 may be deleted | |
| 68 | PRCPEPKSCDTPPPCPRCP | Wherein any one or more of amino acids at any of positions 1-2 may be deleted | 8 |
| 69 | CPEPKSCDTPPPCPRCP | Truncated human IgG3 hinge | 8 |
| 70 | PRCPEPKSCDXPPPCPRCP | Wherein the X at position 11 can be any amino acid except C or T, preferably any amino acid except C, S or T, preferably wherein X is A | 8 |
| | | Wherein any one or more of amino acids at any of positions 1-2 may be deleted | |
| 71 | CPEPKSCDXPPPCPRCP | Wherein the X at position 9 can be any amino acid except C or T, preferably any amino acid except C, S or T, preferably wherein X is A | 8 |
| 72 | PRCPEPKXCDTPPPCPRCP | Wherein the X at position 8 can be any amino acid except C, T or S, preferably A | 8 |
| | | Wherein any one or more of amino acids at any of positions 1-2 may be deleted | |
| 73 | CPEPKXCDTPPPCPRCP | Wherein the X at position 6 can be any amino acid except C, T or S, preferably A | 8 |
| 74 | PRCPEPKXCDXPPPCPRCP | Wherein the X at position 8 can be any amino acid except C, T or S, preferably A | 8 |
| | | Wherein the X at position 11 can be any amino acid except C or T, preferably any amino acid except C, S or T, preferably wherein X is A | |
| | | Wherein any one or more of amino acids at any of positions 1-2 may be deleted | |
| 75 | CPEPKXCDXPPPCPRCP | Wherein the X at position 6 can be any amino acid except C, T or S, preferably A | 8 |
| | | Wherein the X at position 9 can be any amino acid except C or T, preferably any amino acid except C, S or T, preferably wherein X is A | |
| 76 | DTPPPCPRCPEPKSCDTPPPCPRCP | Wherein any one or more of amino acids at any of positions 1-5 may be deleted | 10 |
| 77 | CPRCPEPKSCDTPPPCPRCP | Truncated human IgG3 hinge | 10 |
| 78 | DTPPPCPRCPEPKSCDXPPPCPRCP | Wherein the X at position 17 can be any amino acid except C or T, preferably any amino acid except C, S or T, preferably wherein X is A | |
| | | Wherein any one or more of amino acids at any of positions 1-5 may be deleted | |
| 79 | CPRCPEPKSCDXPPPCPRCP | Wherein the X at position 12 can be any amino acid except C or T, preferably any amino acid except C, S or T, preferably wherein X is A | 10 |
| 80 | DXPPPCPRCPEPKSCDTPPPCPRCP | Wherein the X at position 2 can be any amino acid except C or T, preferably any amino acid except C, S or T, preferably wherein X is A | 10 |
| | | Wherein any one or more of amino acids at any of positions 1-5 may be deleted | |
| 81 | DXPPPCPRCPEPKSCDXPPPCPRCP | Wherein the X at position 2 can be any amino acid except C or T, preferably any amino acid except C, S or T, preferably wherein X is A | 10 |
| | | Wherein the X at position 17 can be any amino acid except C or T, preferably any amino acid except C, S or T, preferably wherein X is A | |
| | | Wherein any one or more of amino acids at any of positions 1-5 may be deleted | |
| 82 | DTPPPCPRCPEPKXCDTPPPCPRCP | Wherein the X at position 14 can be any amino acid except C, T or S, preferably A | 10 |
| | | Wherein any one or more of amino acids at any of positions 1-5 may be deleted | |
| 83 | CPRCPEPKXCDTPPPCPRCP | Wherein the X at position 9 can be any amino acid except C, T or S, preferably A | 10 |
| 84 | DXPPPCPRCPEPKXCDXPPPCPRCP | Wherein the X at position 2 can be any amino acid except C or T, preferably any amino acid except C, S or T, preferably wherein X is A | 10 |
| | | Wherein the X at position 14 can be any amino acid except C, T or S, preferably A | |
| | | Wherein the X at position 17 can be any amino acid except C or T, preferably any amino acid except C, S or T, preferably wherein X is A | |
| | | Wherein any one or more of amino acids at any of positions 1-5 may be deleted | |
| 85 | CPRCPEPKXCDXPPPCPRCP | Wherein the X at position 9 can be any amino acid except C, T or S, preferably A | 10 |
| | | Wherein the X at position 12 can be any amino acid except C or T, preferably any amino acid except C, S or T, preferably wherein X is A | |
| 86 | DXPPPCPRCPEPKXCDTPPPCPRCP | Wherein the X at position 2 can be any amino acid except C or T, preferably any amino acid except C, S or T, preferably wherein X is A | 10 |
| | | Wherein the X at position 14 can be any amino acid except C, T or S, preferably A | |
| | | Wherein any one or more of amino acids at any of positions 1-5 may be deleted | |
| 87 | DTPPPCPRCPEPKXCDXPPPCPRCP | Wherein the X at position 14 can be any amino acid except C, T or S, preferably A | 10 |
| | | Wherein the X at position 17 can be any amino acid except C or T, preferably any amino acid except C, S or T, preferably wherein X is A | |
| | | Wherein any one or more of amino acids at any of positions 1-5 may be deleted | |
| 88 | EPKSCDTPPPCPRCPEPKSCDTPPPCPRCP | Wherein any one or more of amino acids at any of positions 1-4 may be deleted | 12 |
| 89 | CDTPPPCPRCPEPKSCDTPPPCPRCP | Truncated IgG3 hinge | 12 |
| 90 | EPKSCDXPPPCPRCPEPKSCDTPPPCPRCP | Wherein the X at position 7 can be any amino acid except C or T, preferably any amino acid except C, S or T, preferably wherein X is A | 12 |
| | | Wherein any one or more of amino acids at any of positions 1-4 may be deleted | |
| 91 | CDXPPPCPRCPEPKSCDTPPPCPRCP | Wherein the X at position 3 can be any amino acid except C or T, preferably any amino acid except C, S or T, preferably wherein X is A | 12 |
| 92 | EPKSCDTPPPCPRCPEPKSCDXPPPCPRCP | Wherein the X at position 22 can be any amino acid except C or T, preferably any amino acid except C, S or T, preferably wherein X is A | 12 |
| | | Wherein any one or more of amino acids at any of positions 1-4 may be deleted | |
| 93 | CDTPPPCPRCPEPKSCDXPPPCPRCP | Wherein the X at position 18 can be any amino acid except C or T, preferably any amino acid except C, S or T, preferably wherein X is A | 12 |
| 94 | EPKSCDXPPPCPRCPEPKSCDXPPPCPRCP | Wherein the X at position 7 can be any amino acid except C or T, preferably any amino acid except C, S or T, preferably wherein X is A | 12 |
| | | Wherein the X at position 22 can be any amino acid except C or T, preferably any amino acid except C, S or T, preferably wherein X is A | |
| | | Wherein any one or more of amino acids at any of positions 1-4 may be deleted | |
| 95 | CDXPPPCPRCPEPKSCDXPPPCPRCP | Wherein the X at position 3 can be any amino acid except C or T, preferably any amino acid except C, S or T, preferably wherein X is A | 12 |
| | | Wherein the X at position 18 can be any amino acid except C or T, preferably any amino acid except C, S or T, preferably wherein X is A | |
| 96 | EPKXCDTPPPCPRCPEPKSCDTPPPCPRCP | Wherein the X at position 4 can be any amino acid except C, T or S, preferably A | 12 |
| | | Wherein any one or more of amino acids at any of positions 1-4 may be deleted | |
| 97 | EPKSCDTPPPCPRCPEPKXCDTPPPCPRCP | Wherein the X at position 19 can be any amino acid except C, T or S, preferably A | 12 |
| | | Wherein any one or more of amino acids at any of positions 1-4 may be deleted | |
| 98 | CDTPPPCPRCPEPKXCDTPPPCPRCP | Wherein the X at position 15 can be any amino acid except C, T or S, preferably A | 12 |
| 99 | EPKXCDTPPPCPRCPEPKXCDTPPPCPRCP | Wherein the X at position 4 can be any amino acid except C, T or S, preferably A | 12 |
| | | Wherein the X at position 19 can be any amino acid except C, T or S, preferably A | |
| | | Wherein any one or more of amino acids at any of positions 1-4 may be deleted | |
| 100 | EPKXCDXPPPCPRCPEPKXCDXPPPCPRCP | Wherein the X at position 4 can be any amino acid except C, T or S, preferably A | 12 |
| | | Wherein the X at position 7 can be any amino acid except C or T, preferably any amino acid except C, S or T, preferably wherein X is A | |
| | | Wherein the X at position 19 can be any amino acid except C, T or S, preferably A | |
| | | Wherein the X at position 22 can be any amino acid except C or T, preferably any amino acid except C, S or T, preferably wherein X is A | |
| | | Wherein any one or more of amino acids at any of positions 1-4 may be deleted | |
| 101 | CDXPPPCPRCPEPKXCDXPPPCPRCP | Wherein the X at position 3 can be any amino acid except C or T, preferably any amino acid except C, S or T, preferably wherein X is A | 12 |
| | | Wherein the X at position 15 can be any amino acid except C, T or S, preferably A | |
| | | Wherein the X at position 18 can be any amino acid except C or T, preferably any amino acid except C, S or T, preferably wherein X is A | |
| 102 | EPKXCDXPPPCPRCPEPKXCDTPPPCPRCP | Wherein the X at position 4 can be any amino acid except C, T or S, preferably A | 12 |
| | | Wherein the X at position 7 can be any amino acid except C or T, preferably any amino acid except C, S or T, preferably wherein X is A | |
| | | Wherein the X at position 19 can be any amino acid except C, T or S, preferably A | |
| | | Wherein any one or more of amino acids at any of positions 1-4 may be deleted | |
| 103 | CDXPPPCPRCPEPKXCDTPPPCPRCP | Wherein the X at position 3 can be any amino acid except C or T, preferably any amino acid except C, S or T, preferably wherein X is A | 12 |
| | | Wherein the X at position 15 can be any amino acid except C, T or S, preferably A | |
| 104 | EPKXCDXPPPCPRCPEPKSCDXPPPCPRCP | Wherein the X at position 4 can be any amino acid except C, T or S, preferably A | 12 |
| | | Wherein the X at position 7 can be any amino acid except C or T, preferably any amino acid except C, S or T, preferably wherein X is A | |
| | | Wherein the X at position 22 can be any amino acid except C or T, preferably any amino acid except C, S or T, preferably wherein X is A | |
| | | Wherein any one or more of amino acids at any of positions 1-4 may be deleted | |
| 105 | EPKXCDTPPPCPRCPEPKXCDXPPPCPRCP | Wherein the X at position 4 can be any amino acid except C, T or S, preferably A | 12 |
| | | Wherein the X at position 19 can be any amino acid except C, T or S, preferably A | |
| | | Wherein the X at position 22 can be any amino acid except C or T, preferably any amino acid except C, S or T, preferably wherein X is A | |
| | | Wherein any one or more of amino acids at any of positions 1-4 may be deleted | |
| 106 | CDTPPPCPRCPEPKXCDXPPPCPRCP | Wherein the X at position 15 can be any amino acid except C, T or S, preferably A | 12 |
| | | Wherein the X at position 18 can be any amino acid except C or T, preferably any amino acid except C, S or T, preferably wherein X is A | |
| 107 | EPKSCDXPPPCPRCPEPKXCDXPPPCPRCP | Wherein the X at position 7 can be any amino acid except C or T, preferably any amino acid except C, S or T, preferably wherein X is A | 12 |
| | | Wherein the X at position 19 can be any amino acid except C, T or S, preferably A | |
| | | Wherein the X at position 22 can be any amino acid except C or T, preferably any amino acid except C, S or T, preferably wherein X is A | |
| | | Wherein any one or more of amino acids at any of positions 1-4 may be deleted | |
| 108 | EPKXCDXPPPCPRCPEPKSCDTPPPCPRCP | Wherein the X at position 4 can be any amino acid except C, T or S, preferably A | 12 |
| | | Wherein the X at position 7 can be any amino acid except C or T, preferably any amino acid except C, S or T, preferably wherein X is A | |
| | | Wherein any one or more of amino acids at any of positions 1-4 may be deleted | |
| 109 | EPKXCDTPPPCPRCPEPKSCDXPPPCPRCP | Wherein the X at position 4 can be any amino acid except C, T or S, preferably A | 12 |
| | | Wherein the X at position 22 can be any amino acid except C or T, preferably any amino acid except C, S or T, preferably wherein X is A | |
| | | Wherein any one or more of amino acids at any of positions 1-4 may be deleted | |
| 110 | EPKSCDXPPPCPRCPEPKXCDTPPPCPRCP | Wherein the X at position 7 can be any amino acid except C or T, preferably any amino acid except C, S or T, preferably wherein X is A | 12 |
| | | Wherein the X at position 19 can be any amino acid except C, T or S, preferably A | |
| | | Wherein any one or more of amino acids at any of positions 1-4 may be deleted | |
| 111 | EPKSCDTPPPCPRCPEPKXCDXPPPCPRCP | Wherein the X at position 19 can be any amino acid except C, T or S, preferably A | 12 |
| | | Wherein the X at position 22 can be any amino acid except C or T, preferably any amino acid except C, S or T, preferably wherein X is A | |
| 112 | PRCPEPKACDAPPPCPRCP | Truncated human IgG3 hinge | 8 |
| 113 | DXPPPCPRCP | Wherein the X at position 2 can be any amino acid except C or T, preferably any amino acid except C, S or T, preferably wherein X is A | 4 |

| **IgG4-derived hinge** | | | |
|---|---|---|---|
| 114 | ESKYGPPXPSCP | Wherein the X at position 8 can be any amino acid except C, preferably S | 2 |
| 115 | ESKYGPPCPSXP | Wherein the X at position 11 can be any amino acid except C, preferably S | 2 |
| 116 | ESKYGPPXPXCP | Wherein the X at position 8 can be any amino acid except C, preferably S | 2 |
| | | Wherein the X at position 10 can be any amino acid except C, most preferably P and least preferably S, A or G | |
| 117 | ESKYGPPCPXXP | Wherein the X at position 11 can be any amino acid except C, preferably S | 2 |
| | | Wherein the X at position 10 can be any amino acid except C, most preferably P and least preferably S, A or G | |
| 118 | ESKYGPPCPXCP | Wherein the X at position 10 can be any amino acid except C, most preferably P and least preferably S, A or G | 4 |

In some embodiments, the engineered IgG hinge region has an amino acid sequence as set forth in any one of SEQ ID NOs: 8-118, or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to any one of SEQ ID NOs: 8-118. Suitably, the variant comprises or consists of an amino acid sequence having at least 90% (suitably, at least 91%, at least 92%, at least 93%, at least 94%, at least 95%, at least 96%, at least 97%, at least 98%, at least 99%, at least 100%) identity to any one of SEQ ID NOs: 8-118. Suitable fragments of SEQ ID Nos: 8-118 for use according to the invention are described in Table 1.

In some embodiments, the engineered IgG hinge region has an amino acid sequence as set forth in any one of SEQ ID NOs: 8-118, preferably an amino acid sequence as set forth in any one of SEQ ID NOs: 8, 9, 12, 13, 18, 51, 67, 74, 84, 100, 113 or 118, more preferably an amino acid sequence as set forth in any one of SEQ ID NOs: 8, 9, 18, 51, 67, 74, 84, 100, 113 or 118.

In some embodiments, the engineered IgG hinge region is a fragment of an amino acid sequence as set forth in any one of SEQ ID NOs: 8-118, preferably an amino acid sequence as set forth in any one of SEQ ID NOs: 8, 9, 12, 13, 18, 51, 67, 74, 84, 100, 113 or 118, more preferably an amino acid sequence as set forth in any one of SEQ ID NOs: 8, 9, 18, 51, 67, 74, 84, 100, 113 or 118.

In some embodiments, the engineered IgG hinge region comprises or consists of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity to any one of SEQ ID NOs: 8-118, preferably an amino acid sequence as set forth in any one of SEQ ID NOs: 8, 9, 12, 13, 18, 51, 67, 74, 84, 100, 113 or 118, more preferably an amino acid sequence as set forth in any one of SEQ ID NOs: 8, 9, 18, 51, 67, 74, 84, 100, 113 or 118.

In some embodiments, the engineered IgG hinge region is a fragment and a variant of an amino acid sequence as set forth in any one of SEQ ID NOs: 8-118, preferably an amino acid sequence as set forth in any one of SEQ ID NOs: 8, 9, 12, 13, 18, 51, 67, 74, 84, 100, 113 or 118, more preferably an amino acid sequence as set forth in any one of SEQ ID NOs: 8, 9, 18, 51, 67, 74, 84, 100, 113 or 118, wherein the engineered IgG hinge region comprises or consists of a sequence having at least at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 8 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 9 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 12 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 13 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 18 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 51 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 67 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 74 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 84 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 100 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 113 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region has an amino acid sequence as set forth in SEQ ID NO: 118 or is a fragment thereof and/or or a variant thereof comprising or consisting of an amino acid sequence having at least 70% (suitably, at least 75%, at least 80%, at least 85%, at least 90%, at least 91%, at least 92%, at least 93%, at least 94%, at least 95%, at least 96%, at least 97%, at least 98%, at least 99%, at least 100%) identity thereto.

In some embodiments, the engineered IgG hinge region is or is derived from a human, murine, camelid, rabbit, sheep, goat or chicken IgG hinge region.

In some embodiments, the engineered IgG hinge region is or is derived from a human IgG hinge region. This provides the advantage that the engineered IgG hinge region is less immunogenic as compared to a non-human IgG hinge region sequence.

As used herein, the terms "derived from" or "based upon" a native (e.g. a human) IgG hinge region refer to a hinge region which has been mutated as described herein to provide a predetermined number of cysteine residues.

### Antibody

As used herein, the term "antibody" may refer to a protein or polypeptide having an antigen-binding domain which comprises at least one complementarity determining region (CDR).

The term "complementarity determining region" or "CDR" generally refers to one of the 6 hypervariable regions within the variable domain of an antibody.

"Complementarity determining region" or "CDR" with regard to antigen-binding domain or antibody refers to a hypervariable region or a highly variable loop in the variable region of the heavy chain of the light chain of an antibody, which contribute primarily to antigen binding. CDRs can interact with the antigen conformation and largely determine binding to the antigen (although some framework regions are known to be involved in binding). The heavy chain variable region and the light chain variable region each contain 3 CDRs (heavy chain CDRs 1, 2 and 3 and light chain CDRs 1, 2 and 3, numbered from the amino to the carboxy terminus). Techniques for preparing and using various antibody-based constructs and fragments are well known in the art.

As used herein, "antigen binding site" or "antigen-binding domain" means a protein or polypeptide which comprises at least one complementarity determining region (CDR). The antigen binding site may comprise 3 CDRs, i.e. be equivalent to that of a single domain antibody (sdAb) domain such as a VHH domain.

As used herein, the term "constant immunoglobulin domain" may refer to a constant domain of an immunoglobulin, e.g. a CH3 domain.

In conventional, full-length antibodies (e.g. IgG antibodies) that comprise four polypeptides - two light chains and two heavy chains - the Fc region forms a homodimer of the CH2-CH3 domains of each heavy chain polypeptide.

As used herein, the term "fragment crystallisable (Fc) region" may refer to the Fc region of an immunoglobulin, which comprises the CH2-CH3 domains. Suitably, in embodiments of the invention the Fc region may be an IgG1, IgG2, IgG3, IgG4, IgA, IgE, IgM or IgD Fc region. Suitably, in embodiments of the invention the Fc region may be an IgG1, IgG2, IgG3 or IgG4 Fc region. In a preferred embodiment, the Fc region is an IgG1 Fc region. In the context of the present invention, the first polypeptide and the second polypeptide each comprise an Fc region of the same immunoglobulin isotype.

sdAbs are IgG molecules that are found naturally in e.g. camelids and sharks. Camelid sdAbs are devoid of the light chain and lack the first constant domain of the heavy chain (CH1) of conventional IgGs. Consequently, the antigen-binding fragment of sdAbs solely comprises a single variable domain, often referred to as a Variable Heavy domain of Heavy chain (VHH domain) (also referred to herein as a "single domain variable heavy immunoglobulin" domain). Thus, a VHH comprises 3 CDRs.

As used herein, the term "variable immunoglobulin domain" or "variable domain" may refer to a variable domain of an immunoglobulin, e.g. a VHH domain.

The immunoglobulin domains used in the invention are not limited to a specific sequence, and may comprise any suitable known immunoglobulin domains.

In some embodiments, the at least one variable domain of the first polypeptide and/or the second polypeptide is selected from the group consisting of a single-chain variable fragment (scFv); an Fab; an Fab'; an F(ab)'2; an Fv; a single domain antibody (sdAb); a VHH; a single chain variable domain; a designed ankyrin repeat protein (DARPin); and an aptamer.

In some embodiments, the at least one variable domain of the first polypeptide and/or the second polypeptide is selected from the group consisting of a single-chain variable fragment (scFv); an Fab; an Fv; a single domain antibody (sdAb); a VHH; a single chain variable domain; and a designed ankyrin repeat protein (DARPin).

In one embodiment, the at least one variable domain of the first polypeptide is a variable domain of the same type as the at least one variable domain of the second polypeptide. For example, the variable domain N-terminal of the Fc region of the first polypeptide may be a VHH and the variable domain N-terminal of the Fc region of the second polypeptide may be a VHH.

In one embodiment, the at least one variable domain of the first polypeptide is of a different type to the at least one variable domain of the second polypeptide. For example, the variable domain N-terminal of the Fc region of the first polypeptide may be an scFv and the variable domain N-terminal of the Fc region of the second polypeptide may be a VHH. Suitably, the antibody may be produced using CrossMAb technology (Schaefer et al., PNAS, 2011, 108: 11187-92; Takahashi et al., Cell, 1982, 29: 671-679; Grunert et al., ACS Omega 2022, 7, 4, 3671-3679; and Klein et al., mAbs, 2016, 8: 1010-1020).

In some embodiments, the antibody is selected from the group consisting of a full-length immunoglobulin, a scFv-Fc, a Fab-Fc, a Fab'-Fc, a F(ab)'2-Fc, an Fv-Fc, a sdAb-Fc, or a VHH-Fc.

In some embodiments, the antibody is selected from the group consisting of a full-length immunoglobulin, a scFv-Fc, a Fab-Fc, an Fv-Fc, a sdAb-Fc, or a VHH-Fc.

In some embodiments, the antibody is selected from the group consisting of a human antibody, a humanized antibody, a chimeric antibody, a multispecific antibody, a monoclonal antibody, and a polyclonal antibody.

The term "chimeric antibody" generally refers to an antibody obtained by fusing a variable region of a non-human antibody and a constant region of a human antibody, which can reduce an immune response induced by the non-human antibody. The non-human antibody may be, for example, a murine, camelid, rabbit, sheep, goat or chicken antibody. By way of example, for establishment of a chimeric antibody, a hybridoma secreting a specific monoclonal antibody can be established, and a variable region gene is cloned from the mouse hybridoma cells; then a constant region gene of human antibody can be cloned as required, and the mouse variable region gene and the human constant region gene are connected to form a chimeric gene; then the chimeric gene is inserted into an expression vector, wherein chimeric antibody molecules can be expressed in a eukaryotic system or a prokaryotic system.

The term "humanized antibody", also referred to as CDR-grafted antibody, generally refers to an antibody produced by grafting mouse CDR sequences into a human antibody variable region framework, i.e., an antibody produced in a different type of human germline antibody framework sequence. Therefore, the heterogeneous reaction induced by the presence of a large number of mouse protein components in the chimeric antibody can be overcome. Such framework sequences can be obtained from public DNA databases or disclosed references that include germline antibody gene sequences. For example, germline DNA sequences of human heavy and light chain variable region genes can be obtained from the "VBase" human germline sequence database.

The term "fully humanized antibody", "fully human antibody" or "completely human antibody", which may also be known as "fully humanized monoclonal antibody", may have both humanized variable region and constant region so as to eliminate immunogenicity and toxic side effects. The development of monoclonal antibodies has four stages, namely murine monoclonal antibodies, chimeric monoclonal antibodies, humanized monoclonal antibodies and fully humanized monoclonal antibodies. The antibodies or ligands described herein can be fully humanized monoclonal antibodies. Relevant technologies for the preparation of fully human antibodies may be: human hybridoma technology, EBV-transformed B-lymphocyte technology, phage display technology, transgenic mouse antibody preparation technology, single B-cell antibody preparation technology, and the like.

A "monoclonal antibody" refers to an antibody obtained from a substantially homogeneous population of antibodies, i.e., the antibodies composing the population are identical except for possible naturally occurring mutations that may be present in minor amounts. A monoclonal antibody is highly specific and targets a single antigen epitope. In contrast, conventional (polyclonal) antibody preparations typically include a large number of antibodies targeting (or specific for) different epitopes. The modifier "monoclonal" indicates the characteristic of an antibody obtained from a substantially homogeneous population of antibodies, and is not to be construed as producing the antibody by any particular method.

In some embodiments, the sequence of the antibody may be defined using Kabat numbering (Kabat E.A. et al., (1991)).

Antibodies may be obtained by techniques comprising immunizing an animal with a target antigen and isolating the antibody from serum. Monoclonal antibodies may be made by the hybridoma method first described by Kohler et al., Nature 256:495 (1975), or may be made by recombinant DNA methods (see, e.g., U.S. Pat. No. 4,816,567). Monoclonal antibodies may also be isolated from phage antibody libraries using the techniques described in Clackson et al., Nature 352:624-628 (1991) and Marks et al., J. Mol. Biol. 222:581-597 (1991), for example.

In an embodiment, according to the number of antigen-binding domains, e.g. VHH domains, that are present, the antibody for use according to the present invention may be monovalent, bivalent, trivalent, tetravalent or pentavalent. Similarly, in an embodiment, according to the number of different antigen-binding domains, e.g. VHH domains, that are present, the antibody for use according to the invention may be monospecific or multispecific, e.g., bispecific, trispecific, tetraspecific etc.

Any suitable multispecific or multivalent antibody format which is known in the art may be used in the practice of the present invention. Suitably, the bispecific antibody may be an IgG-scFv, IgG-sdAb, IgG-VHH, scFv-Fc-scFv, KiH-IgG, κλ-body, KiH-Fc-Fab/scFv.

The variable domains (e.g. VHH domains) used in the invention are not particularly limiting, and may comprise any antigen binding site that is specific for a selected target protein. Methods for determining binding specificity of an antibody to a particular antigen are known in the art include, but are not limited to, by biolayer interferometry (BLI), surface plasmon resonance (SPR) analysis (e.g. using a BIAcore instrument), ELISA, western blot, *in situ* hybridisation, immunohistochemistry, flow cytometry, Förster resonance energy transfer

(FRET), phage display libraries, yeast two-hybrid screens, co-immunoprecipitation, bimolecular fluorescence complementation and tandem affinity purification. Binding affinity can also be determined using methods such as BLI, SPR analysis (e.g. using a BIAcore instrument), flow cytometry, fluorescence quenching, isothermal titration calorimetry.

Methods for providing variable domains, such as sdAbs (which comprise VHH domains), against a specific target are known in the art (see Caussinus et al.; Nat Struct Mol Biol; 2011; 19(1); 117-121 & Fulcher et al.; Open Biol; 2016; 6(10); pii 160255). Further, methods to isolate antigen-specific VHHs from immune or semisynthetic libraries using phage, yeast, or ribosome display are established in the art (see Muyldermans J Biotechnol. 2001 Jun; 74(4):277-302. & Dufner et al. Trends Biotechnol. 2006 Nov; 24(11):523-9).

By way of example, a VHH can be obtained by immunisation of e.g. dromedaries, camels, llamas or alpacas with the desired antigen and subsequent isolation of the mRNA coding for VHHs. Single domain shark variable domain of new antigen receptor (VNAR) antibodies are also known and suitable for use according to the present invention as an alternative sdAb to a VHH domain. Hence, by way of further example, a VNAR can be obtained by immunisation of sharks with the desired antigen and subsequent isolation of the mRNA coding for VNARs. Reverse transcription and PCR can then be used to generate a library of VHHs or VNARs. Standard screening techniques such as phage display and ribosome display may be used to identify the suitable clones binding the antigen of interest.

Once the most potent clones have been identified, their DNA sequence may be optimized, for example to improve their stability towards enzymes. Humanisation may also be performed.

VHHs and VNARs may be expressed in a cell using conventional vectors, such as those described herein.

The ability of the antibody to specifically bind its target may be determined using methods which are known in the art. For example, determination of binding may be performed e.g. by biolayer interferometry (BLI), surface plasmon resonance (SPR) analysis (using a BIAcore instrument), western blot, flow cytometry, *in situ* hybridisation and/or microscopy. Suitably, determination of binding affinity may be performed by e.g. by biolayer interferometry (BLI), surface plasmon resonance (SPR) analysis (using a BIAcore instrument) and/or flow cytometry. Suitably, determination of binding affinity may be performed as described herein (see Example 3).

In one embodiment, variable domains of the first and the second polypeptide have the same binding specificity.

In one embodiment, the variable domains of the first and the second polypeptide have different binding specificities.

In an embodiment, the variable domain(s) (e.g. VHH domain(s)) of the first polypeptide are specific for a first target and the variable domain(s) (e.g. VHH domain(s)) of the second polypeptide are specific for a second target. In a preferred embodiment, the first and second targets are different. In an embodiment, accordingly, the antibody comprising the first and second polypeptides is a heterodimer. In an alternative embodiment, the variable domain(s) (e.g. VHH domain(s)) of the first polypeptide are specific for the same target as the variable domain(s) (e.g. VHH domain(s)) of the second polypeptide. In an embodiment, accordingly, the antibody comprising the first and second polypeptides is a homodimer.

In an embodiment, the first polypeptide further comprises at least one binding moiety or a cognate of a binding moiety C-terminal of the Fc region. In an embodiment, the at least one binding moiety or cognate is immediately C-terminal of the Fc region. Suitably, the first polypeptide further comprises a binding moiety C-terminal of the Fc region. Suitably, the first polypeptide further comprises a cognate of a binding moiety C-terminal of the Fc region.

In an embodiment, the second polypeptide further comprises at least one binding moiety or a cognate of a binding moiety C-terminal of the Fc region. In an embodiment, the at least one binding moiety or cognate is immediately C-terminal of the Fc region. Suitably, the second polypeptide further comprises a binding moiety C-terminal of the Fc region. Suitably, the second polypeptide further comprises a cognate of a binding moiety C-terminal of the Fc region.

In an embodiment, the first polypeptide further comprises a binding moiety or a cognate of a binding moiety C-terminal of the Fc region of the first polypeptide and the second polypeptide further comprises a binding moiety or a cognate of a binding moiety C-terminal of the Fc region of the second polypeptide. In an embodiment, each of the binding moieties or cognates is immediately C-terminal of the Fc region of the respective polypeptide sequence. Suitably, the each of the first polypeptide and the second polypeptide further comprise a binding moiety C-terminal of the Fc region of the respective polypeptide. Suitably, each of the first polypeptide and the second polypeptide further comprise a cognate of a binding moiety C-terminal of the Fc region of the respective polypeptide. Suitably, the first polypeptide further comprises a binding moiety C-terminal of the Fc region of the first polypeptide and the second polypeptide further comprises a cognate of a binding moiety C-terminal of the Fc region of the second polypeptide. Suitably, the first polypeptide further comprises a cognate of a binding moiety C-terminal of the Fc region of the first polypeptide and the second polypeptide further comprises a binding moiety C-terminal of the Fc region of the second polypeptide.

By "N-terminal of the Fc region" it is meant that the variable domain or engineered IgG hinge region is located N-terminal of the Fc region by any means known in the art. In an embodiment, the engineered IgG hinge region is fused directly to the N-terminus of the Fc region, i.e. the engineered IgG hinge region may be immediately adjacent to the Fc region. However, in an embodiment, the engineered IgG hinge region is connected to the N-terminus of the Fc region by a linking sequence as described herein. The variable domain is connected to the N-terminus of the Fc region by an engineered IgG hinge sequence as described herein.

By "N-terminal of the engineered IgG hinge region" it is meant that the variable domain is located N-terminal of the engineered IgG hinge region by any means known in the art. In an embodiment, the variable domain is fused directly to the N-terminus of the engineered IgG hinge region, i.e. the variable domain may be immediately adjacent to the engineered IgG hinge region. However, in an embodiment, the variable domain is connected to the N-terminus of the engineered IgG hinge region by a linking sequence as described herein.

By "C-terminal of the Fc region" it is meant that the binding moiety or cognate of a binding moiety is located C-terminal of the Fc region by any means known in the art. In an embodiment, the binding moiety or cognate of a binding moiety is fused directly to the C-terminus of the Fc region, i.e. the binding moiety or cognate of a binding moiety may be immediately adjacent to the Fc region. However, in an embodiment, the binding moiety or cognate of a binding moiety is connected to the C-terminus of the Fc region by a linking sequence as described herein.

The linking sequence of the first and/or second polypeptide may comprise one or more linkers (e.g. glycine-serine (GS) linkers), such as those linkers that are widely known in the art. In an embodiment, the linking sequence comprises a GS linker. In an embodiment, the linking sequence consists of a GS linker. In an embodiment, the linking sequence is GS. In a preferred embodiment, the linking sequence is devoid of cysteine residues. Thus, the linking sequences of the first and second polypeptides may not form any inter-chain disulphide bonds. Preferably, the linking sequences of the first and second polypeptides do not form inter-domain disulphide bonds.

The binding moiety of the first and/or second polypeptide may be any suitable binding entity which is capable of specifically binding to a target, such as a target polypeptide sequence. Numerous binding moieties are known in the art, including those based on the antigen binding site of an antibody, antibody mimetics, and T-cell receptors. For example, the binding moiety may comprise: a variable immunoglobulin domain; an antigen binding site of an antibody; a single-chain variable fragment (scFv); a Fab; an Fv; a single domain antibody (sdAb); a VHH; a single chain variable domain (which may be a VH or VL chain, having 3 CDRs); or an artificial single binder such as a Darpin (designed ankyrin repeat protein). In a preferred embodiment, the binding moiety is an antigen-binding fragment of an antibody. In a preferred embodiment, the antigen-binding fragment of an antibody is a VHH.

The cognate of a binding moiety may be any motif that is specifically recognised by a binding moiety. Numerous cognates of binding moieties are provided in the art. In an embodiment, the cognate is an antigen, an epitope or a polypeptide tag that is specifically recognised by an antibody or an antigen-binding fragment of an antibody. In a preferred embodiment, the cognate is a polypeptide tag. In a preferred embodiment, the cognate is a polypeptide tag that is specifically recognised by an antigen-binding fragment of an antibody.

In an embodiment, the binding moiety that is C-terminal of the Fc region of the first polypeptide and/or the second polypeptide is preferably a VHH domain and is specific for a third target. In an embodiment, the third target is different to the first and second targets. In an embodiment, the binding moieties that are C-terminal of the Fc region of each of the first polypeptide and the second polypeptide are preferably VHH domains and are specific for a third target and a fourth target. In an embodiment, each of the first, second, third and fourth targets are different.

In an embodiment, the cognate of a binding moiety that is C-terminal of the Fc region of the first polypeptide and/or the second polypeptide is not specifically recognised by any of the variable domains (e.g. VHH domains) and binding moieties, where present, of the first and second polypeptides.

In an embodiment, a binding moiety or a variable domain (e.g. a VHH) "specific for" a target or "specifically recognising" a target refers to binding to the target with an affinity equivalent to that of a functional antibody fragment. In an embodiment, the targets are target antigens.

In an embodiment in which the first and/or second polypeptides contain a plurality of variable domains and/or binding moieties (e.g. VHH domains), each variable domain and/or binding moiety (e.g. VHH domain) in the same polypeptide may be specific for the same target. In an embodiment in which the first and/or second polypeptides contain a plurality of variable domains and/or binding moieties (e.g. VHH domains), each variable domain and/or binding moiety (e.g. VHH domain) in the same polypeptide may be specific for a different target.

Any suitable Fc region which is known in the art may be employed in the practice of the present invention. In some embodiments, one or more amino acid modifications may be introduced into an Fc region of an antibody provided herein, thus producing an Fc region variant. The Fc region variant may comprise a human Fc region sequence (such as the human IgG1, IgG2, IgG3, or IgG4 Fc region) comprising an amino acid modification (such as substitution) at one or more amino acid positions. For example, Fc engineering for modulated effector functions is known in the art. Fc modifications include, but are not limited to, silencing effector functions, elevating effector functions, and elongating or shortening half-life. A review of Fc modifications is provided by Liu et al. (Liu et al., Antibodies, 2020, 9: 64).

In an embodiment, the first and second polypeptides comprise modifications that enhance the formation of a dimer, preferably a heterodimer, comprising one monomer of the first polypeptide and one monomer of the second polypeptide. In an embodiment, the modifications can be those modifications which are widely known in the art of antibody technology for the purpose of (hetero)dimerisation between antibody chains (e.g. "knobs-into-holes" (KiH) modifications). In a typical embodiment, the modifications are present in the CH3 domains of the first and second polypeptides.

In one embodiment, one or more of the CH3 domains of the first polypeptide and/or one or more of the CH3 domains of the second polypeptide comprise a modification that enhances the formation of the dimer comprising the first and second polypeptide. In an embodiment wherein the first and second polypeptides each comprise more than one CH3 domain, all of the CH3 domains may comprise such modifications.

As used herein, the term "enhances the formation of the dimer comprising the first and second polypeptide" includes promoting dimerisation of the first and second polypeptides. Thus, in an embodiment, the modifications promote dimerisation of the first and second polypeptides. In an embodiment, the modifications promote heterodimerisation of the first and second polypeptides. By heterodimerisation it is meant that the first polypeptide is different to the second polypeptide, and the first polypeptide will form a dimer with the second polypeptide, but the first polypeptide will not form a dimer with the first polypeptide and the second polypeptide will not form a dimer with the second polypeptide. In an embodiment, the modifications promote homodimerisation of the first and second polypeptides. By homodimerisation it is meant that the first polypeptide is the same as the second polypeptide, and the first polypeptide will form a dimer with the second polypeptide.

Various means of promoting dimerisation of two domains (e.g. homo- or hetero-dimerisation) are known in the art. In an embodiment, the two domains may comprise a modification that enhances formation of dimer, e.g. a modification that increases the affinity of the two domains, promotes and/or enables the formation of one or more disulphide bonds, reduces steric hindrance to the dimerisation, promotes electrostatic and/or hydrophilic/hydrophobic interactions between the two domains, or any combination thereof.

Any suitable technology for promoting the dimerisation of Fc regions (e.g. CH3 domains), i.e. of at least one of the CH3 domains of the first polypeptide and at least one of the CH3 domains of the second polypeptide, may be employed in the practice of the invention.

Preferably, the modification that enhances the formation of a dimer between the Fc regions (e.g. CH3 domains) is KiH technology (Ridgeway et al., Protein Engineering, Design and Selection, 1996, 9: 617-621 and Merchant et al., Nat Biotechnol, 1998, 16: 677-681). The KiH technology is based on an engineered pair of CH3 domains that heterodimerises (CH3 heterodimer) in which asymmetric hydrophobic mutations are introduced between the homodimeric CH3 domain. KiH involves introducing mutations that create a protuberance ("knob") in the interface of the first CH3 domain and a corresponding cavity ("hole") in the interface of the second CH3 domain, such that the protuberance can be positioned in the cavity to promote heterodimer assembly and hinder homodimer formation. KiH variants therefore thermodynamically favour the formation of heterodimers rather than homodimers.

By way of further example, the present invention may employ: the strand-exchange engineered domain (SEED) CH3 dimers (Davis et al., Protein Eng Des Sel., 2010, 23:195-202); electrostatic steering employing DD-KK variants with asymmetric electrostatic interactions (Gunasekaran et al., J Biol Chem., 2010 , 285: 19637-46): Azymetric technologies by Zymeworks (https://www.zvmeworks.com/technologies/azymetric/): Bispecific Engagement by Antibodies based on the T-cell receptor (BEAT) (Skegro et al., J Biol Chem., 2017, 292: 9745-9759): Fast-Ig and ART-Ig (https://www.chugai-pharm.co.ip/english/profile/rd/technologies.html): DEKK dimerisation technology (https://merus.nl/technology/multiclonics-platform/ and Nardis et al, J Biol Chem., 2017, 292: 14706-14717): HA-TF variants with asymmetric hydrophobic interactions (Moore et al., MAbs, 2011, 3: 546-57): computationally designed CH3 interfaces, such as the 7.8.60 design (Leaver-Fay et al., Structure, 2016, 24: 641-651): EW-RVT variants, which were designed to replace the conserved electrostatic interactions with asymmetric hydrophobic interactions and to add asymmetric long-range electrostatic interactions at the rim of the heterodimeric CH3 interface (Choi et al., Mol Cancer Ther., 2013, 12: 2748-59): and the K370E_{CH3A}-E357N_{CH3B} mutations employed in the "A107" variant which replaced the homodimer-favouring electrostatic interactions with heterodimer-stabilizing hydrogen bonds.

Any suitable KiH modifications known in the art may be employed in the practice of the present invention. For example, the T366W ("knob") modification and the T366S, L368A and Y407V ("hole") modifications may be used (according to EU residue numbering (Edelman et al., Proc Natl Acad Sci U S A., 1969, 63: 78-85)).

The variable domains (e.g. VHH domains) used in the invention are not particularly limiting, and may comprise any antigen binding site that is specific for a selected target protein. The selected target protein may be a cancer antigen, an immune cell marker, or a non-human cell antigen.

In some embodiments, the at least one variable domain of the first polypeptide, the second polypeptide or both the first polypeptide and the second polypeptide is specific for a cancer antigen.

Various tumour associated antigens (TAA) are known in the art. In an embodiment, the variable domains (e.g. VHH domains) of the present invention are capable of specifically binding to a TAA.

In some embodiments, the at least one variable domain of the first polypeptide, the second polypeptide or both the first polypeptide and the second polypeptide is specific for an immune cell marker. Various immune cell markers are known in the art. For example, anti-CD163 ADCs that target macrophages are known in the art (Liu et al., Expert Opinion on Biological Therapy, 2016, 16: 591-593). By way of further example, ADCs targeting CXCR4 are known in the art. CXCR4 is highly expressed on T cells, B-cells, and monocytes, as well as hematopoietic stem cells, with minimal to no expression on non-hematopoietic cells (Liu et al., Expert Opinion on Biological Therapy, 2016, 16: 591-593).

Moreover, ADCs targeting non-human cells are known in the art. For example, an antibody-antibiotic conjugate (AAC) targeting intracellular *Streptococcus aureus* has been reported (Liu et al., Expert Opinion on Biological Therapy, 2016, 16: 591-593). ***Drug***

The drug to which the antibody is conjugated may also be referred to as a "cargo" or "payload". The payload used in the invention is not particularly limiting, and may comprise any therapeutic or diagnostic agent that can be conjugated to an antibody. In addition to using synthetic cytotoxins, coupling of other payloads with site-specific antibody conjugation has also been reported. These other payloads include non-cytotoxic compounds, such as proteins/peptides, glycans, lipids, and nucleic acids.

Suitably, the payload may be a small molecule, an oligonucleotide (e.g. RNA or DNA), a peptide, a dye (e.g. IRDye 700DX), a cytotoxic drug, a chemotherapeutic drug, an antimicrobial agent (such as an antibiotic), a protein degrader, an enzyme inhibitor, a protein ligand, or an immunomodulatory agent. Suitable payloads are known in the art (see, for example, Liu et al., Expert Opinion on Biological Therapy, 2016, 16: 591-593; and Zhijia Wang et al., Acta Pharmaceutica Sinica B, 2023,13: 4025-4059).

In some embodiments, the drug is selected from the group consisting of a cytotoxic drug, a chemotherapeutic drug, an antimicrobial agent (such as an antibiotic), or an immunomodulatory agent.

The term cytotoxic or chemotherapeutic drug refers to a drug that reduces or eliminates the viability of a cell. Suitable cytotoxic or chemotherapeutic drugs will be known in the art.

The term "cytotoxic drug" generally refers to a toxic drug. Suitably, the cytotoxic drug may be a chemical molecule that is potent enough to disrupt the normal growth of a tumour cell exposed thereto. Cytotoxic drugs can kill tumour cells at a sufficiently high concentration. The "cytotoxic drug" may include toxins, such as small molecule toxins or enzymatically active toxins of bacterial, fungal, plant or animal origin, radioisotopes, toxic drugs, chemotherapeutic drugs, antibiotics and nucleolytic enzymes.

For example, the cytotoxic drug may be selected from the group consisting of Auristatins, Maytansinoids, Tubulysins, Taxoids, Calicheamicins, Amatoxins, Pyrrolobenzodiazepines, Camptothecins and derivatives thereof. Suitably, the cytotoxic drug may be MMAE, MMAF, DM1, DM4, duocarmycin, Dxd, SN38, exatecan, doxorubicin, SG3199, or Taxol.

### Method of Producing an Antibody Drug Conjugate

In a further aspect, the present invention provides a method of producing an antibody-drug conjugate (ADC) having a predetermined drug-to-antibody ratio (DAR), comprising the steps of:
(i) providing an antibody as defined herein;
(ii) reducing the antibody with a reducing agent; and
(iii) conjugating the reduced antibody with the drug.

In a further aspect, the present invention provides a method of producing an antibody-drug conjugate (ADC) having a predetermined drug-to-antibody ratio (DAR), comprising the steps of:
(i)
   (a) providing at least one polynucleotide sequence encoding an antibody comprising a first polypeptide comprising an immunoglobulin fragment crystallisable (Fc) region and at least one variable domain N-terminal of the Fc region;
   (b) modifying the at least one polynucleotide sequence to encode an engineered IgG hinge region between the Fc region and the at least one variable domain, wherein the engineered IgG hinge region is heterologous to the Fc region and/or is mutated to provide a predetermined number of cysteine residues;
   (c) introducing the at least one polynucleotide sequence into a cell;
   (d) culturing the cell under conditions suitable for the expression of the at least one polynucleotide sequence;
   (e) isolating the antibody expressed by the cell;
(ii) reducing the antibody with a reducing agent; and
(iii) conjugating the reduced antibody with the drug.

In some embodiments, the antibody further comprises a second polypeptide comprising an Fc region, and step (i)(b) further comprises modifying the at least one polynucleotide sequence to encode an engineered IgG hinge region N-terminal of the Fc region of the second polypeptide, wherein the engineered IgG hinge region is heterologous to the Fc region of the second polypeptide and/or is mutated to provide a predetermined number of cysteine residues, and wherein the engineered IgG hinge region of the first polypeptide and the engineered IgG hinge region of the second polypeptide are complimentary engineered IgG hinge regions.

In some embodiments, the second polypeptide further comprises at least one variable domain N-terminal of the engineered IgG hinge region.

In a further aspect, the present invention provides a method of producing an antibody-drug conjugate (ADC) having a predetermined drug-to-antibody ratio (DAR), comprising the steps of:
(i)
   (a) providing at least one polynucleotide sequence encoding an antibody wherein the antibody comprises a first polypeptide and a second polypeptide, wherein each of the first polypeptide and the second polypeptide comprises an immunoglobulin fragment crystallisable (Fc) region and at least one variable domain which is a VHH N-terminal of the Fc region;
   (b) modifying the at least one polynucleotide sequence to encode an engineered IgG hinge region between the Fc region and the at least one variable domain of each of the first polypeptide and the second polypeptide, wherein the engineered IgG hinge region of the first polypeptide and the engineered IgG hinge region of the second polypeptide are complimentary engineered IgG hinge regions, and wherein the engineered IgG hinge region is heterologous to the Fc region and/or is mutated to provide a predetermined number of cysteine residues;
   (c) introducing the at least one polynucleotide sequence into a cell;
   (d) culturing the cell under conditions suitable for the expression of the at least one polynucleotide sequence;
   (e) isolating the antibody expressed by the cell;
(ii) reducing the antibody with a reducing agent; and
(iii) conjugating the reduced antibody with the drug.

The at least one polynucleotide sequence may be modified to encode an engineered IgG hinge region by introducing a nucleic acid sequence encoding an engineered IgG hinge region into the at least one polynucleotide sequence. Alternatively, the at least one polynucleotide sequence may be modified to mutate a native IgG hinge region to provide an engineered IgG hinge region in accordance with the invention as described herein.

The modification of the at least one polynucleotide sequence to encode an engineered IgG hinge region according to the invention using conventional techniques in molecular biology is within the capabilities of a person of ordinary skill in the art. Generally speaking, suitable routine methods include directed mutagenesis, gene synthesis and recombinant DNA/RNA technology.

The introduction of at least one polynucleotide sequence encoding a component of the present invention into a cell using conventional molecular and cell biology techniques is within the capabilities of a person of ordinary skill in the art. For example, a vector or an expression cassette could be used.

A vector is a tool that allows or facilitates the transfer of an entity from one environment to another. In accordance with the present invention, and by way of example, some vectors used in recombinant nucleic acid techniques allow entities, such as a segment of nucleic acid (e.g. a heterologous DNA segment, such as a heterologous cDNA segment), to be transferred into and expressed by a target cell. The vector may facilitate the integration of the nucleotide sequence(s) encoding the antibody of the invention to maintain the nucleotide sequence encoding the antibody of the invention and its expression within the target cell. Expression cassettes as described herein comprise regions of nucleic acid containing sequences capable of being transcribed.

The cysteine-based site-specific conjugation may be performed using any suitable method known in the art and as described herein.

In some embodiments, the step of reducing the antibody in step (ii) is a partial reduction of the antibody. Suitably, cysteine-based site-specific conjugation strategies involve partial reduction of the antibody to reduce the inter-chain disulphide bonds of the antibody. This directed reduction of the antibody enables the control of the DAR of the product, i.e. by selectively reducing the inter-chain disulphide bonds of the antibody conjugation of the antibody to the linker-drug will occur via the free cysteine residues generated in the partial reduction step. This assists in the production of a homogenous ADC product with a uniform DAR.

In some embodiments, the step of reducing the antibody in step (ii) is a partial reduction of the antibody to reduce the inter-chain disulphide bonds of the antibody.

In some embodiments, the reducing agent is dithiothreitol (DTT) or tris (2-carboxyethyl) phosphine (TCEP).

In some embodiments, the drug is in the form of a linker-drug conjugate comprising a thiol-reactive group. The linker may be as described herein.

In some embodiments, the linker comprises a thiol-reactive group selected from the group consisting of a maleimide, bromoacetamide, disulphide, α-haloacetamide, α-halocarbonyl, vinylsulfone, heteroaryl sulfone, thiosulfonate, electron deficient aryl halide, ethynylphosphonamidate, vinylphosphonite, palladiumoxidative-addition complex, bis-sulfone, water-soluble allyl sulfone, thiol-yne bioconjugation with terminal alkyne or cyclooctyne, dibromo- (DBM) and dithio-maleimide (DTM), hybrid thiobromomaleimide (TBM), dibromopyridazinediones, divinylpyrimidine, and DiPODS (two oxadiazolyl methyl sulfone moieties connected by a phenyl group).

In some embodiments, the thiol-reactive group is selected from the group consisting of a maleimide, bromoacetamide, disulphide, α-haloacetamide, α-halocarbonyl, vinylsulfone, heteroaryl sulfone, thiosulfonate, electron deficient aryl halide, ethynylphosphonamidate, vinylphosphonite, palladiumoxidative-addition complex.

In some embodiments, step (iii) is performed using a thiol-reactive coupling strategy.

In some embodiments, the thiol-reactive coupling strategy is maleimide-based conjugation.

In some embodiments, the method of the invention provides homogenous ADCs.

In some embodiments, all of the cysteine residues within the engineered IgG hinge region form stable inter-chain disulphide bonds prior to step (ii).

In some embodiments, in step (iii) the antibody is conjugated to the drug by cysteine-based site-specific conjugation through all of the cysteine residues within the engineered IgG hinge region.

In a further aspect, the present invention provides an ADC obtained or obtainable by the methods of the invention.

### Pharmaceutical composition

In a further aspect, the present invention provides a pharmaceutical composition comprising the ADC according to the invention.

The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

Accordingly, in a further aspect, the present invention provides a pharmaceutical composition comprising the ADC according to the invention and a pharmaceutically acceptable carrier, excipient and/or diluent.

### Therapeutic use

In a further aspect, the present invention provides an ADC of the invention or a pharmaceutical composition of the invention for use in therapy.

In a further aspect, the invention provides an ADC of the invention or a pharmaceutical composition of the invention for use in a method of therapy or a diagnostic method.

In a further aspect, the invention provides the use of an ADC of the invention or a pharmaceutical composition of the invention for the manufacture of a medicament or diagnostic agent.

In a further aspect, the invention provides a method of therapy or a diagnostic method comprising administering the ADC of the invention or pharmaceutical composition of the invention to a subject.

A method for therapy includes a method for treating a disease as well as a method for preventing a disease.

A method for treating a disease relates to the therapeutic use of an ADC or pharmaceutical composition of the invention. In this respect, the ADC or pharmaceutical composition according to the invention may be administered to a subject having an existing disease or condition in order to lessen, reduce or improve at least one symptom associated with the disease and/or to slow down, reduce or block the progression of the disease.

The method for preventing a disease relates to the prophylactic use of an ADC or pharmaceutical composition of the invention. In this respect, the ADC or pharmaceutical composition according to the invention may be administered to a subject who has not yet contracted the disease and/or who is not showing any symptoms of the disease to prevent or impair the cause of the disease or to reduce or prevent development of at least one symptom associated with the disease. The subject may have a predisposition for, or be thought to be at risk of developing, the disease.

The disease to be treated and/or prevented may be cancer.

Suitably, the cancer may be a solid tumour or a liquid tumour.

The cancer may be a cancer such as neuroblastoma, prostate cancer, bladder cancer, breast cancer, colon cancer, endometrial cancer, kidney cancer (renal cell), leukaemia, lung cancer, melanoma, non-Hodgkin lymphoma, pancreatic cancer, and thyroid cancer.

In a further aspect, the present invention provides an ADC of the invention, or a pharmaceutical composition of the invention for use in the treatment of a disease or condition selected from the group consisting of cancer, autoimmune disease, infection, infectious diseases, cardiovascular diseases and liver metabolic disorders.

The therapeutic or diagnostic use an ADCs is well-known in the art (see, for example, Liu et al., Expert Opinion on Biological Therapy, 2016, 16: 591-593; and Zhijia Wang et al., Acta Pharmaceutica Sinica B, 2023,13: 4025-4059).

In some embodiments, the subject of the medical uses and methods of treatment according to the present invention may be a mammal.

In some embodiments, the subject may be a human.

In some embodiments, the subject may alternatively be a non-human mammal, including for example, a primate, a monkey, a dog, a cat, a horse, a cow, a sheep, a pig, a rabbit, a rat, or a mouse.

In some embodiments, the subject may be a patient, such as a human patient.

In some embodiments, the subject may suffer from and/or have been diagnosed with one or more cancer(s).

In some embodiments, the subject may suffer from and/or have been diagnosed with an autoimmune disease, infection, infectious diseases, cardiovascular diseases or liver metabolic disorders.

### Nucleic acid sequences and particles

In a further aspect, the invention provides one or more nucleic acid sequences capable of expressing the antibody for use according to the invention.

In a further aspect, the invention provides one or more nucleic acid sequences capable of expressing the first polypeptide sequence as defined herein.

In a further aspect, the invention provides one or more nucleic acid sequences capable of expressing the second polypeptide sequence as defined herein.

In a further aspect, the invention provides one or more nucleic acid sequences capable of expressing the first polypeptide sequence and the second polypeptide sequence as defined herein.

According to the present disclosure, terms such as "capable of expressing", "nucleic acid expressing" and "nucleic acid encoding" or similar terms are used interchangeably herein and with respect to a particular peptide or polypeptide mean that the nucleic acid, if present in the appropriate environment, e.g. within a cell, can be expressed to produce said peptide or polypeptide.

Suitably, the nucleic acid sequences capable of expressing the antibody for use according to the invention may comprise a plurality of nucleic acid sequences which encode components of the construct such as the first polypeptide, second polypeptide, Fc region, variable domain, VHH domain, engineered IgG hinge region and/or linking sequence as described herein.

It will be understood by the skilled person that numerous different polynucleotides and nucleic acids can encode the same polypeptide as a result of the degeneracy of the genetic code. In addition, it is to be understood that skilled persons may, using routine techniques, make nucleotide substitutions that do not affect the polypeptide sequence encoded by the polynucleotides described herein to reflect the codon usage of any particular host organism in which the polypeptides are to be expressed. Suitably, the polynucleotides of the present invention are codon optimised to enable expression in a mammalian cell, in particular a cell as described herein.

Nucleic acids according to the invention may comprise DNA and/or RNA. The nucleic acids may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. Herein, it is to be understood that the polynucleotides may be modified by any method available in the art. Such modifications may be carried out in order to enhance the in vivo activity or life span of polynucleotides of interest.

In a further aspect, the present invention provides a vector comprising the one or more nucleic acid sequences of the present invention.

In an embodiment, the vector comprises a plurality of nucleic acid sequences which encode different components as provided by the present invention. For example, in an embodiment the vector comprises a first nucleic acid sequence which encodes the first polypeptide and a second nucleic acid sequence which encodes the second polypeptide of the invention.

In embodiments, the vector may, for example, be a plasmid or a viral vector, such as a retroviral vector or a lentiviral vector, or a transposon based vector or synthetic mRNA.

In an embodiment, the vector is capable of transfecting or transducing a cell.

The one or more nucleic acid sequences of the present invention may be present in particles comprising (i) the one or more nucleic acid sequences and (ii) at least one cationic or cationically ionizable compound such as a polymer or lipid complexing the one or more nucleic acid sequences. Electrostatic interactions between positively charged molecules such as polymers and lipids and negatively charged nucleic acid are involved in particle formation. This results in complexation and spontaneous formation of nucleic acid particles comprising the one or more nucleic acid sequences.

Accordingly, in a further aspect, the invention provides a nucleic acid particle comprising the nucleic acid sequence(s) according to the invention.

Different types of nucleic acid containing particles have been described previously to be suitable for delivery of nucleic acid (e.g. RNA) in particulate form (cf., e.g., Kaczmarek, J. C. et al., 2017, Genome Medicine 9, 60). For non-viral delivery vehicles, nanoparticle encapsulation of nucleic acids physically protects nucleic acids from degradation and, depending on the specific chemistry, can aid in cellular uptake and endosomal escape.

In the context of the present disclosure, the term "particle" relates to a structured entity formed by molecules or molecule complexes, in particular particle forming compounds. In some embodiments, the particle contains an envelope (e.g., one or more layers or lamellas) made of one or more types of amphiphilic substances (e.g., amphiphilic lipids). In this context, the expression "amphiphilic substance" means that the substance possesses both hydrophilic and lipophilic properties. The envelope may also comprise additional substances (e.g., additional lipids) which do not have to be amphiphilic. In some embodiments, the term "particle" relates to a micro- or nano-sized structure, such as a micro- or nano-sized compact structure. According to the present disclosure, the term "particle" includes nanoparticles.

Nucleic acid particles (such as RNA particles and/or DNA particles) include lipid nanoparticle (LNP)-based and lipoplex (LPX)-based formulations.

In general, a lipoplex (LPX) is obtainable from mixing two aqueous phases, namely a phase comprising nucleic acid (such as RNA and/or DNA) and a phase comprising a dispersion of lipids. In some embodiments, the lipid phase comprises liposomes.

In general, a lipid nanoparticle (LNP) is obtainable from direct mixing of nucleic acid (such as RNA and/or DNA) in an aqueous phase with lipids in a phase comprising an organic solvent, such as ethanol. In that case, lipids or lipid mixtures can be used for particle formation, which do not form lamellar (bilayer) phases in water.

In some embodiments, LNPs comprise or consist of a cationic/ionisable lipid and helper lipids such as phospholipids, cholesterol, and/or polyethylene glycol (PEG) lipids. In some embodiments, in the nucleic acid LNPs (such as DNA LNPs) described herein the nucleic acid (such as DNA) is bound by ionisable lipid that occupies the central core of the LNP. In some embodiments, PEG lipid forms the surface of the LNP, along with phospholipids. In some embodiments, the surface comprises a bilayer. In some embodiments, cholesterol and ionisable lipid in charged and uncharged forms can be distributed throughout the LNP.

In some embodiments, nucleic acid (such as RNA and/or DNA, e.g., mRNA) may be noncovalently associated with a particle as described herein. In embodiments, the nucleic acid (such as RNA and/or DNA, especially mRNA) may be adhered to the outer surface of the particle (surface nucleic acid) and/or may be contained in the particle (encapsulated nucleic acid (such as encapsulated DNA).

In a further aspect, the invention provides a cell comprising the nucleic acid sequence(s) or nucleic acid particle according to the invention.

### General definitions

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino (N) to carboxy (C) orientation, respectively.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

The term "polypeptide" is used in the conventional sense to mean a series of amino acids, typically L-amino acids, connected one to the other, typically by peptide bonds between the α-amino and carboxyl groups of adjacent amino acids. The term "polypeptide" is used interchangeably with the terms "amino acid sequence", "peptide" and/or "protein". The term "residues" is used to refer to amino acids in an amino acid sequence.

The term "variant" in relation to a polypeptide refers to a polypeptide that has an equivalent function to the amino acid sequences described herein, but which includes one or more amino acid substitutions, insertions or deletions.

As used herein, the terms "polynucleotide", "nucleotide", "nucleic acid sequence" and "nucleic acid" are intended to be synonymous with each other.

The terms "variant", "homologue" or "derivative" in relation to a nucleotide sequence include any substitution of, variation of, modification of, replacement of, deletion of or addition of one (or more) nucleic acid from or to the sequence.

"Sequence identity" between two nucleic acid sequences indicates the percentage of nucleotides that are identical between the sequences. The terms "% identical" and "% identity" or similar terms are intended to refer, in particular, to the percentage of nucleotides or amino acids which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to identify local regions of corresponding sequences. The optimal alignment for a comparison may be carried out manually or with the aid of the local homology algorithm by Smith and Waterman, 1981, Ads App. Math. 2, 482, with the aid of the global homology algorithm by Needleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (e.g., at blast.ncbi.nlm.nih.gov/Blast.cgi?PAGE_TYPE=BlastSearch&BLAST_SPEC=blast2seq&LINK _LOC=align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment.

Percentage identity is obtained by determining the number of identical positions at which the sequences to be compared correspond, dividing this number by the number of positions compared (e.g., the number of positions in the reference sequence) and multiplying this result by 100.

In some embodiments, the degree of similarity or identity is given for a region which is at least about 50%, at least about 60%, at least about 70%, at least about 80%, at least about 90% or about 100% of the entire length of the reference sequence. For example, if the reference nucleic acid sequence consists of 200 nucleotides, the degree of identity is given for at least about 100, at least about 120, at least about 140, at least about 160, at least about 180, or about 200 nucleotides, in some embodiments continuous nucleotides. In some embodiments, the degree of similarity or identity is given for the entire length of the reference sequence.

In some embodiments, "isolated" means removed (e.g., purified) from the natural state or from an artificial composition, such as a composition from a production process. For example, a nucleic acid, peptide or polypeptide naturally present in a living animal is not "isolated", but the same nucleic acid, peptide or polypeptide partially or completely separated from the coexisting materials of its natural state is "isolated". An isolated nucleic acid, peptide or polypeptide can exist in substantially purified form, or can exist in a non-native environment such as, for example, a host cell.

The term "expression" as used herein is defined as the transcription and/or translation of a particular nucleotide sequence.

In the context of the present disclosure, the term "transcription" relates to a process, wherein the genetic code in a DNA sequence is transcribed into RNA (especially mRNA). Subsequently, the RNA may be translated into peptide or polypeptide.

With respect to RNA, the term "expression" or "translation" relates to the process in the ribosomes of a cell by which a strand of mRNA directs the assembly of a sequence of amino acids to make a peptide or polypeptide.

The term "nucleic acid" comprises deoxyribonucleic acid (DNA), ribonucleic acid (RNA), combinations thereof, and modified forms thereof. The term comprises genomic DNA, cDNA, mRNA, recombinantly produced and chemically synthesized molecules. A nucleic acid may be present as a single-stranded or double-stranded and linear or covalently circularly closed molecule. A nucleic acid can be isolated. The term "isolated nucleic acid" means, according to the present disclosure, that the nucleic acid (i) was amplified in vitro, for example via polymerase chain reaction (PCR) for DNA or in vitro transcription (using, e.g., an RNA polymerase) for RNA, (ii) was produced recombinantly by cloning, (iii) was purified, for example, by cleavage and separation by gel electrophoresis, or (iv) was synthesized, for example, by chemical synthesis.

The term "nucleoside" (abbreviated herein as "N") relates to compounds which can be thought of as nucleotides without a phosphate group. While a nucleoside is a nucleobase linked to a sugar (e.g., ribose or deoxyribose), a nucleotide is composed of a nucleoside and one or more phosphate groups. Examples of nucleosides include cytidine, uridine, pseudouridine, adenosine, and guanosine.

The five standard nucleosides which usually make up naturally occurring nucleic acids are uridine, adenosine, thymidine, cytidine and guanosine. The five nucleosides are commonly abbreviated to their one letter codes U, A, T, C and G, respectively. However, thymidine is more commonly written as "dT" ("d" represents "deoxy") as it contains a 2'-deoxyribofuranose moiety rather than the ribofuranose ring found in uridine. This is because thymidine is found in deoxyribonucleic acid (DNA) and not ribonucleic acid (RNA). Conversely, uridine is found in RNA and not DNA. The remaining three nucleosides may be found in both RNA and DNA. In RNA, they would be represented as A, C and G, whereas in DNA they would be represented as dA, dC and dG.

A modified purine (A or G) or pyrimidine (C, T, or U) base moiety is preferably modified by one or more alkyl groups, more preferably one or more C1-4 alkyl groups, even more preferably one or more methyl groups. Particular examples of modified purine or pyrimidine base moieties include N7-alkyl-guanine, N6-alkyl-adenine, 5-alkyl-cytosine, 5-alkyl-uracil, and N(1)-alkyl-uracil, such as N7-C1-4 alkyl-guanine, N6-C1-4 alkyl-adenine, 5-C1-4 alkyl-cytosine, 5-C1-4 alkyl-uracil, and N(1)-C1-4 alkyl-uracil, preferably N7-methyl-guanine, N6-methyl-adenine, 5-methyl-cytosine, 5-methyl-uracil, N1-methyl-pseudouridine, and N(1)-methyl-uracil.

Herein, the term "DNA" relates to a nucleic acid molecule which includes deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

DNA may be recombinant DNA and may be obtained by cloning of a nucleic acid, in particular cDNA. The cDNA may be obtained by reverse transcription of RNA.

The term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered/modified nucleotides can be referred to as analogs of naturally occurring nucleotides, and the corresponding RNAs containing such altered/modified nucleotides (i.e., altered/modified RNAs) can be referred to as analogs of naturally occurring RNAs. A molecule contains "a majority of ribonucleotide residues" if the content of ribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (i.e., naturally occurring) nucleotide residues or analogs thereof).

"RNA" includes mRNA, tRNA, ribosomal RNA (rRNA), small nuclear RNA (snRNA), self-amplifying RNA (saRNA), single-stranded RNA (ssRNA), dsRNA, inhibitory RNA (such as antisense ssRNA, small interfering RNA (siRNA), or microRNA (miRNA)), activating RNA (such as small activating RNA) and immunostimulatory RNA (isRNA). In some embodiments, "RNA" refers to mRNA.

It must be noted that as used herein and in the appended claims, the singular forms "a", "an", and "the" include plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of" also include the term "consisting of'.

### Embodiments of the invention

Various preferred features and embodiments of the present invention will now be described with reference to the following numbered paragraphs:
1. An antibody-drug conjugate (ADC),
   wherein the antibody is conjugated to the drug by cysteine-based site-specific conjugation,
   wherein the antibody comprises a first polypeptide comprising an immunoglobulin fragment crystallisable (Fc) region, at least one variable domain N-terminal of the Fc region and an engineered IgG hinge region between the Fc region and the at least one variable domain, and
   wherein the engineered IgG hinge region is heterologous to the Fc region and/or is mutated to provide a predetermined number of cysteine residues.
2. The ADC according to paragraph 1, wherein the at least one variable domain is selected from the group consisting of a single-chain variable fragment (scFv); a Fab; a Fab'; a F(ab)'2; an Fv; a single domain antibody (sdAb); a VHH; a single chain variable domain; a designed ankyrin repeat protein (DARPin); and an aptamer.
3. The ADC according to paragraph 1 or paragraph 2, wherein the antibody is selected from the group consisting of a full-length immunoglobulin, a scFv-Fc, a Fab-Fc, an Fv-Fc, a sdAb-Fc, or a VHH-Fc.
4. The ADC according to any one of the preceding paragraphs, wherein the antibody is a VHH-Fc.
5. The ADC according to any one of the preceding paragraphs, wherein the antibody further comprises a second polypeptide comprising an Fc region and an engineered IgG hinge region N-terminal of the Fc region, wherein the engineered IgG hinge region is heterologous to the Fc region of the second polypeptide and/or is mutated to provide a predetermined number of cysteine residues, and wherein the engineered IgG hinge region of the first polypeptide and the engineered IgG hinge region of the second polypeptide are complimentary engineered IgG hinge regions.
6. The ADC according to paragraph 5, wherein the second polypeptide further comprises at last one variable domain N-terminal of the engineered IgG hinge region, preferably wherein the at least one variable domain is selected from the group consisting of a single-chain variable fragment (scFv); an Fab; an Fab'; an F(ab)'2; an Fv; a single domain antibody (sdAb); a VHH; a single chain variable domain; a designed ankyrin repeat protein (DARPin); and an aptamer; more preferably wherein the at least one variable domain is a VHH.
7. An antibody-drug conjugate (ADC),
   wherein the antibody is conjugated to the drug by cysteine-based site-specific conjugation,
   wherein the antibody comprises a first polypeptide and a second polypeptide, wherein each of the first polypeptide and the second polypeptide comprises an immunoglobulin fragment crystallisable (Fc) region, at least one variable domain which is a VHH N-terminal of the Fc region and an engineered IgG hinge region between the Fc region and the at least one VHH,
   wherein the engineered IgG hinge region of the first polypeptide and the engineered IgG hinge region of the second polypeptide are complimentary engineered IgG hinge regions, and
   wherein the engineered IgG hinge region is heterologous to the Fc region and/or is mutated to provide a predetermined number of cysteine residues.
8. The ADC according to any one of the preceding paragraphs, wherein the engineered IgG hinge region of the first polypeptide or the engineered IgG hinge region of each of the first polypeptide and the second polypeptide comprises at least one cysteine residue, preferably wherein the engineered IgG hinge region comprises two, three, four, five, six, seven, eight or nine cysteine residues.
9. The ADC according to any one of the preceding paragraphs, wherein the engineered IgG hinge region of the first polypeptide comprises at least one additional cysteine residue compared to the native hinge region of the Fc region of the first polypeptide, preferably two, three, four, five, six, seven, eight or nine additional cysteine residues compared to the native hinge region of the Fc region of the first polypeptide.
10. The ADC according to any one of the preceding paragraphs, wherein the engineered IgG hinge region of the first polypeptide comprises at least one fewer cysteine residue compared to the native hinge region of the Fc region of the first polypeptide, preferably two, three, four, five, six, seven, eight or nine fewer cysteine residues compared to the native hinge region of the Fc region of the first polypeptide.
11. The ADC according to any one of paragraphs 5-10, wherein the engineered IgG hinge region of the second polypeptide comprises at least one additional cysteine residue compared to the native hinge region of the Fc region of the second polypeptide, preferably two, three, four, five, six, seven, eight or nine additional cysteine residues compared to the native hinge region of the Fc region of the second polypeptide.
12. The ADC according to any one of paragraphs 5-10, wherein the engineered IgG hinge region of the second polypeptide comprises at least one fewer cysteine residue compared to the native hinge region of the Fc region of the second polypeptide, preferably two, three, four, five, six, seven, eight or nine fewer cysteine residues compared to the native hinge region of the Fc region of the second polypeptide.
13. The ADC according to any one of the preceding paragraphs, wherein the engineered IgG hinge region comprises a sequence having at least 70% identity to a sequence as set forth in any one of SEQ ID NOs: 1, 2, 3, 4 or 5.
14. The ADC according to any one of the preceding paragraphs, wherein the engineered IgG hinge region is an IgG1 hinge region.
15. The ADC according to any one of the preceding paragraphs, wherein the engineered IgG hinge region has an amino acid sequence as set forth in SEQ ID NO: 1, or is a fragment thereof and/or a variant thereof comprising an amino acid sequence having at least 70% identity to SEQ ID NO: 1.
16. The ADC according any one of paragraphs 1-13, wherein the engineered IgG hinge region is an IgG2 hinge region.
17. The ADC according to any one of paragraphs 1-13 or 16, wherein the engineered IgG hinge region has an amino acid sequence as set forth in SEQ ID NO: 2, or is a fragment thereof and/or a variant thereof comprising or an amino acid sequence having at least 70% identity to SEQ ID NO: 2.
18. The ADC according to any one of paragraphs 1-13, wherein the engineered IgG hinge region is an IgG3 hinge region.
19. The ADC according to any one of paragraphs 1-13 or 18, wherein the engineered IgG hinge region has an amino acid sequence as set forth in SEQ ID NO: 3 or SEQ ID NO: 4, or is a fragment thereof and/or a variant thereof comprising an amino acid sequence having at least 70% identity to SEQ ID NO: 3 or to SEQ ID NO: 4.
20. The ADC according to any one paragraphs 1-13, 18 or 19, wherein the engineered IgG hinge region has an amino acid sequence as set forth in SEQ ID NO: 6 or SEQ ID NO: 7, or is a fragment thereof and/or a variant thereof comprising an amino acid sequence having at least 70% identity to SEQ ID NO: 6 or to SEQ ID NO: 7.
21. The ADC according to any one of paragraphs 1-13, wherein the engineered IgG hinge region is an IgG4 hinge region.
22. The ADC according to any one of paragraphs 1-13 or 21, wherein the engineered IgG hinge region has an amino acid sequence as set forth in SEQ ID NO: 5, or is a fragment thereof and/or a variant thereof comprising an amino acid sequence having at least 70% identity to SEQ ID NO: 5.
23. The ADC according to any one of the preceding paragraphs, wherein the engineered IgG hinge region has an amino acid sequence as set forth in any one of SEQ ID NOs: 8-118, preferably an amino acid sequence as set forth in SEQ ID NO: 8, 9, 12, 13, 18, 51, 67, 74, 84, 100, 113 or 118.
24. The ADC according to any one of the preceding paragraphs, wherein the engineered IgG hinge region of the first polypeptide and/or second polypeptide comprises at least one amino acid modification to avoid O-glycosylation compared to the native IgG hinge region of the Fc region of the first polypeptide and/or second polypeptide.
25. The ADC according to paragraph 24, wherein the at least one amino acid modification to avoid O-glycosylation is an amino acid substitution.
26. The ADC according to any one of the preceding paragraphs, wherein the engineered IgG hinge region has an amino acid sequence as set forth in any one of SEQ ID NOs: 34-44, 51-61, 64-67, 70-75, 78-87, 90-111 or 113.
27. The antibody according to any one of paragraphs 24-26, wherein the engineered IgG hinge region is an IgG3 hinge region and the at least one amino acid modification is selected from the group consisting of S^{241MM}X, T^{241JJ}X and the combination thereof, wherein the numbering is according to the according to the Kabat numbering scheme.
28. The ADC according to any one of the preceding paragraphs, wherein the engineered IgG hinge region is or is derived from a human IgG hinge region.
29. The ADC according to any one of the preceding paragraphs, wherein the antibody is selected from the group consisting of a human antibody, a humanized antibody, a chimeric antibody, a multispecific antibody, a monoclonal antibody, and a polyclonal antibody.
30. The ADC according to any one of the preceding paragraphs, wherein the at least one variable domain of the first polypeptide, the second polypeptide or both the first polypeptide and the second polypeptide is specific for a cancer antigen.
31. The ADC according to any one of the preceding paragraphs, wherein the antibody is conjugated to the drug by cysteine-based site-specific conjugation through all of the cysteine residues within the engineered IgG hinge region.
32. The ADC according to any one of the preceding paragraphs, wherein the ADC has a drug-to-antibody-ratio (DAR) of from about 2 to about 12, preferably a DAR of from about 4 to about 8.
33. The ADC according to any one of the preceding paragraphs, wherein the ADC has a drug-to-antibody-ratio (DAR) of about 2, 4, 6, 8, 10, or 12, preferably a DAR of about 8.
34. The ADC according to any one of the preceding paragraphs, wherein the drug is selected from the group consisting of a cytotoxic drug, an antimicrobial agent, or an immunomodulatory agent.
35. A method of producing an antibody-drug conjugate (ADC) having a predetermined drug-to-antibody ratio (DAR), comprising the steps of:
   (i) providing an antibody as defined in any one of paragraphs 1-34;
   (ii) reducing the antibody with a reducing agent; and
   (iii) conjugating the reduced antibody with the drug.
36. A method of producing an antibody-drug conjugate (ADC) having a predetermined drug-to-antibody ratio (DAR), comprising the steps of:
   (i)
      (a) providing at least one polynucleotide sequence encoding an antibody comprising a first polypeptide comprising an immunoglobulin fragment crystallisable (Fc) region and at least one variable domain N-terminal of the Fc region;
      (b) modifying the at least one polynucleotide sequence to encode an engineered IgG hinge region between the Fc region and the at least one variable domain of the first polypeptide, wherein the engineered IgG hinge region is heterologous to the Fc region and/or is mutated to provide a predetermined number of cysteine residues;
      (c) introducing the at least one polynucleotide sequence into a cell;
      (d) culturing the cell under conditions suitable for the expression of the at least one polynucleotide sequence;
      (e) isolating the antibody expressed by the cell;
   (ii) reducing the antibody with a reducing agent; and
   (iii) conjugating the reduced antibody with the drug.
37. A method of producing an antibody-drug conjugate (ADC) having a predetermined drug-to-antibody ratio (DAR), comprising the steps of:
   (i)
      (a) providing at least one polynucleotide sequence encoding an antibody wherein the antibody comprises a first polypeptide and a second polypeptide, wherein each of the first polypeptide and the second polypeptide comprises an immunoglobulin fragment crystallisable (Fc) region and at least one variable domain which is a VHH N-terminal of the Fc region;
      (b) modifying the at least one polynucleotide sequence to encode an engineered IgG hinge region between the Fc region and the at least one variable domain of each of the first polypeptide and the second polypeptide, wherein the engineered IgG hinge region of the first polypeptide and the engineered IgG hinge region of the second polypeptide are complimentary engineered IgG hinge regions, and wherein the engineered IgG hinge region is heterologous to the Fc region and/or is mutated to provide a predetermined number of cysteine residues;
      (c) introducing the at least one polynucleotide sequence into a cell;
      (d) culturing the cell under conditions suitable for the expression of the at least one polynucleotide sequence;
      (e) isolating the antibody expressed by the cell;
   (ii) reducing the antibody with a reducing agent; and
   (iii) conjugating the reduced antibody with the drug.
38. The method according to any one of paragraphs 35-37, wherein the step of reducing the antibody in step (ii) is a partial reduction of the antibody to reduce the inter-chain disulphide bonds of the antibody.
39. The method according to any one of paragraphs 35-38, wherein the reducing agent is dithiothreitol (DTT) or tris (2-carboxyethyl) phosphine (TCEP).
40 The method according to any one of paragraphs 35-39, wherein the drug is in the form of a linker-drug conjugate comprising a thiol-reactive group.
41. The method according to paragraph 40, wherein the thiol-reactive group is selected from the group consisting of a maleimide, bromoacetamide, disulphide, α-haloacetamide, α-halocarbonyl, vinylsulfone, heteroaryl sulfone, thiosulfonate, electron deficient aryl halide, ethynylphosphonamidate, vinylphosphonite, palladiumoxidative-addition complex.
42. The method according to any one of paragraphs 35-41, wherein step (iii) is performed using a thiol-reactive coupling strategy.
43. The method according to paragraph 42, wherein the thiol-reactive coupling strategy is maleimide-based conjugation.
44. The method according to any one of paragraphs 35-43, wherein the method provides homogenous ADCs.
45. The method according to any one of paragraphs 36 or 38-44, wherein the antibody further comprises a second polypeptide comprising an Fc region, and wherein step (i) (b) further comprises modifying the at least one polynucleotide sequence to encode an engineered IgG hinge region N-terminal of the Fc region of the second polypeptide, wherein the engineered IgG hinge region is heterologous to the Fc region of the second polypeptide and/or is mutated to provide a predetermined number of cysteine residues, and wherein the engineered IgG hinge region of the first polypeptide and the engineered IgG hinge region of the second polypeptide are complimentary engineered IgG hinge regions.
46. The method according to paragraph 45, wherein the second polypeptide further comprises at last one variable domain N-terminal of the engineered IgG hinge region, preferably wherein the at least one variable domain is selected from the group consisting of a single-chain variable fragment (scFv); an Fab; an Fab'; an F(ab)'2; an Fv; a single domain antibody (sdAb); a VHH; a single chain variable domain; a designed ankyrin repeat protein (DARPin); and an aptamer; more preferably wherein the at least one variable domain is a VHH.
47. The method according to any one paragraphs 36-46, wherein the engineered IgG hinge region of the first polypeptide or the engineered IgG hinge region of the first polypeptide and the second polypeptide comprises at least one cysteine residue, preferably wherein the engineered IgG hinge region of the first polypeptide or the engineered IgG hinge region of the first polypeptide and the second polypeptide comprises two, three, four, five, six, seven, eight or nine cysteine residues.
48. The method according to any one of paragraphs 36-47, wherein the engineered IgG hinge region of the first polypeptide comprises at least one additional cysteine residue compared to the native hinge region of the Fc region of the first polypeptide, preferably two, three, four, five, six, seven, eight or nine additional cysteine residues compared to the native hinge region of the Fc region of the first polypeptide.
49. The method according to any one of paragraphs 36-47, wherein the engineered IgG hinge region of the first polypeptide comprises at least one fewer cysteine residue compared to the native hinge region of the Fc region of the first polypeptide, preferably two, three, four, five, six, seven, eight or nine fewer cysteine residues compared to the native hinge region of the Fc region of the first polypeptide.
50. The method according to any one of paragraphs 38 or 45-49, wherein the engineered IgG hinge region of the second polypeptide comprises at least one additional cysteine residue compared to the native hinge region of the Fc region of the second polypeptide, preferably two, three, four, five, six, seven, eight or nine additional cysteine residues compared to the native hinge region of the Fc region of the second polypeptide.
51. The method according to any one of paragraphs 38 or 45-49, wherein the engineered IgG hinge region of the second polypeptide comprises at least one fewer cysteine residue compared to the native hinge region of the Fc region of the second polypeptide, preferably two, three, four, five, six, seven, eight or nine fewer cysteine residues compared to the native hinge region of the Fc region of the second polypeptide.
52. The method according to any one of paragraphs 36-51, wherein the engineered IgG hinge region comprises a sequence having at least 70% identity to a sequence as set forth in any one of SEQ ID NOs: 1, 2, 3, 4 or 5.
53. The method according to any one of paragraphs 36-52, wherein the engineered IgG hinge region is an IgG1 hinge region.
54. The method according to any one of paragraphs 36-53, wherein the engineered IgG hinge region has an amino acid sequence as set forth in SEQ ID NO: 1, or is a fragment thereof and/or a variant thereof comprising an amino acid sequence having at least 70% identity to SEQ ID NO: 1.
55. The method according any one of paragraphs 36-52, wherein the engineered IgG hinge region is an IgG2 hinge region.
56. The method according to any one of paragraphs 36-52 or 55, wherein the engineered IgG hinge region has an amino acid sequence as set forth in SEQ ID NO: 2, or is a fragment thereof and/or a variant thereof comprising or an amino acid sequence having at least 70% identity to SEQ ID NO: 2.
57. The method according to any one of paragraphs 36-52, wherein the engineered IgG hinge region is an IgG3 hinge region.
58. The method according to any one paragraphs 36-52 or 57, wherein the engineered IgG hinge region has an amino acid sequence as set forth in SEQ ID NO: 3, SEQ ID NO: 4, SEQ ID NO: 39 or SEQ ID NO: 40, or is a fragment thereof and/or a variant thereof comprising an amino acid sequence having at least 70% identity to SEQ ID NO: 3, to SEQ ID NO: 4, to SEQ ID NO: 39 or to SEQ ID NO: 40.
59. The method according to any one of paragraphs 36-52, wherein the engineered IgG hinge region is an IgG4 hinge region.
60. The method according to any one of paragraphs 36-52 or 59, wherein the engineered IgG hinge region has an amino acid sequence as set forth in SEQ ID NO: 5, or is a fragment thereof and/or a variant thereof comprising an amino acid sequence having at least 70% identity to SEQ ID NO: 5.
61. The method according to any one of paragraphs 36-60, wherein the engineered IgG hinge region has an amino acid sequence as set forth in any one of SEQ ID NOs: 8-118, preferably an amino acid sequence as set forth in SEQ ID NO: 8, 9, 12, 13, 18, 51, 67, 74, 84, 100, 113 or 118.
62. The method according to any one of paragraphs 36-61, wherein the engineered IgG hinge region of the first polypeptide and/or second polypeptide comprises at least one amino acid modification to avoid O-glycosylation compared to the native hinge region of the Fc region of the first polypeptide and/or second polypeptide.
63. The method according to paragraph 62, wherein the at least one amino acid modification to avoid O-glycosylation is an amino acid substitution.
64. The method according to any one of paragraphs 36-63, wherein the engineered IgG hinge region has an amino acid sequence as set forth in any one of SEQ ID NOs: 34-44, 51-61, 64-67, 70-75, 78-87, 90-111 or 113.
65. The method according to any one of paragraphs 62-64, wherein the engineered IgG hinge region is an IgG3 hinge region and the at least one amino acid modification is selected from the group consisting of S^{241MM}X, T^{241JJ}X and the combination thereof, wherein the numbering is according to the according to the Kabat numbering scheme.
66. The method according to any one of paragraphs 36-65, wherein all of the cysteine residues within the engineered IgG hinge region form stable inter-chain disulphide bonds prior to step (ii).
67. The method according to any one of paragraphs 36-66, wherein the engineered IgG hinge region is or is derived from a human IgG hinge region.
68. The method according to any one of paragraphs 36 or 38-67, wherein the antibody is selected from the group consisting of a human antibody, a humanized antibody, a chimeric antibody, a multispecific antibody, a monoclonal antibody, and a polyclonal antibody.
69. The method according to any one of paragraphs 36 or 38-68, wherein the antibody is selected from the group consisting of a full-length immunoglobulin, a scFv-Fc, a Fab-Fc, an Fv-Fc, a sdAb-Fc, or a VHH-Fc.
70. The method according to paragraph 69, wherein the antibody is a VHH-Fc.
71. The method according to any one of paragraphs 36 or 38-70, wherein the at least one variable domain of the first polypeptide and/or second polypeptide is selected from the group consisting of a single-chain variable fragment (scFv); an Fab; an Fab'; an F(ab)'2; an Fv; a single domain antibody (sdAb); a VHH; a single chain variable domain; a designed ankyrin repeat protein (DARPin); and an aptamer.
72. The method according to any one of paragraphs 36-71, wherein the at least one variable domain of the first polypeptide, the second polypeptide or both the first polypeptide and the second polypeptide is specific for a cancer antigen.
73. The method according to any one of paragraphs 36-72, wherein in step (iii) the antibody is conjugated to the drug by cysteine-based site-specific conjugation through all of the cysteine residues within the engineered IgG hinge region.
74. The method according to any one of paragraphs 36-73, wherein the ADC has a drug-to-antibody-ratio (DAR) of from about 2 to about 12, preferably a DAR of from about 4 to about 8.
75. The method according to any one of paragraphs 36-74, wherein the ADC has a drug-to-antibody-ratio (DAR) of about 2, 4, 6, 8, 10, or 12, preferably a DAR of about 8.
76. The method according to any one of paragraphs 36-75, wherein the drug is selected from the group consisting of a cytotoxic drug, an antimicrobial agent, or an immunomodulatory agent.
77. An ADC obtained or obtainable by the method according to any one of paragraphs 35-76.
78. A pharmaceutical composition comprising the ADC according to any one of paragraphs 1-34 or 77 and a pharmaceutically acceptable carrier, excipient and/or diluent.
79. The ADC according to any one of paragraphs 1-34 or 77, or the pharmaceutical composition according to paragraph 78, for use in therapy.
80. The ADC according to any one of paragraphs 1-34 or 77, or the pharmaceutical composition according to paragraph 78, for use in the treatment of a disease or condition selected from the group consisting of cancer, autoimmune disease, infection, infectious diseases, cardiovascular diseases and liver metabolic disorders.

The publications discussed herein are provided solely for their disclosure prior to the filing date of the present application. Nothing herein is to be construed as an admission that such publications constitute prior art to the claims appended hereto.

The invention will now be further described by way of Examples, which are meant to serve to assist one of ordinary skill in the art in carrying out the invention and are not intended in any way to limit the scope of the invention.

### EXAMPLES

### Materials and Methods

### Expression of recombinant VHH fusion proteins

Human codon optimized VHH-Fc sequences comprising engineered hinge sequences were generated by gene synthesis and cloned into pTWIST expression vector (Twist Bioscience). For recombinant expression of the VHH-Fc fusions, Expi293 cells were transfected with ExpiFectamine (ThermoFisher Scientific) using the manufacturer's protocol. Culture supernatants were harvested from Expi293 producer cell lines after 4-5 days, centrifuged and recombinant proteins were used for further characterization and purification.

### Conjugation of toxin payload to VHH-Fc fusion proteins

To a solution of the VHH-Fc fusion protein (1-20 mg/ml, 1.0 equiv) in 20 mM phosphate buffer is added a 100 mM EDTA buffer solution to a final concentration of 1 mM. The pH of the solution is adjusted to 7.0-8.0 with 0.5 M disodium hydrogen phosphate. Then a freshly prepared 25 mM solution of TCEP hydrochloride (3.0-6.0 equiv, adjusted for desired DAR value) in 20 mM phosphate buffer, pH 7.0-8.0 is added to the antibody solution and gently mixed. After 60-90 min, a 30 mM solution of the linker-payload (5.0-10.0 equiv, adjusted for desired DAR value) in DMSO is added to the antibody solution. The resulting solution is gently mixed and incubated at 22-37 °C. After 2 h, the reaction is purified and the buffer exchanged using ultrafiltration or gel filtration with 20 mM histidine buffer, pH 6.0 or another suitable buffer to afford the antibody drug conjugate. The drug-to-antibody-ratio (DAR) was analyzed using hydrophobic interaction chromatography.

### Western Blot for aggregate detection

Western Blot analysis was performed using the cell culture supernatant of the previously cultivated Expi293 cells as sample. Western Blots were performed using the Jess device from BioTechne.

The supernatant was 1:10 diluted and applied to the machine according to the manufacturer's protocol. The separation was performed under non-reducing conditions and the subsequent detection was performed using an anti-human HRP antibody.

### Affinity to the target antigen

For affinity measurements an Octet HTX device from Sartorius was used.

Commercially available AHC biosensors were soaked for at least 10 min in kinetic buffer (KB) purchased from the instrument manufacturer. Following a 60 sec baseline in KB, the VHH-Fc proteins were loaded onto the sensors for 300 sec or until a threshold of 0.9 nm in response was reached. Following a 120 sec baseline in KB, association to antigen 1 was measured over 600 sec using seven different concentrations of the antigen 1 starting at 50 nM in a 1:1 serial dilution series down to 0.78125 nM. As a reference, KB without the antigen 1 was measured. The dissociation was acquired over 600 sec in KB. The signal of the reference well was subtracted from the signal of all other biosensors and the signals of these processed data were aligned to the average of the baseline before the association step. For inter-step correction, the data were aligned to the dissociation step and Savitzky-Golay filtering was applied to all curves. Association and dissociations were globally fitted using a 1:1 Langmuir binding model.

### Affinity to CD64

For affinity measurements an Octet HTX device from Sartorius was used.

Commercially available Ni-NTA biosensors were soaked for at least 10 min in kinetic buffer (KB) purchased from the instrument manufacturer. Following a 60 sec baseline in KB, the His-tagged CD64 was loaded for 300 sec or until a threshold of 1 nm in response was reached. Following a 120 sec baseline in KB, association to the VHH-Fcs was measured over 600 sec using seven different concentrations of the VHH-Fcs starting at 50 nM in a 1:1 serial dilution series down to 0.78125 nM. As a reference, KB without the VHH-Fcs was measured. The dissociation was acquired over 600 sec in KB. The signal of the reference well was subtracted from the signal of all other biosensors and the signals of these processed data were aligned to the average of the baseline before the association step. For inter-step correction, the data were aligned to the dissociation step and Savitzky-Golay filtering was applied to all curves. Association and dissociations were globally fitted using a 1:1 Langmuir binding model.

### Dynamic Light Scattering (DLS) and Nano Differential Scanning Fluorimetry (NanoDSF)

Thermal stabilities (via NanoDSF) and size distribution (via DLS) were investigated using the Prometheus PANTA from NanoTemper. 10 µL of VHH-Fc protein was loaded into capillaries. After loading, the capillaries were mounted into the instrument and the DLS analysis was performed. To assess thermal stability, a heat ramp of 1°C/min from 25°C to 95°C was subsequently applied to all samples. During this process the intrinsic fluorescence of the proteins was measured at 350 nm and 330 nm. The ratio was plotted against the temperature and the first derivative was calculated. Minima and maxima corresponded to the TM values.

### Size Exclusion Chromatography (SEC)

Aggregation analysis was performed via SEC utilizing an Agilent Infinity II HPLC and a Biozen 1.8 µm dSEC-2, 200 Å LC column (300 × 4.6 mm). Flowrates were adjusted to 0.25 mL/min, resulting in approximately 255 bar pressure. As mobile phase 0.2 M potassium phosphate, 250 mM KCI, pH 6.2, 5% acetonitrile was used. Each run took 20 min excluding a 2-3 min wash step between each analysis. 10 µL of the VHH-Fc fusion proteins were applied and detected by absorption at 280 nm.

### EC50 determination

Antigen 1-positive or -negative cells were seeded in a 96-well microtiter plate (round button) to a final density of 2×10⁵ and incubated in the presence of different concentrations the VHH-Fc fusion for 30 min at 4°C. A concentration range of 90 nM, down to 123 pM was covered. Afterwards, cells were washed twice with FACS-buffer (1x phosphate-buffer saline + 10 mL 0.5 M ethylenediaminetetraacetic acid + 10 mL Fetal Bovine Serum (FBS) ) and once with 1x phosphate-buffer saline and subsequently incubated with an anti-human APC detection antibody for 30 min at 4°C. After another washing step with FACS-buffer, cells were fixated with BD Fixative and analysed with the FACS celesta (BD Biosciences). An excitation laser at 633 nm was used to detect the APC signals. Mean fluorescence intensities (MFI) values were plotted against the concentration of the VHH-Fc and the resulting data points were fitted using a non-linear fit, resulting in the EC50 values of the respective VHH-Fc.

### FACS internalization

Antigen 1-positive cells were seeded in FACS tubes to a final density of 2×10⁵ and incubated in the presence of 100 nM or 500 nM VHH-Fc fusion for 30 min at 4°C.

Afterwards, cells were washed twice with FACS-buffer and once with 1x phosphate-buffer saline and were subsequently re-suspended in 1 mL growth medium. Cells were incubated for either 30 min, 1h, 2h or 4h at either 37°C or on ice. Cells were washed twice with FACS buffer and once with PBS before an anti-human APC detection antibody was applied for 30 min. After another washing step with FACS-buffer and PBS, cells were fixated with BD Fixative and analysed with the FACS celesta (BD Biosciences). Excitation laser at 633 nm was used to detect the APC signals. The MFI values of the 4°C samples were compared with their respective 37°C counterpart to visualize the internalization-mediated difference in fluorescence intensity.

### Mass spectrometry of antibodies

For intact mass determination, 10 µg of each VHH-Fc fusion was de-glycosylated in the original buffer by overnight incubation with PNGase F at 37°C. A volume corresponding to 1 µg de-glycosylated protein was separated on a Waters Acquity I-Class UPLC by reversed-phase chromatography on a Waters BEH C4 column maintained at 80°C. Proteins were eluted with a linear gradient of 15-30% acetonitrile over 12 minutes, followed by an increase to 95% acetonitrile in 2 min. 0.1% (v/v) formic acid was used as modifier in all solvents. Mass spectra in the m/z-range of 500-4000 were acquired on a Waters Xevo G2-XS QTOF. Data analysis was performed in ProteinMetrics Inc. Byos software. Raw mass spectra were integrated over the entire elution range and deconvoluted with settings appropriate for the m/z range and instrument resolution. For the annotation of peaks in the deconvoluted mass spectra, homodimers with all possible disulphide-bridges formed and asparagine to aspartic acid converted by de-glycosylation was assumed.

### Example 1 - Western blot analysis of cell culture supernatant of different VHH-Fc fusions

The present inventors elucidated for the first time the utilisation of ADCs comprising an engineered IgG hinge region having a predetermined number of cysteine residues to control the DAR. In particular, to create an ADC comprising an engineered IgG hinge region, the present inventors engineered a VHH-Fc fusion comprising an IgG1 Fc region and a heterologous hinge region. The inventors utilised a human IgG2-derived hinge region or a human IgG3-derived hinge region as the heterologous hinge region. Human IgG hinge region sequences were used to minimize immunogenicity. Furthermore, to provide a homogenous ADC product, the inventors modified the IgG3-derived hinge region to replace the serine and threonine residues which are subject to O-glycosylation with alanine residues. The inventors also compared the VVH-Fcs comprising an engineered IgG hinge with conventional VHH-Fcs comprising an IgG1 Fc region, a truncated IgG1 hinge and a VHH. Hence, a conventional VHH-Fc antibody comprising a truncated IgG1 hinge as well as antibodies comprising engineered IgG hinge regions were produced.

The present inventors generated antibodies comprising 5 different VHH domains, each specific for the same target antigen, to investigate the effects of the engineered IgG hinge regions regardless of the VHH used or the specificity of the VHHs.

All 5 VHHs binding to the target antigen were produced transiently in ExpiHEK cells as VHH-Fc fusions comprising either a truncated IgG1 hinge, or an IgG2- or an IgG3-derived hinge region, resulting in 15 constructs in total. Thus, the inventors first generated 5 conventional VHH-Fcs comprising an IgG1 Fc region and a truncated IgG1 hinge region for comparison. The present inventors then generated 10 VHH-Fcs comprising an IgG1 Fc region and an engineered IgG hinge region: 5 VHH-Fcs comprising an IgG2-derived hinge region and 5 VHH-Fcs comprising an IgG3-derived hinge region.

The IgG hinge regions are as follows:
Truncated IgG1 hinge: DKTHTCPPCP (SEQ ID NO: 12)
IgG2-derived hinge: ERKCCVECPPCP (SEQ ID NO: 8)
IgG3-derived hinge: PRCPEPKACDAPPPCPRCP (SEQ ID NO: 112)

The conventional VHH-Fcs (comprising the truncated IgG1 hinge SEQ ID NO: 12) would provide an ADC having a DAR of 4 following cysteine-based site-specific conjugation. The VHH-Fcs comprising an engineered IgG2- or IgG3-derived hinge region would provide an ADC having a DAR of 8 following cysteine-based site-specific conjugation. Thus, the VHH-Fcs comprising an engineered IgG2- or IgG3-derived hinge region provide antibodies which have been optimised for a higher DAR.

The present inventors then performed analysis of aggregation by Western blot using nonreduced samples. The cell culture supernatants, each containing one of the 15 VHH-Fc fusions, were analysed. Detection was carried out through use of an anti-human antibody.

By utilizing the IgG1 hinge and the IgG3-derived hinge, all VHH-Fcs were successfully produced and did not show any signs of aggregates, dimers or fragmentation products.

The IgG2-derived hinge led to aggregates or dimer formation in some constructs. One exception was the VHH-Fc2 variant, which mainly yielded the desired monomer band.

As such, shown herein are constructs with hinge regions derived from each of IgG2 and IgG3 that are capable of forming monomers. Thus, the VHH-Fc antibodies comprising an engineered IgG hinge region performed comparably to the conventional VHH-Fcs, regardless of the specificity of the VHHs. Moreover, the inventors have shown that modifications to avoid O-glycosylation are tolerated in the hinge regions. As shown below, all of the tested constructs are functional regardless of aggregate or dimer formation.

### Example 2 - Biophysical characterization of VHH-Fc fusions

To further validate the VHH-Fc fusions comprising an engineered IgG hinge region, the present inventors performed biophysical characterization of the 15 VHH-Fc fusions described above (the 5 conventional VHH-Fcs as well as the 10 VHH-Fcs comprising an engineered IgG hinge regions derived from an IgG2 hinge or an IgG3 hinge).

Thermal denaturation values (determined by NanoDSF), and SEC purity are depicted for the VHH-Fc fusions below, in Table 2. DLS results were also determined (data not shown).

Stability analysis of each of the VHH-Fc fusions was carried out using Nano Differential Scanning Fluorimetry (NanoDSF). In general, the stabilities appear mainly mediated by the VHH candidate and are less dependent on the hinge region architecture. High stabilities with TM values of up to 76 °C were observed.

SEC analysis further confirms that monodispersity is very high for the IgG1 hinge and the IgG3-derived hinge candidates, and shown in some IgG2-derived variants.

Thus, the biophysical characterizations confirm the results presented above in Example 1.

**Table 2 - Biophysical characterization of VHH-Fc fusions. SEC purity and thermal denaturation values determined by NanoDSF are depicted for the VHH-Fc fusions.**

| **Protein** | **Thermal Stability** / **NanoDSF** | | | | **SEC** |
|---|---|---|---|---|---|
| | **Thermal stability** | | **Turbidity** | | **Purity** |
| | **TM (°C)** | **ON (°C)** | **IP (°C)** | **ON (°C)** | **%** |
| **VHH-Fc1-IgG1-hinge** | 66.55 | 64.49 | 83.37 | 81.64 | 100 |
| **VHH-Fc1-IgG2-derived** | 66.16 | 63.92 | 83.35 | 58.06 | 76.4 |
| **VHH-Fc1-IgG3-derived** | 61.43 | 55.82 | 63.89 | 55.22 | 99.1 |
| **VHH-Fc2-IgG1-hinge** | 56.19 | 55.00 | 80.82 | 78.94 | 100 |
| **VHH-Fc2-IgG2-derived** | 54.04 | 49.19 | 83.30 | n.d. | 96.1 |
| **VHH-Fc2-IgG3-derived** | 57.28 | 60.00 | 79.74 | 68.68 | 100 |
| **VHH-Fc3-IgG1-hinge** | 57.67 | 53.32 | 72.69 | 64.39 | 100 |
| **VHH-Fc3-IgG2-derived** | 56.80 | 51.40 | 81.49 | 79.36 | 64.0 |
| **VHH-Fc3-IgG3-derived** | 57.86 | 52.62 | 80.64 | 77.45 | 100 |
| **VHH-Fc4-IgG1-hinge** | 75.44 | 72.80 | 82.45 | 80.51 | 100 |
| **VHH-Fc4-IgG2-derived** | 74.06 | 69.19 | n.d. | n.d. | 25.1 |
| **VHH-Fc4-IgG3-derived** | 76.48 | 73.86 | 81.32 | 79.05 | 97.6 |
| **VHH-Fc5-IgG1-hinge** | 56.89 | 56.11 | 56.84 | 56.31 | 100 |
| **VHH-Fc5-IgG2-derived** | 69.27 | 63.91 | 83.74 | 69.58 | 87.1 |
| **VHH-Fc5-IgG3-derived** | 70.79 | 65.19 | 73.98 | 62.99 | 100 |

### Example 3 - Affinity of VHH-Fc fusions

To further validate the VHH-Fcs, the present inventors performed affinity determination for each of the 15 VHH-Fcs described above (the 5 conventional VHH-Fcs as well as the 10 VHH-Fcs comprising an engineered IgG hinge regions derived from an IgG2 hinge or an lgG3 hinge).

An Octet HTX device was used to determine the affinity of the 15 different IgG-derived hinge VHH-Fc fusions to: (i) the target antigen of the VHH-Fcs and, (ii) huCD64. Associations and dissociations were globally fitted using a 1:1 Langmuir binding model, the results of which are shown in Table 3 below.

As indicated below, all three hinge regions mediate identical binding to the antigen, underlining that specificity and affinity is not altered upon switching to another hinge sequence. All target-antigen candidates bound the target antigen and CD64 with high affinity. This suggests that, as FcyR binding is not compromised by the engineered hinge sequences, ADCC and CDC activities were preserved for antibodies comprising the engineered IgG hinge regions.

There appears to be no negative influence upon optimization of the hinge for higher DARs. Moreover, there appears to be no negative influence upon modification of the hinge to avoid O-glycosylation. Further, comparable results are obtained with 5 different VHHs for each design, i.e. regardless of the specificity of the VHHs. CD64 binding is also shown to be independent from the utilized hinge region. This allows the hinge design to be chosen solely on DAR and not on other properties.

**Table 3 - Affinity of VHH-Fc fusions. Affinity to the primary antigen of the VHH-Fcs as well as to CD64 was determined using BLI.**

| **Protein** | **Affinity to target** | | | **Affinity to huCD64** | | |
|---|---|---|---|---|---|---|
| | **KD (M)** | **Kₒₙ (1/Ms)** | **K_{off} (1/s)** | **KD (M)** | **Kₒₙ (1/Ms)** | **K_{off} (1/s)** |
| **VHH-Fc1-IgG1-hinge** | 6.062E-10 | 1.095E05 | 6.640E-05 | 3.953E-09 | 5.378E04 | 2.126E-04 |
| **VHH-Fc1-IgG2-derived** | 3.468E-10 | 1.063E05 | 3.685E-05 | 4.192E-09 | 4.375E04 | 1.834E-04 |
| **VHH-Fc1-IgG3-derived** | 2.454E-10 | 1.083E05 | 2.657E-05 | 7.309E-09 | 5.569E04 | 4.070E-04 |
| **VHH-Fc2-IgG1-hinge** | 3.361E-08 | 1.188E04 | 3.993E-04 | 3.087E-09 | 6.198E04 | 1.913E-04 |
| **VHH-Fc2-IgG2-derived** | 4.600E-08 | 1.037E04 | 4.770E-04 | 3.044E-09 | 6.616E04 | 2.014E-04 |
| **VHH-Fc2-IgG3-derived** | 1.364E-06 | 2.148E02 | 2.931E-04 | 4.786E-09 | 6.576E04 | 3.148E-04 |
| **VHH-Fc3-IgG1-hinge** | 3.569E-10 | 1.982E05 | 7.074E-05 | 5.169E-09 | 7.334E04 | 3.791E-04 |
| **VHH-Fc3-IgG2-derived** | 4.125E-10 | 1.777E05 | 7.330E-05 | 5.394E-09 | 6.093E04 | 3.287E-04 |
| **VHH-Fc3-IgG3-derived** | 2.095E-10 | 2.086E05 | 4.370E-05 | 6.167E-09 | 8.756E04 | 5.400E-04 |
| **VHH-Fc4-IgG1-hinge** | <1.0E-12 | 1.460E05 | <1.0E-07 | 5.321E-09 | 5.841E04 | 3.108E-04 |
| **VHH-Fc4-IgG2-derived** | 1.958E-10 | 1.377E05 | 2.697E-05 | 1.960E-09 | 5.699E04 | 1.117E-04 |
| **VHH-Fc4-IgG3-derived** | 8.201E-12 | 8.132E04 | 6.669E-07 | 7.073E-09 | 6.315E04 | 4.467E-04 |
| **VHH-Fc5-IgG1-hinge** | 6.375E-12 | 7.574E03 | <1.0E-07 | 3.980E-09 | 6.450E04 | 2.567E-04 |
| **VHH-Fc5-IgG2-derived** | 7.752E-12 | 6.237E03 | <1.0E-07 | 3.793E-09 | 5.761E04 | 2.185E-04 |
| **VHH-Fc5-IgG3-derived** | 6.053E-12 | 7.972E03 | <1.0E-07 | 6.844E-09 | 6.546E04 | 4.481E-04 |

### Example 4 - Cell binding of VHH-Fc fusions

To further validate the VHH-Fcs, the present inventors analysed cell binding for VHH-Fcs described above comprising the IgG1 hinge and IgG3-derived hinge regions.

Target positive tumor cells were stained with different concentrations of VHH-Fcs comprising IgG1 hinges and IgG3-derived hinges. Mean fluorescence intensities (MFI) values were plotted against the concentration of the VHH-Fc and the resulting data points were fitted using a non-linear fit, resulting in the EC50 values of the respective VHH-Fc. These values are shown in Table 4 below.

These experiments revealed a majority of sub-nanomolar EC50 values on target cells. The different hinge regions mediate identical binding to the antigen-positive cells. As such, EC50 is not impacted by optimization of the hinge region for higher DARs, modification to avoid O-glycosylation or the specificity of the VHHs. This further emphasizes that one may choose the hinge design solely on DAR and not on other properties.

**Table 4 - Cell binding of VHH-Fc fusions. Target positive tumor cells were stained with different concentrations of VHH-Fcs and EC50 values were determined.**

| **Protein** | **EC50** |
|---|---|
| | **nM** |
| **VHH-Fc1-IgG1-hinge** | 0.6554 |
| **VHH-Fc1-IgG3-derived** | 0.7432 |
| **VHH-Fc2-IgG1-hinge** | nd |
| **VHH-Fc2-IgG3-derived** | nd |
| **VHH-Fc3-IgG1-hinge** | 0.5361 |
| **VHH-Fc3-IgG3-derived** | 0.8071 |
| **VHH-Fc4-IgG1-hinge** | 0.8140 |
| **VHH-Fc4-IgG3-derived** | 0.9101 |
| **VHH-Fc5-IgG1-hinge** | 2.465 |
| **VHH-Fc5-IgG3-derived** | 2.202 |

### Example 5 - Internalization of VHH-Fc fusions

To further validate the VHH-Fcs, the present inventors assessed internalization for the VHH-Fcs described above comprising IgG1 hinges and IgG3-derived hinges.

Internalization of VHH-Fcs comprising IgG1 hinges and IgG3-derived hinges into target positive tumor cells was investigated using a FACS-based assay which confirmed fast internalization of all candidates independent from the engineered Hinge region.

Internalization was studied at four different time points (0.5, 1, 2, and 4 hours). As a control, candidates were incubated on ice to reduce internalization.

Internalization was examined by incubation at 37°C followed by FACS analysis. All candidates clearly show strong and rapid internalization, detectable after just 30 min (**Figure 2**).

It is clear from Figure 2 that all tested hinge regions mediate identical internalization to the antigen-positive cells. Internalization is not detrimentally impacted by optimization of the hinge region for higher DARs or modification to avoid O-glycosylation, since the different hinge regions tested showed the same internalization for a given VHH. This again emphasizes that one may choose the hinge design solely on DAR and not on other properties.

### Example 6 - Intact mass spectrometry data of VHH-Fc fusion proteins

To further validate the VHH-Fcs, the present inventors assessed internalization for the VHH-Fcs described above comprising IgG1 hinges and IgG3-derived hinges.

The intact masses of deglycosylated VHH-Fcs comprising IgG1 hinges and IgG3-derived hinge regions were determined using mass spectrometry.

The calculated mass and deconvoluted (experimental) mass are displayed below in Table 5. The intact masses are extremely close in value to the calculated masses. This confirms that the disulphide bridges are closed. A high resolution was achieved, making the presence of unpaired hinge cysteines extremely unlikely.

**Table 5 - Intact mass data of VHH-Fc fusion proteins. Intact masses of deglycosylated VHH-Fc fusion proteins containing IgG1 hinges and IgG3-derived hinge regions.**

| **Protein** | **Calculated mass [Da]** | **Deconvoluted mass [Da]** | **Mass difference [Da]** | **Delta mass [ppm]** |
|---|---|---|---|---|
| **VHH-Fc1-IgG1-hinge** | 79104.3 | 79104.5 | 0.2 | 2.9 |
| **VHH-Fc1-IgG3-derived** | 80972.6 | 80973.0 | 0.4 | 5.2 |
| **VHH-Fc2-IgG1-hinge** | 76013.1 | 76013.0 | -0.1 | -1.5 |
| **VHH-Fc2-IgG3-derived** | 77881.4 | 77881.5 | 0.1 | 1.9 |
| **VHH-Fc3-IgG1-hinge** | 77132.1 | 77132.1 | -0.1 | -0.8 |
| **VHH-Fc3-IgG3-derived** | 79000.4 | 79000.5 | 0.1 | 1.7 |
| **VHH-Fc4-IgG1-hinge** | 77738.9 | 77739.0 | 0.1 | 0.8 |
| **VHH-Fc4-IgG3-derived** | 79607.2 | 79607.5 | 0.2 | 3.1 |
| **VHH-Fc5-IgG1-hinge** | 78725.5 | 78725.5 | 0.0 | 0.3 |
| **VHH-Fc5-IgG3-derived** | 80593.8 | 80594.0 | 0.2 | 2.4 |

### Discussion

The present inventors have generated an antibody format that enables the control (or predetermination) of the DAR of an ADC by designing engineered IgG hinge regions comprising a predetermined number of cysteine residues. This entirely novel class of antibodies comprising engineered IgG hinge regions provides a functional antibody format that was produced as efficiently as a conventional antibody format.

It was further demonstrated that modifications to avoid O-glycosylation within the engineered IgG hinges were well tolerated. The VHH-Fc constructs comprising such modifications were functional, and would be expected to provide a homogenous ADC product suitable for large-scale manufacture for clinical use.

FACS experiments showed that all of the tested VHH-Fcs comprising engineered IgG hinge regions were able to bind to target positive cells. This underlines the full functionality of the VHH-Fcs.

Thus, this work provides new antibodies that can be designed to have the desired DAR, that provide a homogenous ADC product and that have preserved or elevated ADC properties, including stability, cell binding, internalization compared to conventional antibodies.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. An antibody-drug conjugate (ADC),
wherein the antibody is conjugated to the drug by cysteine-based site-specific conjugation,
wherein the antibody comprises a first polypeptide comprising an immunoglobulin fragment crystallisable (Fc) region, at least one variable domain N-terminal of the Fc region and an engineered IgG hinge region between the Fc region and the at least one variable domain, and
wherein the engineered IgG hinge region is heterologous to the Fc region and/or is mutated to provide a predetermined number of cysteine residues.

2. The ADC according to claim 1, wherein the antibody further comprises a second polypeptide comprising an Fc region and an engineered IgG hinge region N-terminal of the Fc region, wherein the engineered IgG hinge region is heterologous to the Fc region of the second polypeptide and/or is mutated to provide a predetermined number of cysteine residues, and wherein the engineered IgG hinge region of the first polypeptide and the engineered IgG hinge region of the second polypeptide are complimentary engineered IgG hinge regions.

3. The ADC according to claim 2, wherein the second polypeptide further comprises at last one variable domain N-terminal of the engineered IgG hinge region.

4. An antibody-drug conjugate (ADC),
wherein the antibody is conjugated to the drug by cysteine-based site-specific conjugation,
wherein the antibody comprises a first polypeptide and a second polypeptide, wherein each of the first polypeptide and the second polypeptide comprises an immunoglobulin fragment crystallisable (Fc) region, at least one variable domain which is a VHH N-terminal of the Fc region and an engineered IgG hinge region between the Fc region and the at least one VHH,
wherein the engineered IgG hinge region of the first polypeptide and the engineered IgG hinge region of the second polypeptide are complimentary engineered IgG hinge regions, and
wherein the engineered IgG hinge region is heterologous to the Fc region and/or is mutated to provide a predetermined number of cysteine residues.

5. The ADC according to any one of the preceding claims, wherein the engineered IgG hinge region of the first polypeptide or the engineered IgG hinge region of each of the first polypeptide and the second polypeptide comprises at least one cysteine residue, preferably wherein the engineered IgG hinge region comprises two, three, four, five, six, seven, eight or nine cysteine residues.

6. The ADC according to any one of the preceding claims, wherein the engineered IgG hinge region of the first polypeptide comprises at least one additional cysteine residue compared to the native hinge region of the Fc region of the first polypeptide, optionally wherein the engineered IgG hinge region of the second polypeptide comprises at least one additional cysteine residue compared to the native hinge region of the Fc region of the second polypeptide.

7. The ADC according to any one of the preceding claims, wherein the engineered IgG hinge region of the first polypeptide comprises at least one fewer cysteine residue compared to the native hinge region of the Fc region of the first polypeptide, optionally wherein the engineered IgG hinge region of the second polypeptide comprises at least one fewer cysteine residue compared to the native hinge region of the Fc region of the second polypeptide.

8. The ADC according to any one of the preceding claims, wherein the engineered IgG hinge region is an IgG1 hinge region, an IgG2 hinge region, an IgG3 hinge region or an IgG4 hinge region.

9. The ADC according to any one of the preceding claims, wherein the engineered IgG hinge region has:
(i) an amino acid sequence as set forth in SEQ ID NO: 1, or is a fragment thereof and/or a variant thereof comprising an amino acid sequence having at least 70% identity to SEQ ID NO: 1;
(ii) an amino acid sequence as set forth in SEQ ID NO: 2, or is a fragment thereof and/or a variant thereof comprising or an amino acid sequence having at least 70% identity to SEQ ID NO: 2;
(iii) an amino acid sequence as set forth in SEQ ID NO: 3 or SEQ ID NO: 4, or is a fragment thereof and/or a variant thereof comprising an amino acid sequence having at least 70% identity to SEQ ID NO: 3 or to SEQ ID NO: 4;
(iv) an amino acid sequence as set forth in SEQ ID NO: 6 or SEQ ID NO: 7, or is a fragment thereof and/or a variant thereof comprising an amino acid sequence having at least 70% identity to SEQ ID NO: 6 or to SEQ ID NO: 7; or
(v) an amino acid sequence as set forth in SEQ ID NO: 5, or is a fragment thereof and/or a variant thereof comprising an amino acid sequence having at least 70% identity to SEQ ID NO: 5.

10. The ADC according to any one of the preceding claims, wherein the engineered IgG hinge region has an amino acid sequence as set forth in any one of SEQ ID NOs: 8-118, preferably an amino acid sequence as set forth in SEQ ID NO: 8, 9, 12, 13, 18, 51, 67, 74, 84, 100, 113 or 118.

11. The ADC according to any one of the preceding claims, wherein the engineered IgG hinge region of the first polypeptide and/or second polypeptide comprises at least one amino acid modification to avoid O-glycosylation compared to the native IgG hinge region of the Fc region of the first polypeptide and/or second polypeptide.

12. The ADC according to any one of the preceding claims, wherein the engineered IgG hinge region is or is derived from a human IgG hinge region.

13. The ADC according to any one of the preceding claims, wherein the antibody is conjugated to the drug by cysteine-based site-specific conjugation through all of the cysteine residues within the engineered IgG hinge region.

14. The ADC according to any one of the preceding claims, wherein the ADC has a drug-to-antibody-ratio (DAR) of from about 2 to about 12, preferably a DAR of from about 4 to about 8.

15. The ADC according to any one of the preceding claims, wherein the ADC has a drug-to-antibody-ratio (DAR) of about 2, 4, 6, 8, 10, or 12, preferably a DAR of about 8.

16. A method of producing an antibody-drug conjugate (ADC) having a predetermined drug-to-antibody ratio (DAR), comprising the steps of:
(i) providing an antibody as defined in any one of claims 1-15;
(ii) reducing the antibody with a reducing agent; and
(iii) conjugating the reduced antibody with the drug.

17. A method of producing an antibody-drug conjugate (ADC) having a predetermined drug-to-antibody ratio (DAR), comprising the steps of:
(i)
(a) providing at least one polynucleotide sequence encoding an antibody comprising a first polypeptide comprising an immunoglobulin fragment crystallisable (Fc) region and at least one variable domain N-terminal of the Fc region;
(b) modifying the at least one polynucleotide sequence to encode an engineered IgG hinge region between the Fc region and the at least one variable domain of the first polypeptide, wherein the engineered IgG hinge region is heterologous to the Fc region and/or is mutated to provide a predetermined number of cysteine residues;
(c) introducing the at least one polynucleotide sequence into a cell;
(d) culturing the cell under conditions suitable for the expression of the at least one polynucleotide sequence;
(e) isolating the antibody expressed by the cell;
(ii) reducing the antibody with a reducing agent; and
(iii) conjugating the reduced antibody with the drug.

18. A method of producing an antibody-drug conjugate (ADC) having a predetermined drug-to-antibody ratio (DAR), comprising the steps of:
(i)
(a) providing at least one polynucleotide sequence encoding an antibody wherein the antibody comprises a first polypeptide and a second polypeptide, wherein each of the first polypeptide and the second polypeptide comprises an immunoglobulin fragment crystallisable (Fc) region and at least one variable domain which is a VHH N-terminal of the Fc region;
(b) modifying the at least one polynucleotide sequence to encode an engineered IgG hinge region between the Fc region and the at least one variable domain of each of the first polypeptide and the second polypeptide, wherein the engineered IgG hinge region of the first polypeptide and the engineered IgG hinge region of the second polypeptide are complimentary engineered IgG hinge regions, and wherein the engineered IgG hinge region is heterologous to the Fc region and/or is mutated to provide a predetermined number of cysteine residues;
(c) introducing the at least one polynucleotide sequence into a cell;
(d) culturing the cell under conditions suitable for the expression of the at least one polynucleotide sequence;
(e) isolating the antibody expressed by the cell;
(ii) reducing the antibody with a reducing agent; and
(iii) conjugating the reduced antibody with the drug.

19. The method according to any one of claims 16-18, wherein the step of reducing the antibody in step (ii) is a partial reduction of the antibody to reduce the inter-chain disulphide bonds of the antibody.

20. The method according to any one of claims 16-19, wherein:
(a) the drug is in the form of a linker-drug conjugate comprising a thiol-reactive group; and/or
(b) wherein step (iii) is performed using a thiol-reactive coupling strategy.

21. The method according to any one of claims 16-20, wherein the method provides homogenous ADCs.

22. The method according to any one of claims 17 or 19-21, wherein the antibody further comprises a second polypeptide comprising an Fc region, and wherein step (i) (b) further comprises modifying the at least one polynucleotide sequence to encode an engineered IgG hinge region N-terminal of the Fc region of the second polypeptide, wherein the engineered IgG hinge region is heterologous to the Fc region of the second polypeptide and/or is mutated to provide a predetermined number of cysteine residues, and wherein the engineered IgG hinge region of the first polypeptide and the engineered IgG hinge region of the second polypeptide are complimentary engineered IgG hinge regions.

23. The method according to any one of claims 16-22, wherein:
(a) all of the cysteine residues within the engineered IgG hinge region form stable inter-chain disulphide bonds prior to step (ii);
(b) in step (iii) the antibody is conjugated to the drug by cysteine-based site-specific conjugation through all of the cysteine residues within the engineered IgG hinge region;
(c) the ADC has a drug-to-antibody-ratio (DAR) of from about 2 to about 12, preferably a DAR of from about 4 to about 8; and/or
(d) the ADC has a drug-to-antibody-ratio (DAR) of about 2, 4, 6, 8, 10, or 12, preferably a DAR of about 8.

24. An ADC obtained or obtainable by the method according to any one of claims 16-23.

25. A pharmaceutical composition comprising the ADC according to any one of claims 1-15 or 24 and a pharmaceutically acceptable carrier, excipient and/or diluent.

26. The ADC according to any one of claims 1-15 or 24, or the pharmaceutical composition according to claim 25, for use in therapy.

27. The ADC according to any one of claims 1-15 or 24, or the pharmaceutical composition according to claim 25, for use in the treatment of a disease or condition selected from the group consisting of cancer, autoimmune disease, infection, infectious diseases, cardiovascular diseases and liver metabolic disorders.
